# EUROPÄISCHE PATENTANMELDUNG

(11) **EP 1 643 251 A1**
(43) Veröffentlichungstag der Anmeldung: **05.04.2006**
(21) Anmeldenummer: 05018635.2
(22) Anmeldetag: 24.09.2002
(51) Int. Cl.: G01N 33/68, A61P 25/00

(54) **Screeningverfahren für verschiedene Indikationen mit BNPI und/oder DNPI**

(30) Priorität: 24.09.2001 DE 10147006; 25.09.2001 DE 10147028
(62) Teilanmeldung aus: 02777190.6
(71) Anmelder: Grünenthal GmbH, 52078 Aachen (DE)
(72) Erfinder: Weihe, Eberhard, Prof. Dr., 35037 Marburg (DE); Schäfer, Martin K.-H., Dr., 35041 Marburg (DE)
(74) Vertreter: Graf von Stosch, Andreas

(57) **Zusammenfassung**

Die Erfindung betrifft ein Verfahren zur Auffindung pharmazeutisch wirksamer Substanzen unter Verwendung von BPNI bzw. davon abgeleiteter Biomoleküle und die Verwendung dadurch identifizierter Verbindungen, an BNPI und/oder DNPI bindender Wirkstoffe, gegen BNPI und/oder DNPI gerichteter Antikörper, von Antisensenukleotiden gegen BNPI und/oder DNPI, oder von BNPI und/oder DNPI bzw. Teilproteinen davon, bzw. entsprechenden Polynukleotiden zur Herstellung von Arzneimitteln zur Behandlung verschiedener Erkrankungen oder zu Therapie und Diagnostik.

SEQUENZPROTOKOLL

<110> GRUENENTHAL GmbH

<120> Screeningverfahren fuer verschiedene Indikationen mit
      BNPI und/oder DNPI

<130> GR01P010WOEPT2

<150> PCT/EP 02/10707
<151> 2002-09-24

<150> DE10147006.1
<151> 2001-09-24

<160> 14

<170> PatentIn Ver. 2.1

<210> 1
<211> 2366
<212> DNA
<213> Homo sapiens

<400> 1
   ccggcggcag gagccgccac catggagttc cgccaggagg agtttcggaa gctagcgggt 60
   cgtgctctcg ggaagctgca ccgccttctg gagaagcggc aggaaggcgc ggagacgctg 120
   gagctgagtg cggatgggcg cccggtgacc acgcagaccc gggacccgcc ggtggtggac 180
   tgcacctgct tcggcctccc tcgccgctac attatcgcca tcatgagtgg tctgggcttc 240
   tgcatcagct ttggcatccg ctgcaacctg ggcgtggcca tcgtctccat ggtcaataac 300
   agcacgaccc accgcggggg ccacgtggtg gtgcagaaag cccagttcag ctgggatcca 360
   gagactgtcg gcctcataca cggctccttt ttctggggct acattgtcac tcagattcca 420
   ggaggattta tctgtcaaaa atttgcagcc aacagagttt tcggctttgc tattgtggca 480
   acatccactc taaacatgct gatcccctca gctgcccgcg tccactatgg ctgtgtcatc 540
   ttcgtgagga tcctgcaggg gttggtagag ggggtcacat accccgcctg ccatgggatc 600
   tggagcaaat gggccccacc cttagaacgg agtcgcctgg cgacgacagc cttttgtggt 660
   tcctatgctg gggcggtggt cgcgatgccc ctcgccgggg tccttgtgca gtactcagga 720
   tggagctctg ttttctacgt ctacggcagc ttcgggatct tctggtacct gttctggctg 780
   ctcgtctcct acgagtcccc cgcgctgcac cccagcatct cggaggagga gcgcaagtac 840
   atcgaggacg ccatcggaga gagcgcgaaa ctcatgaacc ccctcacgaa gtttagcact 900
   ccctggcggc gcttcttcac gtctatgcca gtctatgcca tcatcgtggc caacttctgc 960
   cgcagctgga cgttctacct gctgctcatc tcccagcccg cctacttcga agaagtgttc 1020
   ggcttcgaga tcagcaaggt aggcctggtg tccgcgctgc cccacctggt catgaccatc 1080
   atcgtgccca tcggcggcca gatcgcggac ttcctgcgga gccgccgcat catgtccacc 1140
   accaacgtgc gcaagttgat gaactgcgga ggcttcggca tggaagccac gctgctgttg 1200
   gtggtcggct actcgcactc caagggcgtg gccatctcct tcctggtcct agccgtgggc 1260
   ttcagcggct tcgccatctc tgggttcaac gtgaaccacc tggacatagc cccgcgctac 1320
   gccagcatcc tcatgggcat ctccaacggc gtgggcacac tgtcgggcat ggtgtgcccc 1380
   atcatcgtgg gggccatgac taagcacaag actcgggagg agtggcagta cgtgttccta 1440
   attgcctccc tggtgcacta tggaggtgtc atcttctacg gggtctttgc ttctggagag 1500
   aagcagccgt gggcagagcc tgaggagatg agcgaggaga agtgtggctt cgttggccat 1560
   gaccagctgg ctggcagtga cgacagcgaa atggaggatg aggctgagcc cccgggggca 1620
   ccccctgcac ccccgccctc ctatggggcc acacacagca catttcagcc ccccaggccc 1680
   ccaccccctg tccgggacta ctgaccatgt gcctcccact gaatggcagt ttccaggacc 1740
   tccattccac tcatctctgg cctgagtgac agtgtcaagg aaccctgctc ctctctgtcc 1800
   tgcctcaggc ctaagaagca ctctcccttg ttcccagtgc tgtcaaatcc tctttccttc 1860
   ccaattgcct ctcaggggta gtgaagctgc agactgacag tttcaaggat acccaaattc 1920
   ccctaaaggt tccctctcca cccgttctgc ctcagtggtt tcaaatctct cctttcaggg 1980
   ctttatttga atggacagtt cgacctctta ctctctcttg tggttttgag gcacccacac 2040
   cccccgcttt cctttatctc cagggactct caggctaacc tttgagatca ctcagctccc 2100
   atctcctttc agaaaaattc aaggtcctcc tctagaagtt tcaaatctct cccaactctg 2160
   ttctgcatct tccagattgg tttaaccaat tactcgtccc cgccattcca gggattgatt 2220
   ctcaccagcg tttctgatgg aaaatggcgg tttcaagtcc ccgattccgt gcccacttca 2280
   catctcccct accagcagat tctgcgaaag caccaaattt ctcaagaccc tcttctccct 2340
   agcttagcat aatgtctggg gaaaca 2366

<210> 2
<211> 560
<212> PRT
<213> Homo sapiens

<400> 2
Met Glu Phe Arg Gln Glu Glu Phe Arg Lys Leu Ala Gly Arg Ala Leu
1               5                   10                  15

Gly Lys Leu His Arg Leu Leu Glu Lys Arg Gln Glu Gly Ala Glu Thr
            20                  25                  30

Leu Glu Leu Ser Ala Asp Gly Arg Pro Val Thr Thr Gln Thr Arg Asp
        35                  40                  45

Pro Pro Val Val Asp Cys Thr Cys Phe Gly Leu Pro Arg Arg Tyr Ile
    50                  55                  60

Ile Ala Ile Met Ser Gly Leu Gly Phe Cys Ile Ser Phe Gly Ile Arg
65                  70                  75                  80

Cys Asn Leu Gly Val Ala Ile Val Ser Met Val Asn Asn Ser Thr Thr
                85                  90                  95

His Arg Gly Gly His Val Val Val Gln Lys Ala Gln Phe Ser Trp Asp
            100                 105                 110

Pro Glu Thr Val Gly Leu Ile His Gly Ser Phe Phe Trp Gly Tyr Ile
        115                 120                 125

Val Thr Gln Ile Pro Gly Gly Phe Ile Cys Gln Lys Phe Ala Ala Asn
    130                 135                 140

Arg Val Phe Gly Phe Ala Ile Val Ala Thr Ser Thr Leu Asn Met Leu
145                 150                 155                 160

Ile Pro Ser Ala Ala Arg Val His Tyr Gly Cys Val Ile Phe Val Arg
                165                 170                 175

Ile Leu Gln Gly Leu Val Glu Gly Val Thr Tyr Pro Ala Cys His Gly
            180                 185                 190

Ile Trp Ser Lys Trp Ala Pro Pro Leu Glu Arg Ser Arg Leu Ala Thr
        195                 200                 205

Thr Ala Phe Cys Gly Ser Tyr Ala Gly Ala Val Val Ala Met Pro Leu
    210                 215                 220

Ala Gly Val Leu Val Gln Tyr Ser Gly Trp Ser Ser Val Phe Tyr Val
225                 230                 235                 240

Tyr Gly Ser Phe Gly Ile Phe Trp Tyr Leu Phe Trp Leu Leu Val Ser
                245                 250                 255

Tyr Glu Ser Pro Ala Leu His Pro Ser Ile Ser Glu Glu Glu Arg Lys
            260                 265                 270

Tyr Ile Glu Asp Ala Ile Gly Glu Ser Ala Lys Leu Met Asn Pro Leu
        275                 280                 285

Thr Lys Phe Ser Thr Pro Trp Arg Arg Phe Phe Thr Ser Met Pro Val
    290                 295                 300

Tyr Ala Ile Ile Val Ala Asn Phe Cys Arg Ser Trp Thr Phe Tyr Leu
305                 310                 315                 320

Leu Leu Ile Ser Gln Pro Ala Tyr Phe Glu Glu Val Phe Gly Phe Glu
                325                 330                 335

Ile Ser Lys Val Gly Leu Val Ser Ala Leu Pro His Leu Val Met Thr
            340                 345                 350

Ile Ile Val Pro Ile Gly Gly Gln Ile Ala Asp Phe Leu Arg Ser Arg
        355                 360                 365

Arg Ile Met Ser Thr Thr Asn Val Arg Lys Leu Met Asn Cys Gly Gly
    370                 375                 380

Phe Gly Met Glu Ala Thr Leu Leu Leu Val Val Gly Tyr Ser His Ser
385                 390                 395                 400

Lys Gly Val Ala Ile Ser Phe Leu Val Leu Ala Val Gly Phe Ser Gly
                405                 410                 415

Phe Ala Ile Ser Gly Phe Asn Val Asn His Leu Asp Ile Ala Pro Arg
            420                 425                 430

Tyr Ala Ser Ile Leu Met Gly Ile Ser Asn Gly Val Gly Thr Leu Ser
        435                 440                 445

Gly Met Val Cys Pro Ile Ile Val Gly Ala Met Thr Lys His Lys Thr
    450                 455                 460

Arg Glu Glu Trp Gln Tyr Val Phe Leu Ile Ala Ser Leu Val His Tyr
465                 470                 475                 480

Gly Gly Val Ile Phe Tyr Gly Val Phe Ala Ser Gly Glu Lys Gln Pro
                485                 490                 495

Trp Ala Glu Pro Glu Glu Met Ser Glu Glu Lys Cys Gly Phe Val Gly
            500                 505                 510

His Asp Gln Leu Ala Gly Ser Asp Asp Ser Glu Met Glu Asp Glu Ala
        515                 520                 525

Glu Pro Pro Gly Ala Pro Pro Ala Pro Pro Pro Ser Tyr Gly Ala Thr
    530                 535                 540

His Ser Thr Phe Gln Pro Pro Arg Pro Pro Pro Pro Val Arg Asp Tyr
545                 550                 555                 560

<210> 3
<211> 2716
<212> DNA
<213> Homo sapiens

<400> 3
cgataagctt gatatcgaat tccggactct tgctcgggcg ccttaacccg gcgttcggtt 60
catcccgcag cgccagttct gcttaccaaa agtggcccac taggcactcg cattccacgc 120
ccggctccac gccagcgagc cgggcttctt acccatttaa agtttgagaa taggttgaga 180
tcgtttcggc cccaagacct ctaatcattc gctttaccgg ataaaactgc gtggcggggg 240
tgcgtcgggt ctgcgagagc gccagctatc ctgagggaaa cttcggaggg aaccagctac 300
tagatggttc gattagtctt tcgcccctat acccaggtcg gacgaccgat ttgcacgtca 360
ggaccgctac ggacctccac cagagtttcc tctggcttcg ccctgcccag gcgatcggcg 420
ggggggaccc gcggggtgac cggcggcagg agccgccacc atggagttcc gccaggagga 480
gtttcggaag ctagcgggtc gtgctctcgg gaagctgcac cgccttctgg agaagcggca 540
ggaaggcgcg gagacgctgg agctgagtgc ggatgggcgc ccggtgacca cgcagacccg 600
ggacccgccg gtggtggact gcacctgctt cggcctccct cgccgctaca ttatcgccat 660
catgagtggt ctgggcttct gcatcagctt tggcatccgc tgcaacctgg gcgtggccat 720
cgtctccatg gtcaataaca gcacgaccca ccgcgggggc cacgtggtgg tgcagaaagc 780
ccagttcagc tgggatccag agactgtcgg cctcatacac ggctcctttt tctggggcta 840
cattgtcact cagattccag gaggatttat ctgtcaaaaa tttgcagcca acagagtttt 900
cggctttgct attgtggcaa catccactct aaacatgctg atcccctcag ctgcccgcgt 960
ccactatggc tgtgtcatct tcgtgaggat cctgcagggg ttggtagagg gggtcacata 1020
ccccgcctgc catgggatct ggagcaaatg ggccccaccc ttagaacgga gtcgcctggc 1080
gacgacagcc ttttgtggtt cctatgctgg ggcggtggtc gcgatgcccc tcgccggggt 1140
ccttgtgcag tactcaggat ggagctctgt tttctacgtc tacggcagct tcgggatctt 1200
ctggtacctg ttctggctgc tcgtctccta cgagtccccc gcgctgcacc ccagcatctc 1260
ggaggaggag cgcaagtaca tcgaggacgc catcggagag agcgcgaaac tcatgaaccc 1320
cctcacgaag tttagcactc cctggcggcg cttcttcacg tctatgccag tctatgccat 1380

catcgtggcc aacttctgcc gcagctggac gttctacctg ctgctcatct cccagcccga 1440
ctacttcgaa gaagtgttcg gcttcgagat cagcaaggta ggcctggtgt ccgcgctgcc 1500
ccacctggtc atgaccatca tcgtgcccat cggcggccag atcgcggact tcctgcggag 1560
ccgccgcatc atgtccacca ccaacgtgcg caagttgatg aactgcggag gcttcggcat 1620
ggaagccacg ctgctgttgg tggtcggcta ctcgcactcc aagggcgtgg ccatctcctt 1680
cctggtccta gccgtgggct tcagcggctt cgccatctct gggttcaacg tgaaccacct 1740
ggacatagcc ccgcgctacg ccagcatcct catgggcatc tccaacggcg tgggcacact 1800
gtcgggcatg gtgtgcccca tcatcgtggg ggccatgact aagcacaaga ctcgggagga 1860
gtggcagtac gtgttcctaa ttgcctccct ggtgcactat ggaggtgtca tcttctacgg 1920
ggtctttgct tctggagaga agcagccgtg ggcagagcct gaggagatga gcgaggagaa 1980
gtgtggcttc gttggccatg accagctggc tggcagtgac gacagcgaaa tggaggatga 2040
ggctgagccc ccgggggcac cccctgcacc cccgccctcc tatggggcca cacacagcac 2100
atttcagccc cccaggcccc caccccctgt ccgggactac tgaccatgtg cctcccactg 2160
aatggcagtt tccaggacct ccattccact catctctggc ctgagtgaca gtgtcaagga 2220
accctgctcc tctctgtcct gcctcaggcc taagaagcac tctcccttgt tcccagtgct 2280
gtcaaatcct ctttccttcc caattgcctc tcaggggtag tgaagctgca gactgacagt 2340
ttcaaggata cccaaattcc cctaaaggtt ccctctccac ccgttctgcc tcagtggttt 2400
caaatctctc ctttcagggc tttatttgaa tggacagttc gacctcttac tctctcttgt 2460
ggttttgagg cacccacacc ccccgctttc ctttatctcc agggactctc aggctaacct 2520
ttgagatcac tcagctccca tctcctttca gaaaaattca aggtcctcct ctagaagttt 2580
caaatctctc ccaactctgt tctgcatctt ccagattggt ttaaccaatt actcgtcccc 2640
gccattccag ggattgattc tcaccagcgt ttctgatgga aaatggcggg aattcctgca 2700
gcccggggga tccact 2716

<210> 4
<211> 560
<212> PRT
<213> Homo sapiens

<400> 4
Met Glu Phe Arg Gln Glu Glu Phe Arg Lys Leu Ala Gly Arg Ala Leu
1               5                   10                  15

Gly Lys Leu His Arg Leu Leu Glu Lys Arg Gln Glu Gly Ala Glu Thr
            20                  25                  30

Leu Glu Leu Ser Ala Asp Gly Arg Pro Val Thr Thr Gln Thr Arg Asp
        35                  40                  45

Pro Pro Val Val Asp Cys Thr Cys Phe Gly Leu Pro Arg Arg Tyr Ile
    50                  55                  60

Ile Ala Ile Met Ser Gly Leu Gly Phe Cys Ile Ser Phe Gly Ile Arg
65                  70                  75                  80

Cys Asn Leu Gly Val Ala Ile Val Ser Met Val Asn Asn Ser Thr Thr
                85                  90                  95

His Arg Gly Gly His Val Val Val Gln Lys Ala Gln Phe Ser Trp Asp
            100                 105                 110

Pro Glu Thr Val Gly Leu Ile His Gly Ser Phe Phe Trp Gly Tyr Ile

115                 120                 125

Val Thr Gln Ile Pro Gly Gly Phe Ile Cys Gln Lys Phe Ala Ala Asn
    130                 135                 140

Arg Val Phe Gly Phe Ala Ile Val Ala Thr Ser Thr Leu Asn Met Leu
145                 150                 155                 160

Ile Pro Ser Ala Ala Arg Val His Tyr Gly Cys Val Ile Phe Val Arg
                165                 170                 175

Ile Leu Gln Gly Leu Val Glu Gly Val Thr Tyr Pro Ala Cys His Gly
            180                 185                 190

Ile Trp Ser Lys Trp Ala Pro Pro Leu Glu Arg Ser Arg Leu Ala Thr
        195                 200                 205

Thr Ala Phe Cys Gly Ser Tyr Ala Gly Ala Val Val Ala Met Pro Leu
    210                 215                 220

Ala Gly Val Leu Val Gln Tyr Ser Gly Trp Ser Ser Val Phe Tyr Val
225                 230                 235                 240

Tyr Gly Ser Phe Gly Ile Phe Trp Tyr Leu Phe Trp Leu Leu Val Ser
                245                 250                 255

Tyr Glu Ser Pro Ala Leu His Pro Ser Ile Ser Glu Glu Glu Arg Lys
            260                 265                 270

Tyr Ile Glu Asp Ala Ile Gly Glu Ser Ala Lys Leu Met Asn Pro Leu
        275                 280                 285

Thr Lys Phe Ser Thr Pro Trp Arg Arg Phe Phe Thr Ser Met Pro Val
    290                 295                 300

Tyr Ala Ile Ile Val Ala Asn Phe Cys Arg Ser Trp Thr Phe Tyr Leu
305                 310                 315                 320

Leu Leu Ile Ser Gln Pro Asp Tyr Phe Glu Glu Val Phe Gly Phe Glu
                325                 330                 335

Ile Ser Lys Val Gly Leu Val Ser Ala Leu Pro His Leu Val Met Thr
            340                 345                 350

Ile Ile Val Pro Ile Gly Gly Gln Ile Ala Asp Phe Leu Arg Ser Arg
        355                 360                 365

Arg Ile Met Ser Thr Thr Asn Val Arg Lys Leu Met Asn Cys Gly Gly
    370                 375                 380

Phe Gly Met Glu Ala Thr Leu Leu Leu Val Val Gly Tyr Ser His Ser
385                 390                 395                 400

Lys Gly Val Ala Ile Ser Phe Leu Val Leu Ala Val Gly Phe Ser Gly
                405                 410                 415

Phe Ala Ile Ser Gly Phe Asn Val Asn His Leu Asp Ile Ala Pro Arg
            420                 425                 430

Tyr Ala Ser Ile Leu Met Gly Ile Ser Asn Gly Val Gly Thr Leu Ser
        435                 440                 445

Gly Met Val Cys Pro Ile Ile Val Gly Ala Met Thr Lys His Lys Thr
    450                 455                 460

Arg Glu Glu Trp Gln Tyr Val Phe Leu Ile Ala Ser Leu Val His Tyr
465                 470                 475                 480

Gly Gly Val Ile Phe Tyr Gly Val Phe Ala Ser Gly Glu Lys Gln Pro
                485                 490                 495

Trp Ala Glu Pro Glu Glu Met Ser Glu Glu Lys Cys Gly Phe Val Gly
            500                 505                 510

His Asp Gln Leu Ala Gly Ser Asp Asp Ser Glu Met Glu Asp Glu Ala
        515                 520                 525

Glu Pro Pro Gly Ala Pro Pro Ala Pro Pro Pro Ser Tyr Gly Ala Thr
    530                 535                 540

His Ser Thr Phe Gln Pro Pro Arg Pro Pro Pro Pro Val Arg Asp Tyr
545                 550                 555                 560

<210> 5
<211> 2024
<212> DNA
<213> Rattus norvegicus

<400> 5
gaattcggca cgagcggagc tgcggggccg ggccgggccg gggcggaccc cgggatcccg 60
gacgcggccg cccgggcccg cgggcggggg gattggcagg ggacccgcgt gggcacagcc 120
accatggagt tccggcagga ggagtttcgg aagctggcgg ggcgcgccct ggggaggctg 180
caccggttac tggagaagcg gcaggaaggc gcggagacat tggagctgag cgccgacggg 240
cgcccagtga ccacacacac gcgggacccg ccggtggtgg actgcacttg ctttggcctc 300
cctcgccgct acatcatcgc gatcatgagc ggtctgggtt tctgcatcag ctttggcatc 360
cgctgcaacc tgggcgtggc catcgtatcc atggtcaaca acagtacaac ccaccgtggg 420
ggccacgtgg tggtgcagaa agcccagttc aactgggatc cagagactgt cggcctcata 480
catggctcct ttttctgggg gtacattgtc actcagattc ctggaggatt tatctgccaa 540
aaattcgcag ccaacagggt ctttggcttt gccattgtgg ctacctccac cctaaatatg 600
ttgatccctt cagcagcccg tgttcactat ggctgtgtca tcttcgtgag gatccttcag 660
ggattggtgg agggggtcac ataccctgct tgccatggca tctggagcaa atgggcccct 720

cccttagaac ggagtcggct ggcgacgaca gccttttgcg gttcctatgc cggggcagtg 780
gttgccatgc ctctggctgg ggtcctggta cagtattcag gatggagttc tgtcttctat 840
gtctatggca gcttcgggat cttttggtac ctgttctggt tgcttgtctc ctacgagtca 900
cctgcactac accccagcat ctccgaggag gagcgcaaat acattgagga tgccatcgga 960
gaaagcgcca agctcatgaa ccctgttacg aagtttaaca caccctggag gcgcttcttt 1020
acctccatgc cggtctatgc catcattgtc gccaactttt gccgcagctg gactttctac 1080
ctgctcctca tctcccagcc cgcctacttt gaagaagtgt tcggctttga gatcagcaag 1140
gtgggactgg tgtcggcact gcctcacctt gtcatgacta tcatcgtacc catcggaggc 1200
cagatcgccg acttcctgcg cagtcgtcat ataatgtcca cgaccaatgt gcgaaagctg 1260
atgaactgcg ggggtttcgg gatggaagct acgctgctgc tggtggtcgg atactcacac 1320
tccaagggcg tggccatctc cttcctggtc ctggctgtgg gcttcagtgg ctttgctatc 1380
tctgggttta acgtgaacca cttggacatc gcccctcgat atgccagcat cttgatgggc 1440
atttccaatg gcgtgggcac actgtctggg atggtgtgcc ccatcatcgt gggtgcaatg 1500
accaagcaca agacgcggga ggagtggcag tacgtgttcc tcatagcctc cctggtgcac 1560
tatggaggtg tcatcttcta tggggtcttt gcttcgggag agaaacagcc gtgggcagag 1620
ccggaggaga tgagcgagga gaagtgtggc tttgttggcc acgaccagct ggctggcagt 1680
gacgaaagtg aaatggaaga cgaggttgag cccccggggg caccccccgc acctccgcct 1740
tcctacgggg ccacacacag cacagttcag cctccaaggc ccccaccccc tgtccgggac 1800
tactgaccac gtgcctccca ctggtgggca gtttccagga cctccactcg atacacctct 1860
agcctaaacg gcagtgtcga ggaaccccac tcctctcctg cctcaggctt aagatgcaag 1920
tcttcccttg tgcccagtgc tgtccgacca gccctctctc cttctcaact gcctcttgca 1980
ggggtgaagc tgcacactag cagtttcaag ctcgtgccga attc 2024

<210> 6
<211> 560
<212> PRT
<213> Rattus norvegicus

<400> 6
Met Glu Phe Arg Gln Glu Glu Phe Arg Lys Leu Ala Gly Arg Ala Leu
1               5                   10                  15

Gly Arg Leu His Arg Leu Leu Glu Lys Arg Gln Glu Gly Ala Glu Thr
            20                  25                  30

Leu Glu Leu Ser Ala Asp Gly Arg Pro Val Thr Thr His Thr Arg Asp
        35                  40                  45

Pro Pro Val Val Asp Cys Thr Cys Phe Gly Leu Pro Arg Arg Tyr Ile
    50                  55                  60

Ile Ala Ile Met Ser Gly Leu Gly Phe Cys Ile Ser Phe Gly Ile Arg
65                  70                  75                  80

Cys Asn Leu Gly Val Ala Ile Val Ser Met Val Asn Asn Ser Thr Thr
                85                  90                  95

His Arg Gly Gly His Val Val Val Gln Lys Ala Gln Phe Asn Trp Asp
            100                 105                 110

Pro Glu Thr Val Gly Leu Ile His Gly Ser Phe Phe Trp Gly Tyr Ile
        115                 120                 125

Val Thr Gln Ile Pro Gly Gly Phe Ile Cys Gln Lys Phe Ala Ala Asn
    130                 135                 140

Arg Val Phe Gly Phe Ala Ile Val Ala Thr Ser Thr Leu Asn Met Leu
145                 150                 155                 160

Ile Pro Ser Ala Ala Arg Val His Tyr Gly Cys Val Ile Phe Val Arg
                165                 170                 175

Ile Leu Gln Gly Leu Val Glu Gly Val Thr Tyr Pro Ala Cys His Gly
            180                 185                 190

Ile Trp Ser Lys Trp Ala Pro Pro Leu Glu Arg Ser Arg Leu Ala Thr
        195                 200                 205

Thr Ala Phe Cys Gly Ser Tyr Ala Gly Ala Val Val Ala Met Pro Leu
    210                 215                 220

Ala Gly Val Leu Val Gln Tyr Ser Gly Trp Ser Ser Val Phe Tyr Val
225                 230                 235                 240

Tyr Gly Ser Phe Gly Ile Phe Trp Tyr Leu Phe Trp Leu Leu Val Ser
                245                 250                 255

Tyr Glu Ser Pro Ala Leu His Pro Ser Ile Ser Glu Glu Glu Arg Lys
            260                 265                 270

Tyr Ile Glu Asp Ala Ile Gly Glu Ser Ala Lys Leu Met Asn Pro Val
        275                 280                 285

Thr Lys Phe Asn Thr Pro Trp Arg Arg Phe Phe Thr Ser Met Pro Val
    290                 295                 300

Tyr Ala Ile Ile Val Ala Asn Phe Cys Arg Ser Trp Thr Phe Tyr Leu
305                 310                 315                 320

Leu Leu Ile Ser Gln Pro Ala Tyr Phe Glu Glu Val Phe Gly Phe Glu
                325                 330                 335

Ile Ser Lys Val Gly Leu Val Ser Ala Leu Pro His Leu Val Met Thr
            340                 345                 350

Ile Ile Val Pro Ile Gly Gly Gln Ile Ala Asp Phe Leu Arg Ser Arg
        355                 360                 365

His Ile Met Ser Thr Thr Asn Val Arg Lys Leu Met Asn Cys Gly Gly
    370                 375                 380

Phe Gly Met Glu Ala Thr Leu Leu Leu Val Val Gly Tyr Ser His Ser
385                 390                 395                 400

Lys Gly Val Ala Ile Ser Phe Leu Val Leu Ala Val Gly Phe Ser Gly

405                 410                 415

Phe Ala Ile Ser Gly Phe Asn Val Asn His Leu Asp Ile Ala Pro Arg
            420                 425                 430

Tyr Ala Ser Ile Leu Met Gly Ile Ser Asn Gly Val Gly Thr Leu Ser
        435                 440                 445

Gly Met Val Cys Pro Ile Ile Val Gly Ala Met Thr Lys His Lys Thr
    450                 455                 460

Arg Glu Glu Trp Gln Tyr Val Phe Leu Ile Ala Ser Leu Val His Tyr
465                 470                 475                 480

Gly Gly Val Ile Phe Tyr Gly Val Phe Ala Ser Gly Glu Lys Gln Pro
                485                 490                 495

Trp Ala Glu Pro Glu Glu Met Ser Glu Glu Lys Cys Gly Phe Val Gly
            500                 505                 510

His Asp Gln Leu Ala Gly Ser Asp Glu Ser Glu Met Glu Asp Glu Val
        515                 520                 525

Glu Pro Pro Gly Ala Pro Pro Ala Pro Pro Pro Ser Tyr Gly Ala Thr
    530                 535                 540

His Ser Thr Val Gln Pro Pro Arg Pro Pro Pro Pro Val Arg Asp Tyr
545                 550                 555                 560

<210> 7
<211> 3946
<212> DNA
<213> Homo sapiens

<400> 7
cgtttaaaag ccatcagatt tgagagcaat aagtcttcaa aaccgggaat ttacattgtt 60
tttcagctga ccgacttcca ggaaaaggac tcaaccgcat ctacccaaat accgtggcac 120
tgcttgcgct ctttgccacc ggatactccc cttccaatga gactttctga ttgtgtctac 180
caactctcct attaggaaac ccgtgggttg catgcagcta ttctgttgta ttctcattct 240
cactctccct cccttctctc actctcactc ttgctggagg cgagccacta ccattctgct 300
gagaaggaaa agcccgcaac tactttaaga gattaagaca atatgcgcaa tcctcgcctt 360
tcctagcaat cactatttaa atctggcaag aactgacaac agtctttgca agaatggaat 420
ccgtaaaaca aaggattttg gccccaggaa aagaggggct aaagaatttt gctggaaaat 480
cactcggcca gatctacagg gtgctggaga agaagcaaga caccggggag acaatcgagc 540
tgacggagga tgggaagccc ctagaggtgc ccgagaggaa ggcgccgctg tgcgactgca 600
cgtgcttcgg cctgccccgc cgctacatta tcgccatcat gagcggcctg ggcttctgca 660
tctccttcgg tatccgctgc aacctgggcg tggccattgt ggacatggtc aacaacagca 720
ccatccaccg cgggggcaag gtcatcaagg agaaagccaa attcaactgg gacccggaaa 780

ccgtggggat gatccacggt tccttctttt ggggctacat catcactcag attccgggag 840
gctacatcgc gtctcggctg gcagccaaca gggttttcgg agctgccata cttcttacct 900
ctaccctaaa tatgctaatt ccatcagcag ccagagtgca ttatggatgt gtcatctttg 960
tcagaatact gcagggactt gttgagggtg tgacctaccc agcatgtcat gggatatgga 1020
gcaaatgggc cccacctcta gagaggagta gactggcaac cacctccttt tgtggttcct 1080
atgccggagc tgtgattgca atgcctttag ctggcattct tgtgcagtac actggctggt 1140
cttcagtgtt ttatgtctac ggaagctttg gaatggtctg gtacatgttt tggcttttgg 1200
tgtcttatga aagtcctgca aagcatccta ctattacaga tgaagaacgt aggtacatag 1260
aagaaagcat tggagagagt gcaaatcttt taggtgcaat ggaaaaattc aagactccat 1320
ggaggaagtt ttttacatcc atgccagtct atgcaataat tgttgcaaac ttctgcagaa 1380
gctggacttt ttatttattg cttattagtc agccagcata ttttgaggaa gtctttggat 1440
ttgaaattag caaggttggt atgctatctg ctgtgccaca cttagtaatg acaattattg 1500
tgcctattgg gggacaaatt gcagatttcc taagaagcaa gcagattctt tcaactacga 1560
cagtgagaaa gatcatgaat tgtggtggtt ttggcatgga agccacactg ctcctggtcg 1620
ttggctattc tcatactaga ggggtagcaa tctcattctt ggtacttgca gtgggattca 1680
gtggatttgc tatatctggt ttcaatgtta accacttgga tatcgctcca agatatgcca 1740
gtatcttaat gggcatttcg aatggtgttg gcacattgtc aggaatggtt tgtcctatca 1800
ttgttggtgc aatgacaaag aataagtcac gtgaagagtg gcagtatgtc ttcctgatcg 1860
ctgccctagt ccactatggt ggagttatat tttatgcaat atttgcctca ggagagaaac 1920
aaccctgggc agacccggag gaaacaagtg aagaaaaatg tggatttatt catgaagatg 1980
aactcgatga agaaacaggg gacattactc aaaattatat aaattatggt accaccaagt 2040
cttatggtgc cacaacacag gccaatggag gttggcctag tggttgggaa aagaaagagg 2100
aatttgtaca aggagaagta caagactcac atagctataa ggaccgagtt gattattcat 2160
aacaaaacta attactggat ttatttttag tgtttgtgat taaattcatt gtgattgcac 2220
aaaaatttta aaaacacgtg atgtaaactt gcaagcatat caaccaggca agtcttgctg 2280
taaaaatgaa aacaaaacaa acccatgagg ttaccatcaa gtgcaatctg taaaattgtg 2340
aagttccatc atttccattc aagtcatcca ttcttgcatt tgtgacttaa aggttgactg 2400
gtcaaaattg tagaaacaag tagttaccca ttggattcat atgagctaaa actcatcact 2460
atttactaaa gcacaacatc tcatcctaca aaagttaaga agccaaagct acttgatcat 2520
gcaaaatgca cttatatatt tgttacactg tattgcaaga tagcacacag aagttggctg 2580
cgtcaagtag aggcgacatt tattaagtga aaatcatgga gttgggatat ctctcaatta 2640
aagaaataca ttgtgaacta tcagctacaa agttgtactg aataactatt agaattgcat 2700
aatgtgagat attttgttag tcctcaaaag gaatatcttg cagtgttttc tatgaaatgc 2760
ttgggcacaa acacttattt ctgtgaaaga gaacatgtaa gttgaggggt atgcttcatg 2820
ttcttccatc catttaccta atagtatgaa acagttcaca tttcaataaa atcaaacttt 2880
tcatgtagcg tatcacataa cttttttgca aaaaatataa aaagaaataa acttcaatgt 2940
attttttatt acaactttgt actggttgta acttgcatta gaaaaaaaaa agagatatat 3000
aaaccacaaa gaatctaata agaaatttat tatggagata tagcccttaa aatgcaatat 3060
taagaacaaa gaaatagaaa atggtttaga tatctttctt ccttcataat taaatactat 3120
atgaaacttg tgccacagag ctatatgtaa tatgaaaaga ttaacttcat agagatattg 3180
taagtaggta attttattat ttaaagtcct attaagaaat atttgtctta aatatatagg 3240
acaatacatt atattaaaat ggtctctctc tatatatatc tgtatatctt atacatgtcc 3300
atacacagaa acataataaa caatcttcac acgaaaccaa aaatagcata cacctaatgt 3360
tgggttaggg aattgcaatt tctactttca tagagtcata gaattttagg tggggaagag 3420
gcattttgct tgtcatttct taatataact caacaagaat tgcaacattt gtgtaccaag 3480
caataagtgc aatgcataaa atttcctgtc tgtatattac cttcattttg cttgtagtag 3540
ctgtttgggt ggttggaata attttatttt tcttttaaaa aagctaacat cagacccctt 3600
tataatgtcc taaaattatg ataatacatt tcccaattca actcaaaata ttattggtgt 3660
attttgtcta ttctggatat ttgatctgtt taatgtactg tgctagtgac tggaggccct 3720
gctactgcaa atataaaacc taaagtttgt ttaaaaaaat gcaaatcatt ctttacctta 3780
agaaaaaaaa aatacccttt gctttgtgcc tcaaagtgat gtaatgtgat cacagctttt 3840
gttgtgttga atgaaaatat gtggactgtc attttgttgc agcaaaaaag tgttaataaa 3900
atgctctatt tatccttttt taaaaaaaaa aaaaaaaaaa aaaaaa 3946

<210> 8
<211> 582
<212> PRT
<213> Homo sapiens

<400> 8
Met Glu Ser Val Lys Gln Arg Ile Leu Ala Pro Gly Lys Glu Gly Leu
1               5                   10                  15

Lys Asn Phe Ala Gly Lys Ser Leu Gly Gln Ile Tyr Arg Val Leu Glu
            20                  25                  30

Lys Lys Gln Asp Thr Gly Glu Thr Ile Glu Leu Thr Glu Asp Gly Lys
        35                  40                  45

Pro Leu Glu Val Pro Glu Arg Lys Ala Pro Leu Cys Asp Cys Thr Cys
    50                  55                  60

Phe Gly Leu Pro Arg Arg Tyr Ile Ile Ala Ile Met Ser Gly Leu Gly
65                  70                  75                  80

Phe Cys Ile Ser Phe Gly Ile Arg Cys Asn Leu Gly Val Ala Ile Val
                85                  90                  95

Asp Met Val Asn Asn Ser Thr Ile His Arg Gly Gly Lys Val Ile Lys
            100                 105                 110

Glu Lys Ala Lys Phe Asn Trp Asp Pro Glu Thr Val Gly Met Ile His
        115                 120                 125

Gly Ser Phe Phe Trp Gly Tyr Ile Ile Thr Gln Ile Pro Gly Gly Tyr
    130                 135                 140

Ile Ala Ser Arg Leu Ala Ala Asn Arg Val Phe Gly Ala Ala Ile Leu
145                 150                 155                 160

Leu Thr Ser Thr Leu Asn Met Leu Ile Pro Ser Ala Ala Arg Val His
                165                 170                 175

Tyr Gly Cys Val Ile Phe Val Arg Ile Leu Gln Gly Leu Val Glu Gly
            180                 185                 190

Val Thr Tyr Pro Ala Cys His Gly Ile Trp Ser Lys Trp Ala Pro Pro
        195                 200                 205

Leu Glu Arg Ser Arg Leu Ala Thr Thr Ser Phe Cys Gly Ser Tyr Ala
    210                 215                 220

Gly Ala Val Ile Ala Met Pro Leu Ala Gly Ile Leu Val Gln Tyr Thr
225                 230                 235                 240

Gly Trp Ser Ser Val Phe Tyr Val Tyr Gly Ser Phe Gly Met Val Trp
                245                 250                 255

Tyr Met Phe Trp Leu Leu Val Ser Tyr Glu Ser Pro Ala Lys His Pro
            260                 265                 270

Thr Ile Thr Asp Glu Glu Arg Arg Tyr Ile Glu Glu Ser Ile Gly Glu
        275                 280                 285

Ser Ala Asn Leu Leu Gly Ala Met Glu Lys Phe Lys Thr Pro Trp Arg
    290                 295                 300

Lys Phe Phe Thr Ser Met Pro Val Tyr Ala Ile Ile Val Ala Asn Phe
305                 310                 315                 320

Cys Arg Ser Trp Thr Phe Tyr Leu Leu Leu Ile Ser Gln Pro Ala Tyr
                325                 330                 335

Phe Glu Glu Val Phe Gly Phe Glu Ile Ser Lys Val Gly Met Leu Ser
            340                 345                 350

Ala Val Pro His Leu Val Met Thr Ile Ile Val Pro Ile Gly Gly Gln
        355                 360                 365

Ile Ala Asp Phe Leu Arg Ser Lys Gln Ile Leu Ser Thr Thr Thr Val
    370                 375                 380

Arg Lys Ile Met Asn Cys Gly Gly Phe Gly Met Glu Ala Thr Leu Leu
385                 390                 395                 400

Leu Val Val Gly Tyr Ser His Thr Arg Gly Val Ala Ile Ser Phe Leu
                405                 410                 415

Val Leu Ala Val Gly Phe Ser Gly Phe Ala Ile Ser Gly Phe Asn Val
            420                 425                 430

Asn His Leu Asp Ile Ala Pro Arg Tyr Ala Ser Ile Leu Met Gly Ile
        435                 440                 445

Ser Asn Gly Val Gly Thr Leu Ser Gly Met Val Cys Pro Ile Ile Val
    450                 455                 460

Gly Ala Met Thr Lys Asn Lys Ser Arg Glu Glu Trp Gln Tyr Val Phe
465                 470                 475                 480

Leu Ile Ala Ala Leu Val His Tyr Gly Gly Val Ile Phe Tyr Ala Ile
                485                 490                 495

Phe Ala Ser Gly Glu Lys Gln Pro Trp Ala Asp Pro Glu Glu Thr Ser
            500                 505                 510

Glu Glu Lys Cys Gly Phe Ile His Glu Asp Glu Leu Asp Glu Glu Thr
        515                 520                 525

Gly Asp Ile Thr Gln Asn Tyr Ile Asn Tyr Gly Thr Thr Lys Ser Tyr
    530                 535                 540

Gly Ala Thr Thr Gln Ala Asn Gly Gly Trp Pro Ser Gly Trp Glu Lys
545                 550                 555                 560

Lys Glu Glu Phe Val Gln Gly Glu Val Gln Asp Ser His Ser Tyr Lys
                565                 570                 575

Asp Arg Val Asp Tyr Ser
            580

<210> 9
<211> 3982
<212> DNA
<213> Rattus norvegicus

<400> 9
agacagtaag gttcttttgc ttttttccct tacacaagga ttcgatgacg tttttggtca 60
atctgattaa aagacagcgg atttggttgc gttaagactt caaaaccggg aatttacgtt 120
gtttttcggt gaggtgactt ccagaacggg gactcatcag cacccgccca aataccacgg 180
cactgcgcgc gccctcggcc accggatcct ccccttccaa tgagactttg tgactgtgtg 240
taccaattct cctattagga aacccgtggg ctgaatgcag ctattccgtt gtactctctt 300
tctcgctctc cctcccctct ccaactcaca gccttgctga aaagctcatc tctgctgaga 360
agaaaacgtt ctaccttaac ctattaagac tatgcgcaga actcgccttt catagccatc 420
acaatttaaa tctggtaagg ctggacacga gtctttacaa gaatggagtc ggtaaaacaa 480
aggattttgg ccccggggaa agaggggata aagaattttg ctggaaaatc cctcggacag 540
atctacaggg tgctggagaa gaagcaggat aaccgagaga ccatcgagct gacagaggac 600
ggcaagcccc tggaggtgcc tgagaagaag gctccgctat gcgactgtac gtgcttcggc 660
ctgccgcgcc gctacatcat agccatcatg agcggcctcg gcttctgcat ctcctttggt 720
atccgctgta acctgggtgt ggccattgtg gacatggtca acaacagcac catccaccgg 780
ggtggcaaag ttatcaagga gaaagccaag tttaactggg accccgagac tgtggggatg 840
attcacgggt cgttcttctg gggctatatc atcacgcaga ttccgggcgg atacatcgca 900
tcgcgactgg ctgctaaccg ggtctttggg gctgccatac tgcttacctc taccctcaat 960
atgctgatcc catctgcagc cagagtgcat tatggatgcg tcatctttgt tagaatattg 1020
caaggacttg tggagggcgt cacctaccca gcctgtcacg ggatatggag caagtgggcc 1080
cctcctttgg agaggagtag gttggctacc acctccttct gtggttccta tgctggagca 1140
gtcattgcaa tgcccctagc tggtatcctg gtgcagtaca ctggatggtc ttcagtattt 1200
tacgtatatg gaagctttgg tatggtctgg tatatgttct ggcttctggt gtcttacgag 1260
agccccgcaa agcatccaac cataacagac gaagaacgta ggtacataga agagagcatc 1320
ggggagagcg caaatctgtt aggagcaatg gagaaattca agaccccatg gaggaagttt 1380
ttcacatcca tgcccgtcta tgcgataatt gttgcaaact tctgcaggag ttggactttt 1440
tatttactgc tcatcagtca accagcttat ttcgaggagg tttttggatt tgaaatcagc 1500
aaggttggca tgttgtctgc ggtcccacac ctggtcatga caatcattgt gcctatcggg 1560
gggcaaattg cagactttct aaggagcaag caaattcttt caacaactac agtgcgaaag 1620
atcatgaact gcgggggttt tggcatggaa gccacactgc ttctggttgt tggctactct 1680
catactagag gggtggccat ctccttcttg gtgcttgcag tgggattcag tggatttgct 1740
atctctggtt tcaatgtgaa ccacttggat attgccccga gatatgccag tatcttaatg 1800
ggcatttcaa atggtgttgg cacgctgtcg ggaatggtct gcccgatcat tgttggtgca 1860
atgacgaaga acaagtcccg tgaagaatgg cagtatgtct tcctcatcgc tgcactggtc 1920

cactatggtg gagtcatatt ttatgcacta tttgcctcag gagagaagca accttgggca 1980
gaccctgagg aaacaagcga agaaaagtgt ggcttcattc atgaagatga actggatgaa 2040
gaaacggggg acatcactca gaattacata aattacggta ccaccaaatc ctacggcgcc 2100
acctcacagg agaacggagg ctggcctaac ggctgggaga aaaaggaaga atttgtgcaa 2160
gaaagtgcgc aagacgcgta ctcctataag gaccgagatg attattcata acgaagctag 2220
ttgctggatt cctttgtagt gtttgtgatt aaattaattg tgattgcaca aaatcatttt 2280
aagaaatgtg gtgtaaacat gtaaacacat caaccaagca agtcttgctg ttcaaaaaat 2340
aataataata tgaattcaaa acagaccgtg agagtcccat caagtgcaat ctgtggcggc 2400
agtcacgtga cgccatttcc attcaggcca ttcgtccttt tcgtttgtga tttaaaggtt 2460
tcctgtagaa ataagtaggt attcgttgga tccatcacca cgttagagag tacaactaca 2520
acagttggca catgtcatcc tacggaagtt aggaagccaa agctactgga ttatgtgaac 2580
tgcatttatt tatttattac actggactgc aaaatatccc agggaaatcc tgtctagaga 2640
catagtagaa ctggaaagat ggctagattg ggtactgacg ataatcattg tgtgtatatc 2700
atggagtggc tatatctttt aattggagaa ctatattgta tagctagcaa aattgtactg 2760
aattattact aggagtgcac agtgtgtgat attttgtgat cttccaaaag cttatcttgc 2820
agtgttttgt gaaacgcttg ggcacaaaca cttattttta tgaacaagag cttgtaaagg 2880
gaggagtatg ctccatgctc tcccattcac tacctgacag tatcaaacct tcacatttca 2940
atgaaatcca acgtccatgt aacatatcac atgacttttt ttgcaaaaaa gaatataaga 3000
agaaatagac ttcaatgtat tttttattac aactttgtac tggttgtaac ttgcattagg 3060
aaaaatgatt aatatatgta taatcgtaaa gaatctaata aaaatttact atgaagatat 3120
agcccttaaa atgcaatatt aacaacaaaa atatatagaa aatttagata atcttccttg 3180
ataactagag actatatgga actcacacca caaagctata tataatatga aaagataaac 3240
aatagagatt gtatatgtag acgattttat gacctaatgt cccatttaag aggtatttgt 3300
cttgagtata tagtacaaag tatattaaaa ttatatctac atccctgtat atcttataca 3360
tatccactca cacaaacata acaaatactt ttcacacaga accaaaaaca agcatacacc 3420
taatgttggg tttggggatt gcaatttcta ctttcataga gtcatagaat tttagatggg 3480
aaaaaaaaag gcattttgct cgtcatttct taatataatt aattcaacag gaactgcaac 3540
atttgtgtac caagcaataa gtgcgaagca taaacctgct gtgtgtaaac tatccccata 3600
ctgcttgtgg tagcactgat ttctttcttt taaagaactt aacatcggag ctctttacaa 3660
tgttttgcgc tgataagaat gcacatccca atttaacgca aagtgtcacc tggtgtgttt 3720
acctgtctgt tttgggtatt tggtctgttt ggtgtcctgt gctcttgact ggaggccctg 3780
ctactgcgaa tataaaacgt gaagtttgtt tctaaatgca aaccactcct gaccttaaga 3840
aactaaagtc cctctctgct ttgtgtctcc aagtactatc atgtgaccat aacccttgct 3900
gtgctgagta aaaagatgtg aactgtcatt ttgttgctgc gaagcaagtg ttaataaaat 3960
gttctattta aaaaaaaaaa aa 3982

<210> 10
<211> 582
<212> PRT
<213> Rattus norvegicus

<400> 10
Met Glu Ser Val Lys Gln Arg Ile Leu Ala Pro Gly Lys Glu Gly Ile
1               5                   10                  15

Lys Asn Phe Ala Gly Lys Ser Leu Gly Gln Ile Tyr Arg Val Leu Glu
            20                  25                  30

Lys Lys Gln Asp Asn Arg Glu Thr Ile Glu Leu Thr Glu Asp Gly Lys
        35                  40                  45

Pro Leu Glu Val Pro Glu Lys Lys Ala Pro Leu Cys Asp Cys Thr Cys

50                  55                  60

Phe Gly Leu Pro Arg Arg Tyr Ile Ile Ala Ile Met Ser Gly Leu Gly
65                  70                  75                  80

Phe Cys Ile Ser Phe Gly Ile Arg Cys Asn Leu Gly Val Ala Ile Val
                85                  90                  95

Asp Met Val Asn Asn Ser Thr Ile His Arg Gly Gly Lys Val Ile Lys
            100                 105                 110

Glu Lys Ala Lys Phe Asn Trp Asp Pro Glu Thr Val Gly Met Ile His
        115                 120                 125

Gly Ser Phe Phe Trp Gly Tyr Ile Ile Thr Gln Ile Pro Gly Gly Tyr
    130                 135                 140

Ile Ala Ser Arg Leu Ala Ala Asn Arg Val Phe Gly Ala Ala Ile Leu
145                 150                 155                 160

Leu Thr Ser Thr Leu Asn Met Leu Ile Pro Ser Ala Ala Arg Val His
                165                 170                 175

Tyr Gly Cys Val Ile Phe Val Arg Ile Leu Gln Gly Leu Val Glu Gly
            180                 185                 190

Val Thr Tyr Pro Ala Cys His Gly Ile Trp Ser Lys Trp Ala Pro Pro
        195                 200                 205

Leu Glu Arg Ser Arg Leu Ala Thr Thr Ser Phe Cys Gly Ser Tyr Ala
    210                 215                 220

Gly Ala Val Ile Ala Met Pro Leu Ala Gly Ile Leu Val Gln Tyr Thr
225                 230                 235                 240

Gly Trp Ser Ser Val Phe Tyr Val Tyr Gly Ser Phe Gly Met Val Trp
                245                 250                 255

Tyr Met Phe Trp Leu Leu Val Ser Tyr Glu Ser Pro Ala Lys His Pro
            260                 265                 270

Thr Ile Thr Asp Glu Glu Arg Arg Tyr Ile Glu Glu Ser Ile Gly Glu
        275                 280                 285

Ser Ala Asn Leu Leu Gly Ala Met Glu Lys Phe Lys Thr Pro Trp Arg
    290                 295                 300

Lys Phe Phe Thr Ser Met Pro Val Tyr Ala Ile Ile Val Ala Asn Phe
305                 310                 315                 320

Cys Arg Ser Trp Thr Phe Tyr Leu Leu Leu Ile Ser Gln Pro Ala Tyr
                325                 330                 335

Phe Glu Glu Val Phe Gly Phe Glu Ile Ser Lys Val Gly Met Leu Ser
            340                 345                 350

Ala Val Pro His Leu Val Met Thr Ile Ile Val Pro Ile Gly Gly Gln
        355                 360                 365

Ile Ala Asp Phe Leu Arg Ser Lys Gln Ile Leu Ser Thr Thr Thr Val
    370                 375                 380

Arg Lys Ile Met Asn Cys Gly Gly Phe Gly Met Glu Ala Thr Leu Leu
385                 390                 395                 400

Leu Val Val Gly Tyr Ser His Thr Arg Gly Val Ala Ile Ser Phe Leu
                405                 410                 415

Val Leu Ala Val Gly Phe Ser Gly Phe Ala Ile Ser Gly Phe Asn Val
            420                 425                 430

Asn His Leu Asp Ile Ala Pro Arg Tyr Ala Ser Ile Leu Met Gly Ile
        435                 440                 445

Ser Asn Gly Val Gly Thr Leu Ser Gly Met Val Cys Pro Ile Ile Val
    450                 455                 460

Gly Ala Met Thr Lys Asn Lys Ser Arg Glu Glu Trp Gln Tyr Val Phe
465                 470                 475                 480

Leu Ile Ala Ala Leu Val His Tyr Gly Gly Val Ile Phe Tyr Ala Leu
                485                 490                 495

Phe Ala Ser Gly Glu Lys Gln Pro Trp Ala Asp Pro Glu Glu Thr Ser
            500                 505                 510

Glu Glu Lys Cys Gly Phe Ile His Glu Asp Glu Leu Asp Glu Glu Thr
        515                 520                 525

Gly Asp Ile Thr Gln Asn Tyr Ile Asn Tyr Gly Thr Thr Lys Ser Tyr
    530                 535                 540

Gly Ala Thr Ser Gln Glu Asn Gly Gly Trp Pro Asn Gly Trp Glu Lys
545                 550                 555                 560

Lys Glu Glu Phe Val Gln Glu Ser Ala Gln Asp Ala Tyr Ser Tyr Lys

                565                 570                 575

Asp Arg Asp Asp Tyr Ser
            580

<210> 11
<211> 2836

<212> DNA
<213> Mus musculus

<400> 11
cggccgcccg ggcccgcggg cggggggatt ggcaggggac ccgcgtgggc acagccacca 60
tggagttccg gcaggaggag tttcggaagc tggcggggcg cgccctgggg aggctgcacc 120
ggttactgga gaagcggcag gaaggcgcgg agacactgga gctgagtgcc gacgggcggc 180
cagtgaccac gcacactcgg gacccgcctg tggtggactg cacctgcttt ggcctccctc 240
gtcgctacat catcgccatc atgagcggtc tgggtttctg tatcagcttt ggcatccgct 300
gcaacctggg cgtggccatc gtgtccatgg tcaacaacag cacaacccac cgtgggggcc 360
acgtggtggt gcagaaagcc cagttcaact gggatccaga gactgtcggc ctcatacatg 420
gctccttttt ctggggctac attgtcactc agattcctgg aggatttatc tgccaaaaat 480
tcgcagccaa cagggtcttt ggctttgcca ttgtggctac ctccacccta aacatgttga 540
tcccttcagc agcccgcgtt cactatggct gtgtcatctt cgtgaggatc cttcagggat 600
tggtggaggg ggtcacatac cctgcttgcc atggcatctg gagcaaatgg gcccctccct 660
tagaacggag tcggctggca acgacagcct tttgcggttc ctatgctggg gcggtggttg 720
ccatgccctt ggctggggtc cttgtgcagt attcaggatg gagttctgtc ttctatgtct 780
atggcagctt cgggatcttt tggtacctgt tctggttgct tgtctcctat gagtcaccgg 840
cactgcaccc cagcatctct gaggaggagc gcaaatacat tgaggatgcc atcggggaga 900
gcgccaagct catgaaccct gttacgaagt ttaacacacc ctggaggcgc ttctttacgt 960
ccatgcccgt ctatgccatc atcgttgcga acttttgccg cagctggacc ttctacctgc 1020
tcctcatctc tcagcccgcc tactttgaag aagtgttcgg ctttgagatc agcaaggtgg 1080
ggctggtgtc ggcgctgcct caccttgtca tgaccatcat cgtacccatt ggaggccaga 1140
tcgctgactt tttgcgcagt cgtcacataa tgtccactac caacgtgcga aagctcatga 1200
actgcggggg tttcgggatg gaagccacgc tgctgctggt ggtcggatac tcgcactcca 1260
agggcgtggc catctccttc ctggtcctgg ctgtgggctt cagtggcttt gccatctctg 1320
ggtttaacgt gaaccacttg gacatcgccc ctcgctatgc cagcatcttg atgggcattt 1380
ccaatggcgt gggcacactg tctgggatgg tgtgccccat catcgtgggt gcaatgacca 1440
agcacaagac gcgggaggag tggcagtacg tgttcctcat agcctccctg gtgcactacg 1500
gcggtgtcat cttctatggg gtctttgctt cgggagagaa gcagccgtgg gcagagccgg 1560
aggagatgag cgaggagaag tgtggctttg ttggccacga ccagctggct ggcagtgacg 1620
aaagtgaaat ggaggacgag gctgagcccc caggggcgcc ccccgcgccg cctccgtcct 1680
acggggccac acacagcaca gtgcagcctc cgaggccccc gccccctgtc cgggactact 1740
gaccacgggc ctcccactgt ggggcagttt ccaggacttc cactccatac acctctagcc 1800
tgagcggcag tgtcgaggaa ccccactcct cccctgcctc aggcttaaga tgcaagtcct 1860
cccttgttcc cagtgctgtc cgaccagccc tctttccctc tcaactgcct cctgcggggg 1920
gtgaagctgc acactagcag tttcaaggat acccagactc ccctgaaagt cgttctccgc 1980
ttgtttctgc ctgtgtgggc tcaaatctcc cctttgaggg ctttatttgg agggacagtt 2040
caacctcttc ctctcttgtg gttttgaggt ttcacccctt cccccaagac cccagggatt 2100
ctcaggctac cccgagatta ttcaggtggt cccctactca gaagacttca tggtcgtcct 2160
ctattagttt caaggctcgc ctaaccaatt ctacattttt ccaagctggt ttaacctaac 2220
caccaatgcc gccgttccca ggactgattc tcaccagcgt ttctgaggga aaatggcggt 2280
ttcaagtccc cccacccccc ttttcttccc tcgtcccctc accagcacac tttgccgggc 2340
cttgacctta gcttagtaca atcattgtcc agggaaatgg ccaaaatggc tctgctcacc 2400
ccgtgctctt tttctgactc agttttcagg tctcagtagt ggctgcccaa agctattaat 2460
tcagcggctc gaggccacct cttcctcccc gtggtggttt caggatcccc ctcgcccccc 2520
cccccccaaa tccttgcact ttattctcct gggtggttcc aggccgccct cggtttctca 2580
gtggccattt gttgtgtgtc cctcaggggc taaatgattc caaatctggg gtgcttcccc 2640
tcatagacac ccctctctca acgtagaaat ctgggtgggg gtgaggtgtg tgagagaagt 2700
tacagaatcc caggaaaggg agcggggctg ggaggagagg gttgttcctg gggcagggtc 2760
gtgtcttggt gtctgtctct gtgacgtaaa tcctgccctg ccccacccca ctcccaataa 2820
acgctctggt gtacgg 2836

<210> 12
<211> 560
<212> PRT
<213> Mus musculus

<400> 12
Met Glu Phe Arg Gln Glu Glu Phe Arg Lys Leu Ala Gly Arg Ala Leu
1               5                   10                  15

Gly Arg Leu His Arg Leu Leu Glu Lys Arg Gln Glu Gly Ala Glu Thr
            20                  25                  30

Leu Glu Leu Ser Ala Asp Gly Arg Pro Val Thr Thr His Thr Arg Asp
        35                  40                  45

Pro Pro Val Val Asp Cys Thr Cys Phe Gly Leu Pro Arg Arg Tyr Ile
    50                  55                  60

Ile Ala Ile Met Ser Gly Leu Gly Phe Cys Ile Ser Phe Gly Ile Arg
65                  70                  75                  80

Cys Asn Leu Gly Val Ala Ile Val Ser Met Val Asn Asn Ser Thr Thr
                85                  90                  95

His Arg Gly Gly His Val Val Val Gln Lys Ala Gln Phe Asn Trp Asp
            100                 105                 110

Pro Glu Thr Val Gly Leu Ile His Gly Ser Phe Phe Trp Gly Tyr Ile
        115                 120                 125

Val Thr Gln Ile Pro Gly Gly Phe Ile Cys Gln Lys Phe Ala Ala Asn
    130                 135                 140

Arg Val Phe Gly Phe Ala Ile Val Ala Thr Ser Thr Leu Asn Met Leu
145                 150                 155                 160

Ile Pro Ser Ala Ala Arg Val His Tyr Gly Cys Val Ile Phe Val Arg
                165                 170                 175

Ile Leu Gln Gly Leu Val Glu Gly Val Thr Tyr Pro Ala Cys His Gly
            180                 185                 190

Ile Trp Ser Lys Trp Ala Pro Pro Leu Glu Arg Ser Arg Leu Ala Thr
        195                 200                 205

Thr Ala Phe Cys Gly Ser Tyr Ala Gly Ala Val Val Ala Met Pro Leu
    210                 215                 220

Ala Gly Val Leu Val Gln Tyr Ser Gly Trp Ser Ser Val Phe Tyr Val
225                 230                 235                 240

Tyr Gly Ser Phe Gly Ile Phe Trp Tyr Leu Phe Trp Leu Leu Val Ser
                245                 250                 255

Tyr Glu Ser Pro Ala Leu His Pro Ser Ile Ser Glu Glu Glu Arg Lys
            260                 265                 270

Tyr Ile Glu Asp Ala Ile Gly Glu Ser Ala Lys Leu Met Asn Pro Val
        275                 280                 285

Thr Lys Phe Asn Thr Pro Trp Arg Arg Phe Phe Thr Ser Met Pro Val
    290                 295                 300

Tyr Ala Ile Ile Val Ala Asn Phe Cys Arg Ser Trp Thr Phe Tyr Leu
305                 310                 315                 320

Leu Leu Ile Ser Gln Pro Ala Tyr Phe Glu Glu Val Phe Gly Phe Glu
                325                 330                 335

Ile Ser Lys Val Gly Leu Val Ser Ala Leu Pro His Leu Val Met Thr
            340                 345                 350

Ile Ile Val Pro Ile Gly Gly Gln Ile Ala Asp Phe Leu Arg Ser Arg
        355                 360                 365

His Ile Met Ser Thr Thr Asn Val Arg Lys Leu Met Asn Cys Gly Gly
    370                 375                 380

Phe Gly Met Glu Ala Thr Leu Leu Leu Val Val Gly Tyr Ser His Ser
385                 390                 395                 400

Lys Gly Val Ala Ile Ser Phe Leu Val Leu Ala Val Gly Phe Ser Gly
                405                 410                 415

Phe Ala Ile Ser Gly Phe Asn Val Asn His Leu Asp Ile Ala Pro Arg
            420                 425                 430

Tyr Ala Ser Ile Leu Met Gly Ile Ser Asn Gly Val Gly Thr Leu Ser
        435                 440                 445

Gly Met Val Cys Pro Ile Ile Val Gly Ala Met Thr Lys His Lys Thr
    450                 455                 460

Arg Glu Glu Trp Gln Tyr Val Phe Leu Ile Ala Ser Leu Val His Tyr
465                 470                 475                 480

Gly Gly Val Ile Phe Tyr Gly Val Phe Ala Ser Gly Glu Lys Gln Pro
                485                 490                 495

Trp Ala Glu Pro Glu Glu Met Ser Glu Glu Lys Cys Gly Phe Val Gly
            500                 505                 510

His Asp Gln Leu Ala Gly Ser Asp Glu Ser Glu Met Glu Asp Glu Ala
        515                 520                 525

Glu Pro Pro Gly Ala Pro Pro Ala Pro Pro Pro Ser Tyr Gly Ala Thr
    530                 535                 540

His Ser Thr Val Gln Pro Pro Arg Pro Pro Pro Pro Val Arg Asp Tyr
545                 550                 555                 560

<210> 13
<211> 2528
<212> DNA
<213> Mus musculus

<400> 13
ggcacgaggc tcagtcttaa ttccactctg ccactctgcc gcagagcaca attacgccgg 60
cgcgatggga ggagaccatg ttaaggcagg aagctaacag cagccgctca cctgaggcct 120
aggaagctcc caagggttct gagagctatg agctctgatc agcaaagtca ccattttaga 180
cagtagggtt cttttgcttt tttccttaca caagggttcg atgacgtttc tggtcaatct 240
gattaaaaga cagcggattt gattgcgata agacttcaaa accgggaatt tacgttgttt 300
ttcggtgagg tgacttccag aacagggact catcagcacc cgcccaaata ccacggcact 360
gcgcgcgccc tcggccaccg gatcctcccc ttccaatgag actttgtgac tgtgtgtacc 420
aattctccta ttaggaaacc cgtgggctgc atgcagctat tctgttgtac tctctttctc 480
gccctccctc ccctctccaa ctcacagcct tgctggaaag ctcacctctg ctgagaagaa 540
aaagctctac cttaaccaac taagactatg cgcagaatcc gtctttcata gccacaacaa 600
tttaaatctg gtaaggctgg acaccagtct ttacaagaat ggagtcggta aaacaaagga 660
ttttggcccc ggggaaagag gggataaaga attttgctgg aaaatccctc ggacagatct 720
acagggtgct ggagaagaag caggacaacc gagagaccat cgagctgaca gaggacggta 780
agcccctgga ggtgcctgag aagaaggctc cgctatgcga ctgcacgtgc ttcggcctgc 840
cgcgccgcta catcatagcc atcatgagcg gcctcggctt ctgcatatcc ttcggcatcc 900
gctgtaacct gggcgtggcc atcgtggaca tggtcaacaa cagcactatc caccgcggag 960
gcaaagttat caaggagaaa gccaaattta actgggaccc cgagaccgtg gggatgatcc 1020
acggatcgtt cttctggggc tatatcatca cccagattcc aggaggatat atcgcatcgc 1080
ggctggctgc taaccgggtc tttggggctg cgatactgct cacctctacc ctcaatatgc 1140
tgatcccatc tgcagccaga gtgcattatg gatgtgtcat ctttgttagg atattgcaag 1200
gacttgtgga gggtgtcacc tacccagcct gtcatgggat atggagcaag tgggcccctc 1260
ccttggagag gagtaggttg gctacaacct ccttttgtgg ttcctatgct ggagcagtca 1320
ttgcaatgcc cttagctggt atccttgtgc agtacactgg atggtcgtca gtattttatg 1380
tgtatggaag ctttggcatg gtctggtaca tgttctggct tctggtgtct tatgagagcc 1440
ctgcaaagca tcctaccatt acagatgaag aacgtaggta catagaggag agcattggag 1500
agagcgcaaa tctgctaggt gcaatggaaa aatttaagac cccatggagg aagtttttca 1560
catccatgcc cgtctacgcg ataattgttg ccaacttctg caggagctgg actttttatt 1620
tactgctcat cagtcagcca gcttattttg aggaggtttt tggatttgaa atcagcaagg 1680

ttggcatgtt gtctgcagtc cctcaccttg tcatgacaat cattgtgcct atcggggggc 1740
aaattgcaga tttcctaagg agcaagcaaa ttctctcaac aactacagtg agaaagatca 1800
tgaattgtgg gggttttggc atggaagcca cgctgcttct ggttgttggc tactctcata 1860
ctagaggggt ggccatctcc ttcttggtgc ttgcagtagg attcagtgga tttgctatct 1920
ctggtttcaa tgttaatcac ttggatattg ctccaagata tgccagtatc ttaatgggca 1980
tttcaaatgg cgttggcacg ctgtcgggga tggtttgccc tatcattgtt ggtgcaatga 2040

caaagaataa gtcccgtgaa gaatggcagt atgtcttcct cattgctgca ctcgtccact 2100
atggtggagt catattttat gcactatttg cctcaggaga gaaacaacct tgggcagacc 2160
ctgaggaaac aagcgaagaa aaatgtggct tcattcacga agatgaactg gatgaagaaa 2220
cgggggacat cactcagaat tacataaatt acggtaccac caaatcttac ggtgctacct 2280
cacaggagaa tggaggctgg cctaacggct gggagaaaaa ggaagaattt gtgcaagaag 2340
gtgcgcaaga cgcgtacacc tataaggacc gagatgatta ttcataacga tgctagttgc 2400
tggattcatt tgtagtgttt gtgaatcaat taattgtgat tgcacaaaaa taattttaaa 2460
aatgtggtgt gaacatgtaa acatatcaac caagcaagtc ttgctgttca aaaaaaaaaa 2520
aaaaaaaa 2528

<210> 14
<211> 582
<212> PRT
<213> Mus musculus

<400> 14
Met Glu Ser Val Lys Gln Arg Ile Leu Ala Pro Gly Lys Glu Gly Ile
1               5                   10                  15

Lys Asn Phe Ala Gly Lys Ser Leu Gly Gln Ile Tyr Arg Val Leu Glu
            20                  25                  30

Lys Lys Gln Asp Asn Arg Glu Thr Ile Glu Leu Thr Glu Asp Gly Lys
        35                  40                  45

Pro Leu Glu Val Pro Glu Lys Lys Ala Pro Leu Cys Asp Cys Thr Cys
    50                  55                  60

Phe Gly Leu Pro Arg Arg Tyr Ile Ile Ala Ile Met Ser Gly Leu Gly
65                  70                  75                  80

Phe Cys Ile Ser Phe Gly Ile Arg Cys Asn Leu Gly Val Ala Ile Val
                85                  90                  95

Asp Met Val Asn Asn Ser Thr Ile His Arg Gly Gly Lys Val Ile Lys
            100                 105                 110

Glu Lys Ala Lys Phe Asn Trp Asp Pro Glu Thr Val Gly Met Ile His
        115                 120                 125

Gly Ser Phe Phe Trp Gly Tyr Ile Ile Thr Gln Ile Pro Gly Gly Tyr
    130                 135                 140

Ile Ala Ser Arg Leu Ala Ala Asn Arg Val Phe Gly Ala Ala Ile Leu
145                 150                 155                 160

Leu Thr Ser Thr Leu Asn Met Leu Ile Pro Ser Ala Ala Arg Val His
                165                 170                 175

Tyr Gly Cys Val Ile Phe Val Arg Ile Leu Gln Gly Leu Val Glu Gly
            180                 185                 190

Val Thr Tyr Pro Ala Cys His Gly Ile Trp Ser Lys Trp Ala Pro Pro
        195                 200                 205

Leu Glu Arg Ser Arg Leu Ala Thr Thr Ser Phe Cys Gly Ser Tyr Ala
    210                 215                 220

Gly Ala Val Ile Ala Met Pro Leu Ala Gly Ile Leu Val Gln Tyr Thr
225                 230                 235                 240

Gly Trp Ser Ser Val Phe Tyr Val Tyr Gly Ser Phe Gly Met Val Trp
                245                 250                 255

Tyr Met Phe Trp Leu Leu Val Ser Tyr Glu Ser Pro Ala Lys His Pro
            260                 265                 270

Thr Ile Thr Asp Glu Glu Arg Arg Tyr Ile Glu Glu Ser Ile Gly Glu
        275                 280                 285

Ser Ala Asn Leu Leu Gly Ala Met Glu Lys Phe Lys Thr Pro Trp Arg
    290                 295                 300

Lys Phe Phe Thr Ser Met Pro Val Tyr Ala Ile Ile Val Ala Asn Phe
305                 310                 315                 320

Cys Arg Ser Trp Thr Phe Tyr Leu Leu Leu Ile Ser Gln Pro Ala Tyr
                325                 330                 335

Phe Glu Glu Val Phe Gly Phe Glu Ile Ser Lys Val Gly Met Leu Ser
            340                 345                 350

Ala Val Pro His Leu Val Met Thr Ile Ile Val Pro Ile Gly Gly Gln
        355                 360                 365

Ile Ala Asp Phe Leu Arg Ser Lys Gln Ile Leu Ser Thr Thr Thr Val
    370                 375                 380

Arg Lys Ile Met Asn Cys Gly Gly Phe Gly Met Glu Ala Thr Leu Leu
385                 390                 395                 400

Leu Val Val Gly Tyr Ser His Thr Arg Gly Val Ala Ile Ser Phe Leu
                405                 410                 415

Val Leu Ala Val Gly Phe Ser Gly Phe Ala Ile Ser Gly Phe Asn Val
            420                 425                 430

Asn His Leu Asp Ile Ala Pro Arg Tyr Ala Ser Ile Leu Met Gly Ile
        435                 440                 445

Ser Asn Gly Val Gly Thr Leu Ser Gly Met Val Cys Pro Ile Ile Val
    450                 455                 460

Gly Ala Met Thr Lys Asn Lys Ser Arg Glu Glu Trp Gln Tyr Val Phe
465                 470                 475                 480

Leu Ile Ala Ala Leu Val His Tyr Gly Gly Val Ile Phe Tyr Ala Leu
                485                 490                 495

Phe Ala Ser Gly Glu Lys Gln Pro Trp Ala Asp Pro Glu Glu Thr Ser
            500                 505                 510

Glu Glu Lys Cys Gly Phe Ile His Glu Asp Glu Leu Asp Glu Glu Thr
        515                 520                 525

Gly Asp Ile Thr Gln Asn Tyr Ile Asn Tyr Gly Thr Thr Lys Ser Tyr
    530                 535                 540

Gly Ala Thr Ser Gln Glu Asn Gly Gly Trp Pro Asn Gly Trp Glu Lys
545                 550                 555                 560

Lys Glu Glu Phe Val Gln Glu Gly Ala Gln Asp Ala Tyr Thr Tyr Lys
                565                 570                 575

Asp Arg Asp Asp Tyr Ser
            580

## Beschreibung

Die Erfindung betrifft ein Verfahren zur Auffindung pharmazeutisch wirksamer Substanzen unter Verwendung von BNPI und/oder DNPI bzw. davon abgeleiteter Biomoleküle und die Verwendung dadurch identifizierter Verbindungen, an BNPI und/oder DNPI bindender Wirkstoffe, gegen BNPI und/oder DNPI gerichteter Antikörper, von Antisensenukleotiden gegen BNPI und/oder DNPI, oder von BNPI und/oder DNPI bzw. Teilproteinen davon, bzw. entsprechenden Polynukleotiden zur Herstellung von Arzneimitteln zur Behandlung verschiedener Erkrankungen oder zu Therapie und Diagnostik.

Das Auffinden der Angriffsorte pharmazeutischer Wirkstoffe, der sogenannten "Targets" ist eine der wichtigsten Aufgaben moderner Pharmaforschung. Über die Affinitäten an diesen Targets oder auch über die durch eine Wechselwirkung mit diesen Targets ausgelösten physiologischen Effekte lassen sich über sogenannte "Screeningverfahren" aus der Vielzahl von bekannter Substanzen, beispielsweise aus den Substanzbibliotheken der pharmazeutischen Forschung, interessante Substanzen oder Substanzklassen herausfiltern, die in den mit diesem Target assoziierten Indikationen mit hoher Wahrscheinlichkeit wirksam sind. Zu den wichtigsten Vertretern dieser Targets gehören Proteine, im allgemeinen Rezeptoren, insbesondere G-Protein gekoppelte Rezeptoren, und Transportproteine. Die Auffindung dieser Targets gestaltet sich aber teilweise sehr schwierig, da die potentielle Auswahl sehr groß ist. Zur Orientierung und Identifizierung dienen zum einen Erkenntnisse über die (physiologische) Funktion mit der (potentiellen) Position in Signalkaskaden und Stoffwechselwegen zum anderen aber auch Lokalisation und Expressionsgrad in den verschiedenen Geweben. Im Rahmen dieser Erfindung wurde dabei ein besonderes Augenmerk auf das zentrale Nervensystem gerichtet, wo es nicht nur auf eine generelle Lokalisation sondern auf eine sehr spezifische und präzise Verteilung in den verschiedenen Regionen ankommt.

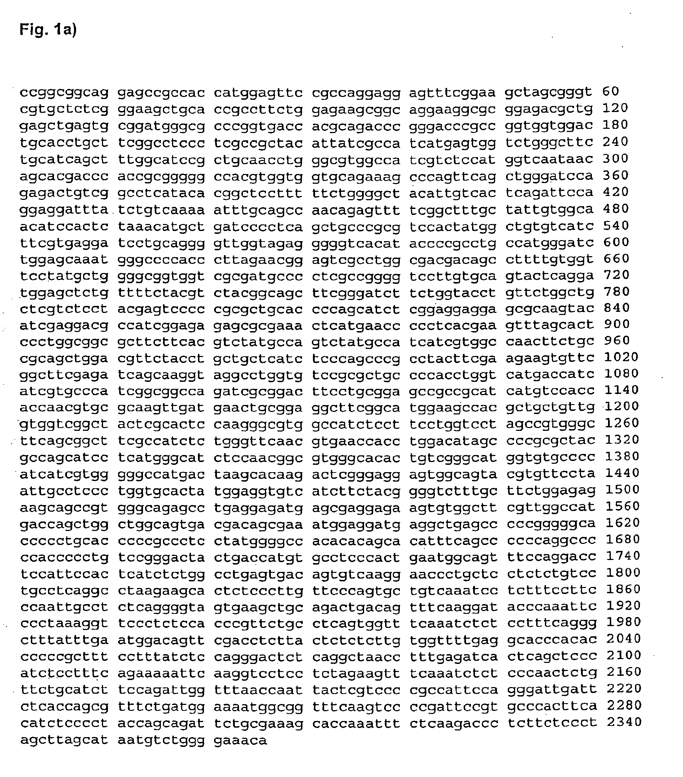

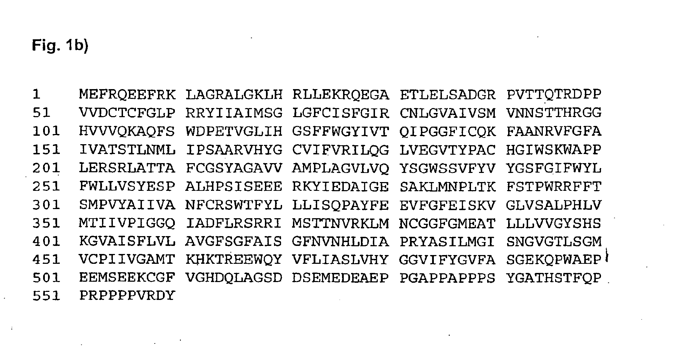

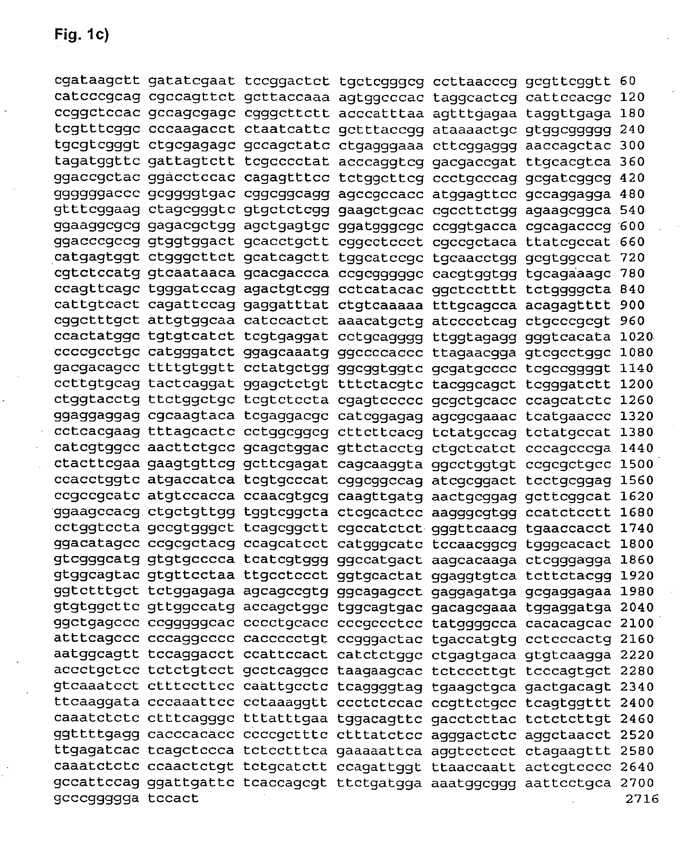

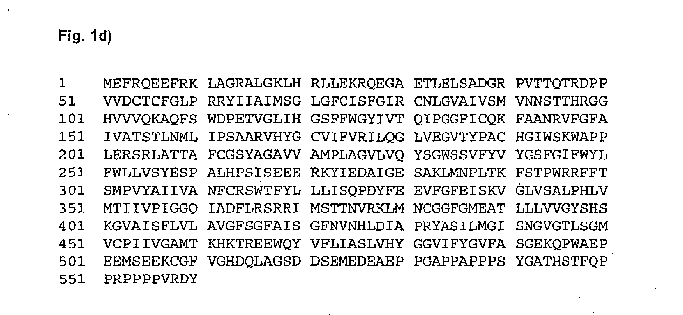

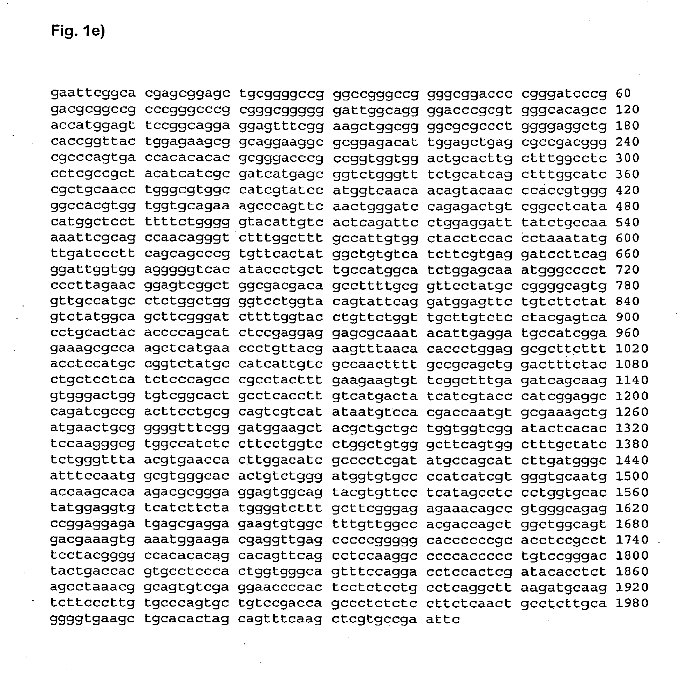

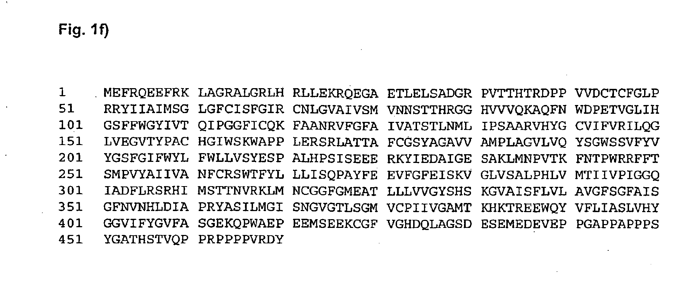

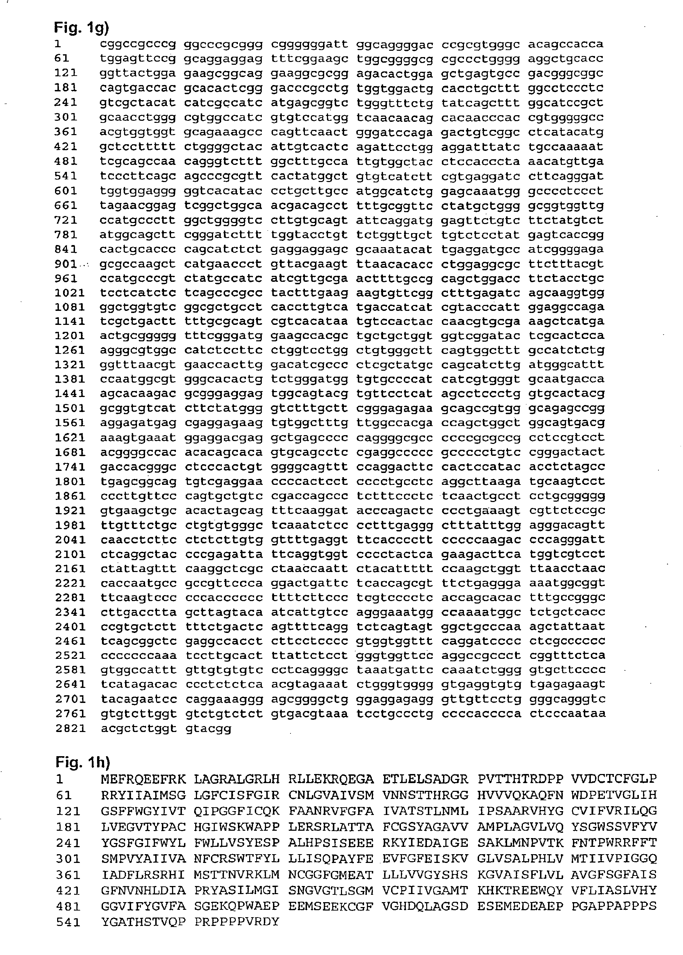

Aufgabe der Erfindung war daher die Auffindung und Identifizierung eines oder mehrerer derartiger Targets, insbesondere mit zentralnervöser Lokalisation und Wirksamkeit, und die Entwicklung eines entsprechenden Screeningverfahrens. Die Erfindung betrifft daher ein Verfahren zur Auffindung pharmazeutisch relevanter Substanzen mit Wirksamkeit in den Indikationen oder zur Behandlung von
Sehstörungen, Retinitis pigmentosa, Opticus Degeneration, Hörstörungen, Tinitus, M. Menière, Hörsturz, Schizophrenie, Manien, Depression, Schlaganfall, Hirntrauma, Querschnittslähmung, amyotrophe Lateralsklerose, Neuralgie, Gewichtsregulation, Obesitas, Anorexia nervosa, Epilepsie, Hemibalismus, Chorea Huntington, Stress, Morbus Parkinson, TIA (Transiente Ischämische Attacken), Emesis, insbesondere Hyperemesis beispielsweise bei Chemotherapie, Schwindel, in jeglicher Form, Katarakt, Arthritis, Hyperaktivität, Entwicklungsstörungen, Tollwut, Virus-Infektionen oder bakterielle Infektionen, Grippe, Malaria, Creutzfeld-Jacob, Inflammatory Bowel disease, Morbus Crohn, cardiovaskuläre und cardiorespiratorische Funktionsstörungen, Hypertonie, Störungen der Baroafferenz oder Chemoafferenz, Toxoplasmose, Asthma, Autoimmunität im zentralen und peripheren Nervensystem, diabetische Neuropathie, autoimmuner Diabetes, alkoholische Neuropathie, HIV-Neuro-Aids; Störungen des autonomen Nervensystems, Störungen des Nervensystems des Verdauungstraktes, Übererregbarkeit, insbesondere glutamatvermittelte Übererregbarkeit, Neurodegeneration insbesondere bei Morbus Alzheimer, Morbus Alzheimer, Ischämie; Encephalitis insbesondere virale oder bakterielle; Prionerkrankung, Rasmussen-Encephalitis, HIV-Encephalitis, Demyelinisierung insbesondere bei multipler Sklerose, Retinadegeneration, Glaukom, Nystagmus, Netzhautablösung, Erkrankungen des Cerebellums, cerebelläre Ataxie, Erkrankungen der Basalganglien, Erkrankungen des Pallidums, Erkrankungen des Hör- und/oder Gleichgewichtsorgans, Erkrankungen der Hörbahn oder Vesibularbahn, Störungen des Gedächtnisses, Störungen des Lernens, Störungen der Kognition, Stiff-Man-Syndrom, Restless Leg-Syndrom, Angstzustände, Phobien, Schlafstörungen; Drogenabhängigkeit, -sucht und -entzug insbesondere bei Alkohol, Nikotin, Opiaten, Ecstasy oder Kokain; Hepatoencephalopathie mit Alkoholintoxikation, Hepatoencephalopathie ohne Alkoholintoxikation, neurotoxikofogisch bedingte Erkrankungen, Erkrankungen des spinalen Motoneurons, Muskelatrophien, Muskeldystrophien, Erkrankungen der Hinterstrangbahnen, alkoholische Neuropathien, Neuroinflammation, Befindlichkeitsstörungen bei Infektionen oder Fieber, Stress, Geschmacksstörungen, Nahrungsmittelallergien, Chinese-Restaurant-Syndrom, Agression, Paranoia, Hirnerschütterung, neuroendokrine Störungen, Tourrette-Syndrom, cerebrovaskuläre Spasmen, neuronale Apoptose, Neurodegeneration, neuronale Nekrose, Astrocytose, Burn-out-Syndrom, Sudden-Infant-Death (plötzlicher Kindstod), Herzinfarkt, Insomnia, retrograde Amnesie, multiple Sklerose, Jet-lag, Störungen der Sexualfunktion, wie Impotenz oder Priapismus oder mit Wirksamkeit zur Förderung der Mikrogliaaktivierung, des Lernens, der Kognition oder des Gedächtnisses, zur Neuroprotektion, zur Liquordiagnostik neurostatischer Erkrankungen oder zur adjuvanten Therapie per Elektrostimulation des Nucleus subthalamicus bei Parkinson
mit folgenden Verfahrensschritten:
(a) Inkubation einer zu testenden Substanz unter geeigneten Bedingungen mit mindestens einem Biomolekül aus Gruppe I ausgewählt aus:
   dem Protein BNPI und/oder DNPI und/oder einem Protein gemäß einer der Abbildungen 1b), 1d), 1f), 1h), 2b), 2d) oder 2f) und/oder einem zu einem dieser vorgenannten Proteine zu mindestens 90 % ähnlichen Protein und/oder einem Protein, für das ein Polynukleotid gemäß einer der Abbildungen 1a), 1c), 1e), 1g), 2a), 2c) oder 2e) oder ein dazu zu mindestens 90 % ähnliches Polynukleotid kodiert, und/oder einem Protein, das durch eine Nukleinsäure kodiert wird, die unter stringenten Bedingungen an ein Polynukleotid gemäß einer der Abbildungen 1a), 1c), 1e), 1g), 2a), 2c) oder 2e) oder deren Antisense Polynukleotide bindet, oder einem mindestens 10, vorzugsweise mindestens 15, insbesondere mindestens 20 Aminosäuren langen Teilprotein eines der vorgenannten Proteine
   und/oder einer Zelle und/oder einer Präparation aus einer solchen Zelle, die mindestens eines der vorgenannten Proteine und Teilproteine, bzw. Biomoleküle aus Gruppe I, synthetisiert hat,
(b) Messung der Bindung der Testsubstanz an dem oder den gegebenenfalls von einer Zelle synthetisierten Biomolekül/en aus Gruppe I oder an einer Zelle und/oder einer Präparation aus einer solchen Zelle, die mindestens eines der vorgenannten Biomoleküle aus Gruppe I synthetisiert hat, oder Messung mindestens eines der durch die Bindung der Testsubstanz an dem oder den gegebenenfalls von einer Zelle synthetisierten Biomolekül/en aus Gruppe I oder an einer Zelle und/oder einer Präparation aus einer solchen Zelle, die mindestens eines der vorgenannten Biomoleküle aus Gruppe I synthetisiert hat, veränderten funktionellen Parameter.

Dieses neue Screeningverfahren basiert darauf, daß hier eine potentielle medizinische Wirksamkeit einer Substanz über ihre Wechselwirkung mit mindestens einer physiologisch relevanten Protein- oder Peptidstruktur, einem Target, BNPI und/oder DNPI oder verwandten Strukturen, aufgefunden werden kann. BNPI wird in der Literatur und zum Teil auch dieser Erfindung als VGLUT1 bezeichnet und DNPI als VGLUT2. Diese Begriffe sind daher insbesondere im Rahmen dieser Erfindung als völlig gleichbedeutend anzusehen. BNPI und DNPI bzw. die davon abgeleiteten hier aufgezählten Proteine und Teilproteine bzw. Peptide oder für diese kodierenden Nukleinsäuren wurden im Rahmen dieser Erfindung als interessante Targets identifiziert. Dabei zeigten BNPI und DNPI eine Lokalisation in den unterschiedlichsten ZNS-Bereichen, aber überraschenderweise - trotz teilweise engster Nachbarschaft - auch eine stets streng getrennte Lokalisation, wobei gerade diese strenge Trennung deutlich darauf hindeutet, daß über DNPI und BNPI wichtige physiologische Prozeße gesteuert werden. BNPI (VGLUT1) wird vor allem von einer Population großer DRG-Neurone stark exprimiert, die Substanz P negativ sind. DNPI (VGLUT2) hingegen mRNA findet sich vor allem in den mittelgroßen und kleinen Substanz-P-positiven Neuronen. Die Lokalisation von VGLUT1 (BNPI) und VGLUT2 (DNPI) ist völlig unabhängig von der Lokalisation des verwandten Transporters VGLUT3. Da DNPI und BNPI in therapeutisch sehr interessanten Bereichen des ZNS lokalisiert sind und daher für eine entsprechende Vielzahl von Indikationen von Interesse sind, sind DNPI und BNPI entsprechend wichtige Targets, mit denen Screeningverfahren auf pharmakologisch wirksame Verbindungen durchgeführt werden können. Folglich ist auch bevorzugt, wenn in einem Screeningverfahren gleichzeitig BNPI und DNPI bzw. jeweils eines der davon abgeleiteten hier aufgezählten Biomoleküle wie Proteine und Teilproteine bzw. Peptide oder eine für diese kodierende Nukleinsäure eingesetzt werden, oder das Ergebniss zweier getrennter Screeningverfahren einmal mit BNPI bzw. einem der davon abgeleiteten hier aufgezählten Biomoleküle wie Protein und Teilprotein bzw. Peptid oder einer für diese kodierenden Nukleinsäure und einmal mit DNPI bzw. einem der davon abgeleiteten hier aufgezählten Biomoleküle wie Protein und Teilprotein bzw. Peptid oder einer für diese kodierenden Nukleinsäure durchgeführt werden und in beiden Fällen durch differentiellen Abgleich der Daten optimiert pharmakologisch wirksame Substanzen identifiziert werden, die dann hochspezifisch sind.

Dabei bezieht sich die Begriffe pharmazeutisch relevant oder pharmakologisch wirksam auf einen potentiell heilenden oder lindernden Einfluß der Substanz auf bestimmte Krankheitsbilder. Der Begriff Substanz umfaßt jede als Arzneimittel-Wirkstoff geeignete Verbindung, insbesondere also niedermolekulare Wirkstoffe, aber auch andere wie Nukleinsäuren, Fette, Zucker, Peptide oder Proteine0 wie Antikörper.

Die Inkubation unter geeigneten Bedingungen ist hier so zu verstehen, daß die zu untersuchende Substanz mit dem Biomolekül oder der Zelle oder der entsprechenden Präparation in einem wässrigen Medium eine definierte Zeit vor der Messung reagieren kann. Dabei kann das wässrige Medium temperiert werden, beispielsweise zwischen 4°C und 40°C, vorzugsweise bei Raumtemperatur oder bei 37°C. Die Inkubationszeit kann zwischen wenigen Sekunden und mehreren Stunden variiert werden, je nach der Wechselwirkung der Substanz mit dem Biomolekül oder Teilprotein oder Protein. Bevorzugt sind aber Zeiten zwischen 1 min und 60 min. Das wäßrige Medium kann geeignete Salze und/oder Puffersysteme enthalten, so daß bei der Inkubation beispielsweise ein pH zwischen 6 und 8, vorzugsweise pH 7,0 - 7,5 im Medium herrscht. Dem Medium können weiter geeignete Substanzen, wie Coenzyme, Nährstoffe etc. beigefügt werden. Die geeigneten Bedingungen können vom Fachmann in Abhängigkeit von der zu untersuchenden Wechselwirkung der Substanz mit dem Teilprotein oder Protein aufgrund seiner Erfahrung, der Literatur oder weniger, einfacher Vorversuche leicht festgelegt werden, um im Verfahren einen möglichst deutlichen Meßwert zu erhalten.

Eine Zelle, die ein bestimmtes Teilprotein oder Protein bzw. Biomolekül synthetisiert hat, ist eine Zelle, die dieses Teilprotein oder Protein bereits endogen exprimiert hat oder eine solche, die gentechnisch verändert wurden, so daß sie dieses Teilprotein oder Protein bzw. Biomolekül exprimiert und entsprechend vor Beginn des erfindungsgemäßen Verfahrens das Teilprotein oder Protein enthält. Die Zellen können Zellen aus evt. immortalisierten Zellinien sein oder native aus Geweben stammende und aus diesen isolierte Zellen sein, wobei der Zellverband meist aufgelöst ist. Die Präparation aus diesen Zellen umfaßt insbesondere Hömogenate aus den Zellen, das Cytosol, eine Membranfraktion der Zellen mit Membranfragmenten, eine Suspension isolierter Zellorganellen etc.

Aus welcher Spezies diese Proteine,Teilproteine oder Biomoleküle stammen, ist für die Funktion des Verfahrens unerheblich, es ist aber bevorzugt, die humane, Maus- oder Ratten-Variante zu verwenden. BNPI und DNPI sind in Hinblick auf die kodierende DNA- und die Aminosäure-Sequenz bekannt und auch in Ihrer generellen Funktion beschrieben. BNPI, der "brain Na+ dependent inorganic phosphate cotransporter" ist in der WO 96/34288 beschrieben und der DNPI, der "differentiationassociated Na+ dependent inorganic phosphate Cotransporter", wurde von Aihara et al. (2000) im J. Neurochem. 74, 2622-2625 beschrieben. Neben der Funktion als natriumabhängigen Phosphattransporter wurde für BNPI auch eine Funktion als vesikulärer Glutamat-Transporter beschrieben und BNPI als VGlutT1 bezeichnet (Bellocchio et al. (2000), Science 189:957-960; Takamori et al. (2000), Nature 407; 189-194).

Der Maßstab, über den das Verfahren die Auffindung interessanter Substanzen erlaubt, ist entweder die Bindung an das Biomolekül, das Protein oder Teilprotein, die z.B. durch Verdrängung eines bekannten Liganden oder das Ausmaß gebundener Substanz nachgewiesen werden kann, oder die Veränderung eines funktionellen Parameters durch die Wechselwirkung der Substanz mit dem Teilprotein oder Protein bzw. Biomolekül. Diese Wechselwirkung kann insbesondere in einer Regulation, Hemmung und/oder Aktivierung von Rezeptoren, lonenkanälen und/oder Enzymen liegen und veränderte funktionelle Parameter können beispielsweise die Genexpression, das lonenmilieus, der pH oder das Membranpotentials, bzw. die Veränderung der Enzymaktivität oder der Konzentration der 2^{nd} messenger sein.

Zur Erläuterung der Erfindung werden im folgenden neben den im allgemeinen Text zu Begriffen gegebenen Erklärungen weitere Definitionen angegeben, um klarzustellen, wie bestimmte, insbesondere in den Ansprüchen verwendete Begriffe im Sinne dieser Erfindung zu verstehen und auszulegen sind.
- Substanz: Damit ist eine chemische Verbindung gemeint. Hier handelt es sich im engeren Sinne um Verbindungen, die potentiell eine Wirkung im Körper entfalten können, niedermolekulare Wirkstoffe, Nukleinsäuren, Fette, Zucker, Peptide oder Proteine, insbesondere hier niedermolekulare Wirkstoffe.
- pharmazeutisch relevante Substanz: Im Sinne der Erfindung ist eine pharmazeutisch relevante Substanz eine Substanz, die über die Bindung an die Biomoleküle der Gruppen I bis III in wenigstens einer der genannten Indikationen wirksam sein könnte und theoretisch das Potential besitzt, physiologisch die Symptome direkt oder indirekt zu beeinflußen, insbesondere so erscheint, als könne sie therapeutisch, beispielsweise in einem Arzneimittel, eingesetzt werden.
- schmerzregulierend: Im Sinne der Erfindung heißt schmerzregulierend, daß die Substanz die Wahrnehmung von Schmerz direkt oder indirekt beeinflußt, insbesondere natürlich analgetisch wirkt.
- Schmerz: Im Sinne der Erfindung bedeutet Schmerz insbesondere ein Schmerzempfinden, präziser akkuter, chronischer, neuropathischer und entzündlicher Schmerz inclusive Migräne, insbesondere ist der Schmerz zugehörig zu folgenden Arten:
   chronischem Schmerz, insbesondere muskuloskelettalem Schmerz; neuropathischem Schmerz, insbesondere allodynischem Schmerz, mechanischer Hyperalgesie oder diabetischer Neuropathie; viszeralem Schmerz, cerebralem Schmerz, peripherem Schmerz oder entzündungsbedingtem Schmerz, insbesondere peripherem Entzündungsschmerz; sowie Migräne, Cluster-Kopfschmerz oder den Schmerz bei Trigeminus Neuralgie.
- Inkubation: Unter Inkubation ist das Einbringen und Belassen eines biologischen Untersuchungsobjektes, beispielsweise einer Zelle oder eines Proteins, in einem temperierten Medium wie in einem Brutschrank oder auf einem Wasserbad zu verstehen. Dabei heiß hier unter geeigneten Bedingungen eine Inkubation unter physiologischen Bedingungen (z.B. 37°C pH7,2) oder bei den Bedingungen, bei denen eine optimale Messung im Verfahren möglich wird.
- Zelle: Die Zelle ist ein sich selbst regulierendes, offenes, mit seiner Umgebung durch permanenten Stoffaustausch in einem Fließgleichgewicht stehendes System mit eigenem Stoffwechsel, und Vermehrungsfähigkeit. Die Zelle kann separat kultiviert oder Teil eines Gewebes, insbesondere aus einem Organ, sein, und dort vereinzelt oder noch im Zellverband vorliegen.
- Präparation aus einer Zelle: Darunter versteht man Präparate, die mittels chemischer, biologischer, mechanischer oder physikalischer Methoden unter Änderung der Zellstruktur hergestellt werden, beispielsweise Membranfragmente, isolierte Zellkompartimente, isoliertes Cytosol, oder aus Gewebe gewonnenes Homogenat.
- Peptid: Verbindung aus über peptidische Bindungen zu Ketten verknüpften Aminosäuren. Ein Oligopeptid besteht aus zwischen 2 und 9 Aminosäuren, ein Polypeptid aus zwischen 10 und 100 Aminosäuren.
- Protein: Verbindung aus über peptidische Bindungen zu Ketten verknüpften mehr als 100 Aminosäuren u.U. mit einer definierten Raumstruktur.
- Teilprotein: Verbindung aus über peptidische Bindungen zu Ketten verknüpften mehr als 10 Aminosäuren u.U. mit einer definierten Raumstruktur aber ausgeschnitten bzw. ausgewählt aus einem definierten Priotein. Ein Teilprotein kann ein Peptid sein.
- PIM1-Kinase; PIM3-Kinase: Ein Proto-Oncogen und eine Serin-Threonin-Kinase.
- Polynukleotid: Das zugrundeliegende Nukleotid ist ein grundsätzlich aus Nucleinbase, Pentose und Phosphorsäure bestehender Grundbaustein der Nucleinsäuren. Diese entspricht einem hochmolekularen Polynucleotid aus mehreren Nukleotiden, die über Phosphorsäure-Pentose-Veresterung miteinander verknüpft sind. Unter dierr Erfindung fallen aber auch modifizierte Polynukleotide, die zwar die Basenabfolge beibehalten, aber über ein modifiziertes Rückrat statt der Phosphorsäure-Pentose verfügen.
- zu mindestens 90 (95, 97)% ähnlich: Darunter ist zu verstehen, daß die mit erfaßten Polynucleotide in ihrem kodierenden Bereich beuzüglich der Basenabfolge zu mindestens 90% (95%, 97%) identisch mit der Referenz (Abbildung etc.) sind und die mit erfaßten Peptide und Proteine in ihrer Primärstruktur, der Abfolge der Aminosäuren zu mindestens 90% (95%, 97%) mit der Referenz identisch sind.
- Gen: Mit dem Begriff Gen wird ein Genomabschnitt mit einer definierten Nukleotidsequenz bezeichnet, der die Information zur Synthese einer m- oder prä-mRNA oder einer sonstigen RNA (z.B. tRNA, rRNA, snRNA etc.) enthält. Es besteht aus kodierenden und nicht kodierenden Abschnitten.
- Genfragment: Nukleinsäureabschnitt, der in seiner Basenabfolge einen Teilbereich eines Gens beinhaltet
- Biomolekül: Allgemeiner Begriff für Nukleinsäuren oder Polyaminosäuren, insbesondere auch DNA, RNA, Peptide (Teilproteine) und Proteine, wobei diese Moleküle auch künstlich verändert sein dürfen. Im Sinne dieser Erfindung vorzugsweise Peptide (Teilproteine) und Proteine.
- Bindung an das Peptid, Teilprotein oder Protein: Wechselwirkung zwischen Substanz und Peptid, Teilprotein oder Protein, die zu Fixierung führt.
- funktionelle Parameter: darunter versteht man Meßgrößen eines Experimentes, die mit der Funktion eines Proteins (lonenkanal, Rezeptor, Enzym) korrelieren
- gentechnisch manipuliert: Manipulation von Zellen, Geweben oder Organismen derart, daß hier genetisches Material eingebracht wird
- endogen exprimiert: Expression eines Proteins, die eine Zelllinie unter geeigneten Kulturbedingungen aufweist, ohne das dieses entsprechende Protein durch gentechnische Manipulation zur Expression veranlasst wurde
- G-Protein: International übliche Abkürzung für ein Gunaosintriphosphat (GTP)-bindendes Protein, das als Signalprotein durch G-Protein gekoppelte Rezeptoren aktiviert wird
- Reportergen: Generelle Bezeichnung für Gene, deren Genprodukte sich mit Hilfe einfacher biochemischer Methoden oder histochemischer Methoden einfach nachweisen lassen, wie z.B. Luziferase, alkalische Phosphatase oder Green Fluorescent Protein (GFP).
- (rekombinantes) DNA-Konstrukt: Generelle Bezeichnung für jede Art von DNA-Molekülen, die durch die in vitro-Verknüpfung von DNA-Molekülen entstanden sind.
- Klonierungsvektor: Generelle Bezeichnung für Nukleinsäure-Moleküle, die beim Klonieren als Träger von Fremdgenen oder Teilen dieser Gene dienen.
- Expressionsvektor: Bezeichnung für speziell konstruierte Klonierungsvektoren, die nach Einbringen in eine geeignete Wirtszelle die Transcription und Translation des in den Vektor einklonierten Fremdgens erlauben.
- LTR-Sequenz: Abkürzung für Long terminal repeat. Generelle Bezeichnung für längere Sequenzbereiche, die an beiden Enden eines linearen Genoms zu finden sind. Derartige Sequenzbereiche kommen z.B. in den Genomen von Retroviren und an den Enden eukaryontischer Transposons vor.
- Poly-.A-Schwanz: die am 3'-Ende von messenger-RNAs durch Polyadenylierung angeheftenen Adenyl-Reste (ca. 20-250).
- Promotor-Sequenz: Bezeichnung für einen DNA-Sequenzbereich, von dem aus die Transkription eines Gens, d.h. die Synthese der mRNA, gesteuert wird.
- ORI-Sequenz: Abkürzung für Origin of replication. Die ORI-Sequenz erlaubt einem DNA-Molekül, sich als autonome Einheit in der Zelle zu vermehren.
- Enhancer-Seguenz: Bezeichung für relativ kurze, zum Teil als Repetitionen auftretende, genetische Elemente, die in der Regel die Expression mancher Gene in unterschiedlichem Maße verstärken.
- Transkriptionsfaktor: Bezeichnung für ein Protein, das über eine Bindung an spezifische DNA-Sequenzen, die Transkription eines Gens beeinflußt.
- kultivieren: Zellen oder Gewebe unter geeigneten Kulturbedingungen halten
- Bedingungen, die eine Expression erlauben, darunter versteht man die Auswahl und Anwendung von Kulturbedingungen die eine Expression des interessierenden Proteins erlauben, darunter gehören Temperaturänderung, Mediumwechsel, Zusatz von induzierenden Substanzen, Weglassen hemmender Substanzen.
- Inkubationszeit: Zeitdauer für die Zellen oder Gewebe inkubiert, d.h. einer definierten Temperatur ausgesetzt werden.
- Selektionsdruck: Anwendungen von Kulturbedingungen die Zellen mit einem bestimmtem Genprodukt, dem sog. Selektionsmarker, einen Wachstumsvorteil verschaffen.
- Amphibienzelle, Zelle aus einem Tier der Klasse der Amphibia
- Bakterienzelle, Zelle die dem Überreich der Eubacteria oder Archaebacteria zuzuordnen ist, oder von ihr abstammt.
- Hefezelle, Zelle die der Ordnung der Endomycetalse zuzuordnen ist, oder von ihr abstammt.
- Insektenzelle, Zelle die der Ordnung der Hexapoda zuzuordnen ist, oder von ihr abstammt.
- native Säugetierzelle, aus einem Säugetier stammende Zelle, die in ihren relevanten Merkmalen der im Organismus befindlichen Zelle entspricht.
- immortalisierte Säugetierzelle: Zelle die durch die angewendeten Kulturbedingungen oder gentechnische Manipulation die Eigenschaft erlangt hat, sich über die normalerweise übliche Teilungshäufigkeit hinaus (ca.100) in der Kultur zu teilen.
- markiert: durch entsprechende Modifizierung oder Derivatisierung für eine Nachweisreaktion zugänglich gemacht. Beispielsweise radioaktiv, fluoreszierend oder lumineszierend.
- Ligand: Substanz, die an ein im Körper oder einer Zelle befindliches Molekül, im speziellen einen Rezeptor, bindet
- Verdrängung: vollständiges oder partielles Entfernen eines Liganden von seiner Bindungsstelle
- gebundene Aktivität: Biochemisch oder physikalisch erfaßter Meßwert, der mit der an einem Rezptor gebundenen Ligandenmenge korreliert
- Regulation: die als Teil eines Regelprozese erfolgte Hemmung oder Aktivierung eines Vorgangs
- Hemmung: als Sonderfall der Regulation die Verhinderung/Minderung eines Vorgangs
- Aktivierung: als Sonderfall der Regulation die Verstärkung eines Vorgangs
- Rezeptoren, Im weitesten Sinne alle im pro- oder eukaryoten Organismus vorhandenen Moleküle, an die ein Wirkstoff binden kann. Im engeren Sinne membrangebundene Proteine oder Komplexen mehrerer Proteine, die durch Bindung eines Wirkstoffes eine Änderung in der Zelle hervorrufen.
- lonenkanäle: Membrangebundene Proteine oder Komplexe mehrerer Proteine, durch die Kationen oder Anionen durch die Membran hindurchgelangen können.
- Enzyme: Bezeichnung für Proteine oder Komplexe aus einer aktivierenden Nichteiweißkomponente mit einem Protein, die katalytische Eigenschaften besitzen.
- Genexpression (exprimieren/exprimierbar): das Übersetzen der genetischen Information eines Genes in RNA (RNA-Expression) oder in Protein (Proteinexpression).
- Ionenmilieu: lonenkonzentration eines oder mehrerer Ionen in einem bestimmten Kompartiment.
- Membranpotential: Spannungsdifferenz über eine Membran aufgrund eines Überschusses an Kationen auf der einen Seite und Anionen auf der anderen Seite der Membran.
- Veränderung der Enzymaktivität: Hemmung oder Induktion der katalytischen Aktivität eines Enzyms.
- 2^{nd} messenger: kleines Molekül, das als Antwort auf ein extrazelluläres Signal entweder im Cytosol gebildet wird oder in das Cytosol hineinwandert und dabei hilft die Information an das Zelliniere weiterzugeben, wie zum Beispiel cAMP, IP₃.
- (Gen-)Sonde: Bezeichnung für jede Art von Nukleinsäuren, mit deren Hilfe man ein gesuchtes Gen oder eine bestimmte DNA-Sequenz nachweisen kann. Durch Derivatisierung der Gensonde (z.B. Biotin, magnetische Beads, Digoxinin) können zudem DNA-Moleküle aus einem Gemisch herausgezogen werden. Als Sonden werden klonierte Gene, Genfragmente, chemisch synthetisierte Oligonukleotide und auch RNA verwendet, die meist radioaktiv markiert ist.
- DNA: Internationale Bezeichnung für Desoxyribonukleinsäure
- genomische DNA: Generelle Bezeichnung für die bei eukaryontischen Organismen aus dem Zellkern einer Zelle stammenden DNA.
- cDNA: Abkürzung für complementary DNA. Bezeichnung für die einzel- bzw. doppelsträngige DNA-Kopie eines RNA-Moleküls.
- cDNA-Bank/Bibliothek: Bezeichung für eine Sammlung von willkürlich klonierten cDNA-Fragmenten, die zusammengenommen die Gesamtheit aller von einer Zelle oder einem Gewebe synthetisierten RNA repäsentieren.
- cDNA-Klon: Bezeichnung für eine Population genetisch einheitlicher Zellen, die sich von einer einzigen Zelle ableiten, derart, daß diese Zelle eine künstlich eingebrachtes cDNA-Fragment enthält.
- Hybridisierung: Durch Basenpaarung bewirkte Ausbildung eines doppelsträngigen Nukleinsäuremoleküls aus zwei getrennten Einzelsträngen.
- stringente Bedingungen: Bedingungen, unter denen nur perfekt basengepaarte Nukleinsäure-Stränge gebildet werden und stabil bleiben.
- isolieren: ein gesuchtes Molekül aus einem Gemisch herausfinden und abtrennen.
- DNA-Sequenzierung: Bestimmung der Abfolge der von Basen in einem DNA-Molekül.
- Nukleinsäuresequenz: Bezeichnung für die Primärstruktur eines DNA-Moleküls, d.h. die Abfolge der einzelnen Basen, aus denen sich eine DNA zusammensetzt.
- Genspezifische Oligonukleotid Primer: Oligonukleinsäuren, also 10-40 Basen lange Nukleinsäurefragmente, die in ihrer Basenzusammensetzung eine stringente Hybridisierung an das gesuchte Gen oder die gesuchte cDNA erlauben.
- Ermitteln von Oligonukleotid Primern: Die manuelle oder Computerunterstützte Suche von Oligonukleotiden zu einer vorgegebenen DNA-Sequenz, die für eine Hybridisierung und/oder eine Polymerase-Ketten Reaktion optimal geeignet sind.
- PCR: Abkürzung für Polymerase-Kettenreaktion. In vitro-Verfahren zur selektiven Anreicherung von Nucleinsäure-Bereichen definierter Länge und definierter Sequenz aus einem Gemisch von NukleinsäureMolekülen.
- DNA-Template: Nukleinsäuremolekül oder ein Gemisch von Nukleinsäuremolekülen, aus denen ein DNA-Abschnitt mit Hilfe der PCR (s.o.) vervielfältigt wird.
- RNA: International gebräuchliche Abkürzung für Ribonukleinsäuren
- mRNA: International gebräuchliche Abkürzung für messenger-Ribonukleinsäuren, die am Transfer der genetischen Information aus dem Kern in die Zelle beteiligt sind und die Information für die Synthese eines Polypetids oder eines Proteins beinhalten.
- Antisense Polynukleotid: Eine aus mehreren natürlichen oder modifizierten Nukleinsäuren bestehendes Molekül, deren Basenabfolge komplementär zur Basenabfolge eines Teilbereiches einer in der Natur vorkommenden RNA ist.
- PNA: International gebräuchliche Abkürzung für peptidic Nucleic Acids. Hierbei bilden peptidisch verknüpfte Aminosäuren eine Kette, wobei die Aminosäuren als Seitenkette eine für die Hybridisierung mit DNA oder RNA fähigen Base trägt.
- Sequenz: Abfolge von Nukleotiden oder Aminosäuren. Im spezifischen Sinne dieser Erfindung ist damit die Nukleinsäuresequenz gemeint.
- Ribozym: Bezeichnung für eine katalytisch aktive Ribonukleinsäure (z.B. Ligase, Endonuklease, Polymerase, Exonuklease)
- DNA-Enzym: Bezeichnung für ein DNA-Molekül, das katalytische Aktivität beinhaltet (z.B. Ligase, Endonuklease, Polymerase, Exonuklease)
- katalytische RNA/DNA: generelle Bezeichnung für Ribozyme bzw. DNA-Enzyme (s.o.).
- Adenovirus: bei Vertebraten vorkommender cytopathogener Virus
- Adenoassoziiertes Virus (AAV): Gehört zur Familie der Parvoviren. Für eine effektive Vermehrung des AAV ist eine Coinfektion der Wirtszellen mit Helferviren (z.B. Herpes-, Vaccina- oder Adenoviren) erforderlich. Die Eigenschaft von AAV, stabil in das Wirtsgenom zu integrieren, macht es als Transduktionsvektor für Säugetierzellen besonders interessant.
- Herpesvirus: Viraler Erreger der Herpes-Infektion
- posttranslationale Modifikation: Veränderung an Proteinen oder Polypetiden, die nach der Translation durchgeführt wird, hierzu zählen z.B. Phosphorylierung, Glykosylierung, Amidierung, Acetylierung oder Proteolyse.
- glykosilieren: Bezeichnung für das Anhängen von einzelnen Zuckermolekülen oder ganzen Zuckerketten an Proteine.
- phosphorylieren: Bezeichnung für das Anhängen von einem oder mehreren Phosphatresten an ein Protein, bevorzugt an die OH-Gruppen der Aminosäuren Serin, Threonin oder Tyrosin.
- amidieren. Die Bezeichnung für das Umwandeln einer Carboxylfunktion in eine Amidfunktion, z.B. an den carboxyterminalen Aminosäurerest eines Peptides oder Proteins.
- mit Membrananker versehen: Posttranslationelle Modifikation eines Proteins, oder eines anderen organischen Moleküls, derart daß es durch Anhängen eines hydrophoben Moleküls, geeigneterweise eine Fettsäure oder ein Derivat derselben, an die Lipiddoppelschicht-Membran von Zellen verankert wird.
- spalten: in diesem spezifischen Fall die Spaltung eines Peptids oder Proteins in mehrere Untersequenzen.
- verkürzen: Ein aus mehreren Einzelteilen bestehendes Molekül um eine oder mehrere Teile zu verkürzen.
- Antikörper: Lösliche, oder an Zellmembranen gebundene, als Immunglobuline bezeichnete Proteine mit einer spezifischen Bindungsstelle für Antigene.
- monoklonaler Antikörper: sind gegen eine einzige antigene Determinante eines Antigens gerichtete Antikörper mit extrem hoher Selektivität.
- polyklonaler Antikörper: Gemisch aus Antikörpern, die gegen mehrere Determinanten eines Antigens gerichtet sind.
- transgen: genetisch verändert.
- nichthumanes Säugetier: Die Gesamtheit der Säugetiere (Klasse der Mammalia) mit Ausnahme der Spezies Mensch.
- Keim-Zelle: Zelle mit haploidem Genom, die durch Verschmelzung mit einer zweiten Keimzelle die Bildung eines neuen Organismus ermöglicht.
- somatische Zelle: diploide Zelle als Bestandteil eines Organismus.
- chromosomale Einbringung: Eingriff in die Nukleotidsequenz auf chromosomaler Ebene.
- Genom: Allgemeine Beschreibung für die Gesamtheit aller Gene in einem Organismus.
- Vorfahr des Tieres: Ein Tier (der Vorfahr), das auf natürliche oder künstliche Weise durch Weitergabe an seinem genetischen Material in direkter Linie mit einem anderen Tier (dem Nachfahren) verwandt ist.
- exprimierbar: Ein Nukleinsäuremolekül ist dann exprimierbar, wenn es die Information zur Synthese eine Proteins oder Polypetids beinhaltet und mit ensprechenden regulatotischen Sequenzen versehen ist, die eine Synthese dieses Proteins oder Polypeptids in vitro oder in vivo erlauben. Wenn diese Voraussetzungen nicht mehr gegeben sind, beispielsweise durch Eingriff in der kodierenden Sequenz, ist das Nukleinsäuremolekül nicht mehr exprimierbar.
- Nagetier: Tier aus der Ordnung der Rodentia, z.B. Ratte oder Maus.
- als schmerzregulierende Substanz identifizierbar: Substanz die bei Einbringung in einen lebenden Organismus eine Verhaltensänderung bewirkt, die der Fachmann als schmerzhemmend bezeichnet (antinozizeptiv, antihyperalgetisch oder antiallodynisch). Im Falle des Screeningverfahrens bezieht sich das darauf, daß die Substanz beim Screening durch stärkere Bindung oder Auslösung einer Änderung eines funktionellen Parameters deutlich, beispielsweise 100 %, die Bindung oder Wechselwirkung des Durchschnitts der getesteten Substanzen übertrifft.
- Verbindung: ein anderer Name für Molekül, als aus mehreren Atomen bestehend, hier ein durch das erfindungsgemäße Verfahren identifiziertes Molekül.
- Wirkstoff: Eine Verbindung, die bei Anwendung an einem Organismus eine Veränderung in diesem Organismus hervorruft. Im Besonderen werden darunter organisch-chemisch- synthetisierte Moleküle verstanden, die auf den Organismus eine heilende Wirkung ausüben. Hier insbesondere Moleküle, die an die erfindungsgemäßen Proteine und Peptide binden.
- niedermolekular: Molekül mit einem Molekulargewicht < 2kDa.
- Arzneimittel: ein Stoff entsprechend der Definition im Artikel 1 §2 des Gesetzes über den Verkehr mit Arzneimitteln.
- Diagnostikum: Verbindung oder Verfahren, das verwendet werden kann, um eine Krankheit zu diagnostizieren.
- Behandlung von Schmerz: Verfahren, mit dem Ziel Schmerzen zu lindern oder aufzuheben, oder das zu erwartende Auftreten von Schmerzen zu hemmen (preemptive Analgesie).
- chronischer Schmerz: eine Schmerzempfindung von länger anhaltender Dauer, oft dadurch gekennzeichnet, daß sie über Zeitpunkt und Ort des initialen Stimulus hinausreicht, die Schmerzempfindlichkeit des Körpers steigert.
- Gentherapie: Unter Gentherapie versteht man alle Verfahren, die das Ziel haben, genetische Erkrankungen durch geeignete Veränderungen des Genoms kausal zu behandeln.
- In-vivo-Gentherapie: Einbringen von genetischem Material in den lebenden Organismus mit dem Ziel der Gentherapie. Man kann zwischen somatischem und Keimbahn-Eingriff unterscheiden, der einmal an diploiden Zellen und einmal an haploiden Zellen stattfindet.
- In-vitro-Gentherapie: Einbringen von genetischem Material in Zellen außerhalb des menschlichen Körpers mit dem Ziel diese nachher wieder durch Einbringen in den menschlichen Körper zur Gentherapie zur verwenden.
- Diagnostik: Verfahren, um eine Krankheit zu identifizieren.
- Wirksamkeitsuntersuchung: Untersuchung mit dem Ziel die Wirksamkeit einer Verbindung nach Einwirkung auf einen lebenden Organismus zu untersuchen.

In einer bevorzugten Ausführungsform des Verfahrens wird die Zelle vor dem Schritt (a) gentechnisch manipuliert. Dabei wird genetisches Material in die Zelle eingebracht, insbesondere eine oder mehrere Polynukleotidsequenzen. In einer weiter bevorzugten Variante dieser Ausführungsform erlaubt die gentechnische Manipulation die Messung mindestens eines der durch die Testsubstanz veränderten funktionellen Parameter. In dieser Ausführungsform werden durch gentechnische Manipulation Voraussetzungen geschaffen unter denen die Veränderung eines funktionellen Parameters überhaupt oder verbessert gemessen werden kann. Dabei ist es insbesondere bevorzugt, daß durch die gentechnische Manipulion eine in der Zelle nicht endogen exprimierte Form eines G-Proteins exprimiert oder ein Reportergen eingeführt wird. Darunter ist insbesondere die gentechnische Einführung eines endogen nicht vorhandenen oder physiologisch nicht exprimierten G-Proteins (GTPbindenden Proteins) in die Zelle zu verstehen, beispielsweise die Einführung eines chimären G-Proteins, das eine Veränderung des Signalweges erlaubt oder eines promiskuitiven G-Proteins, das sehr bindungsfreudig ist. Die Einführung eines Reportergens wiederum erlaubt die Messung einer (extrazellulär ausgelösten) induzierten Expression des Genproduktes.

In einer weiteren bevorzugten Ausführungsform wird die Zelle gentechnisch so manipuliert wird, daß die Zelle mindestens ein Polynukleotid gemäß einer der Abbildungen 1a), 1c), 1e), 1g), 2a), 2c) oder 2e) oder ein dazu zu mindestens 90 %, vorzugsweise zu mindestens 95 %, insbesondere zu mindestens 97 % ähnliches Polynukleotid enthält. Damit kann beispielsweise erreicht werden, daß ein Teilprotein oder Protein, das in der im Verfahren verwendeten Zelle oder Präparation nicht endogen exprimiert wird, von der Zelle synthetisiert wird. Dabei ist es insbesondere bevorzugt, wenn das Polynukleotid in einem rekombinanten DNA-Konstrukt enthalten ist. Unter einem (rekombinanten) DNA-Konstrukt versteht man ein in-vitro hergestelltes DNA-Molekül.

Wenn beim Verfahren vor dem Schritt a) die Zelle gentechnisch manipuliert wird, ist es bevorzugt, daß die Zelle nach der gentechnischen Manipulation und vor dem Schritt (a) unter Bedingungen, die eine Expression erlauben, kultiviert wird, gegebenenfalls unter Selektionsdruck. Unter kultivieren versteht man, Zellen oder Gewebe bei Bedingungen, die ein Überleben der Zellen, bzw. deren Nachfolgegeneration sichern, zu halten. Dabei sollten die Bedingungen hier so gewählt werden, daß eine Expression des durch die gentechnische Manipulation eingefügten Materials ermöglicht wird. Dazu sollten pH, Sauerstoffgehalt, und Temperatur physiologisch gehalten sein und ausreichend Nährstoffe und notwendige Cofaktoren beigefügt sein. Der Selektionsdruck erlaubt, nur die Zellen weiter zu kultivieren, bei denen die gentechnische Manipulation zumindest teilweise erfolgreich war.
Dazu gehört beispielsweise die Einführung einer Antibiotikaresistenz über das DNA-Konstrukt.

Es ist bei dem erfindungsgemäßen Verfahren besonders bevorzugt, wenn die verwendete Zelle eine Amphibienzelle, Bakterienzelle, Hefezelle, Insektenzelle oder eine immortalisierte oder native Säugetierzelle ist. Beispiele für Amphibienzellen sind Xenopus Oocyten, für Bakterienzellen E-coli-Zellen, für Hefezellen auch Saccharomyces cerevisiae, für Insektenzellen Sf9-Zellen, für immortalisierte Säugetierzelle HeLa-Zellen und für native Säugetierzellen die CHO (Chinese Hamster Ovary)-Zelle.

Bei einer bevorzugten Meßmethode zur Feststellung der Bindung der Substanz an das Biomolekül oder Teilprotein oder Protein im erfindungsgemäßen Verfahren erfolgt die Messung der Bindung über die Verdrängung eines bekannten markierten Liganden des Biomoleküls, bzw. des Teilproteins oder Proteins und/oder über die daran gebundene Aktivität einer markierten Testsubstanz. Dabei ist ein Ligand ein mit hoher Spezifität an das Protein oder Teilprotein bindendes Molekül, das durch eine ebenfalls bindende, zu testende Substanz aus der Bindungsstelle verdrängt wird. Unter Markierung ist eine den Nachweis erleichternde künstliche Modifikation am Molekül zu verstehen. Beispiele sind radioaktive, fluoreszierende oder lumineszierende Markierung.

Bei einer anderen bevorzugten Meßmethode zur Feststellung der durch die Bindung der Substanz an Teilprotein oder Protein im erfindungsgemäßen Verfahren ausgelösten Veränderung der funktionellen Parameter, erfolgt die Messung mindestens eines der durch die Testsubstanz veränderten funktionellen Parameter über Messung der Regulation, Hemmung und/oder Aktivierung von Rezeptoren, lonenkanälen und/oder Enzymen, insbesondere über Messung der Veränderung der Genexpression, des Ionenmilieus, des pH oder des Membranpotentials, über Veränderung der Enzymaktivität oder der Konzentration der 2^{nd} messenger. Damit ist auf der einen Seite direkt die Messung der Wirkung der Substanz über die Beeinflußung von Rezeptoren, lonenkanälen und/oder Enzymen erfaßt, auf der anderen Seite als bevorzugt zu messende Beispiele sich ändernder Parameter wie Genexpression, lonenmilieu, pH, Membranpotential, Enzymaktivität oder Konzentration der 2^{nd} messenger. Dabei versteht man unter lonenmilieu insbesondere die Konzentration eines oder mehrer lonen in einem Zellkompartiment, insbesondere dem Cytosol, unter Membranpotential die Ladungsdiffferenz zwischen zwei Seiten einer Biomembran und unter 2^{nd} messenger Botenstoffe des intrazellulären Signalwegs wie z.B. zyklisches AMP (cAMP), Inosotoltriphosphat (IP3) oder Diacylglycerol (DAG).

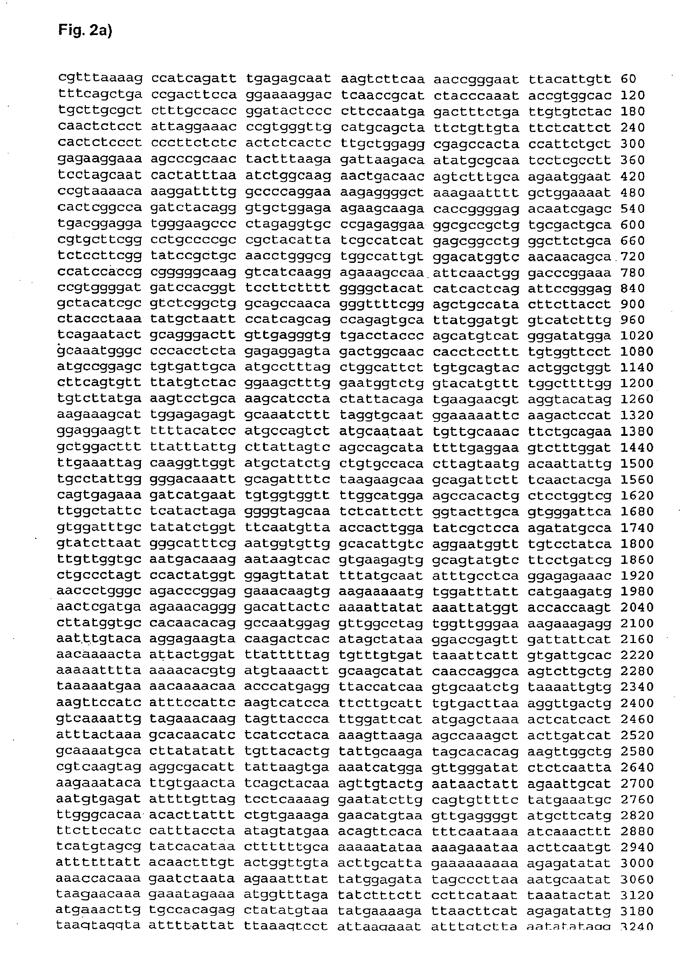

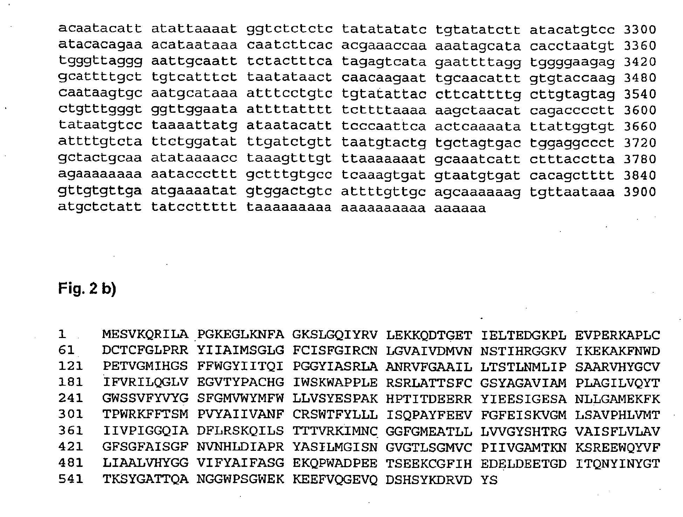

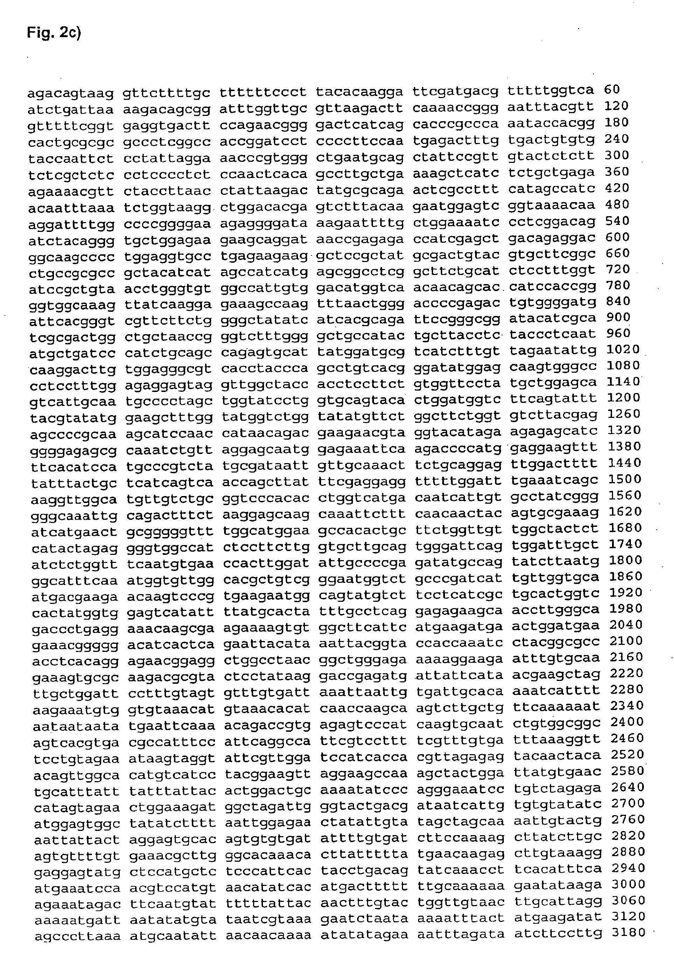

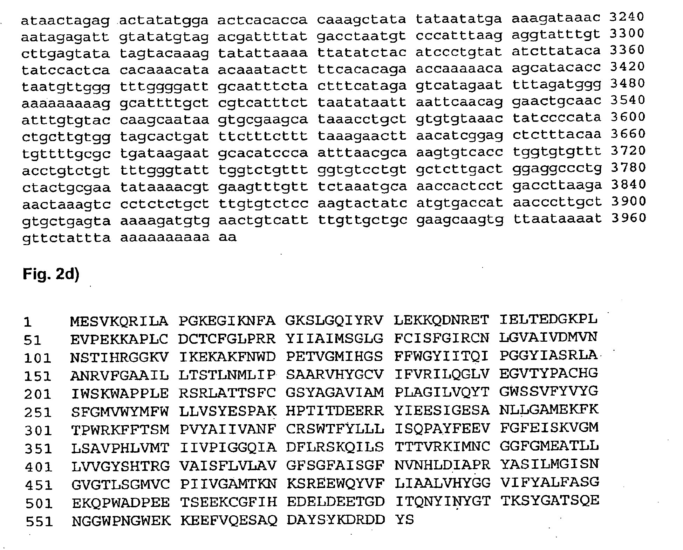

Ein besonders bevorzugter Gegenstand der Erfindung ist ein erfindungsgemäßes Verfahren, bei dem ein erstes erfindungsgemäßes Verfahren mit einem zweiten erfindungsgemäßen Verfahren derart gekoppelt wird, daß die Meßwerte und Ergebnisse des ersten Verfahrens hinsichtlich der zu messenden Substanz mit den Meßwerten und Ergebnissen des zweiten Verfahrens hinsichtlich der zu messenden Substanz verglichen werden, dadurch gekennzeichnet, daß in einem der zwei Verfahren, im folgenden Hauptverfahren genannt, im Schritt (a) die zu testende Substanz
entweder
mit einem Biomolekül ausgewählt aus Gruppe II:
dem Protein BNPI und/oder einem Protein gemäß einer der Abbildungen 1b), 1d), 1f) oder 1h) und/oder einem zu einem dieser vorgenannten Proteine zu mindestens 90 % ähnlichen Protein und/oder einem Protein, für das ein Polynukleotid gemäß einer der Abbildungen 1a), 1c), 1e) oder 1g) oder ein dazu zu mindestens 90 % ähnlichen Polynukleotid kodiert, und/oder einem Protein, das durch eine Nukleinsäure kodiert wird, die unter stringenten Bedingungen an ein Polynukleotid gemäß einer der Abbildungen 1a), 1c), 1e) oder 1g) oder deren Antisense Polynukleotide bindet, oder einem mindestens 10, vorzugsweise mindestens 15, insbesondere mindestens 20 Aminosäuren langen Teilprotein eines der vorgenannten Proteine
und/oder einer Zelle und/oder einer Präparation aus einer solchen Zelle, die mindestens eines der vorgenannten Proteine und Teilproteine, bzw. Biomoleküle aus Gruppe II, synthetisiert hat,
oder
mit einem Biomolekül ausgewählt aus Gruppe III:
dem Protein DNPI und/oder einem Protein gemäß einer der Abbildungen 2b), 2d) oder 2f) und/oder einem zu einem dieser vorgenannten Proteine zu mindestens 90 % ähnlichen Protein und/oder einem Protein, für das ein Polynukleotid gemäß einer der Abbildungen 2a), 2c) oder 2e) oder ein dazu zu mindestens 90 % ähnliches Polynukleotid kodiert, und/oder einem Protein, das durch eine Nukleinsäure kodiert wird, die unter stringenten Bedingungen an ein Polynukleotid gemäß einer der Abbildungen 2a), 2c) oder 2e) oder deren Antisense Polynukleotide bindet, oder einem mindestens 10, vorzugsweise mindestens 15, insbesondere mindestens 20 Aminosäuren langen Teilprotein eines der vorgenannten Proteine
und/oder einer Zelle und/oder einer Präparation aus einer solchen Zelle, die mindestens eines der vorgenannten Proteine und Teilproteine, bzw. Biomoleküle aus Gruppe III, synthetisiert hat,
und
daß im anderen der zwei Verfahren, im folgenden Nebenverfahren genannt, im Schritt (a) die zu testende Substanz mit einem Biomolekül aus der Gruppe I oder mit einem Biomolekül aus derjenigen Gruppe ausgewählt aus Gruppe II und Gruppe III inkubiert wird, aus der das Biomolekül, mit der die Substanz im Hauptverfahren inkubiert wird, nicht ausgewählt ist.
Unter dieser besonders bevorzugten Ausführungsform ist insbesondere die Kombination der Messung der Bindung an BNPI oder davon abgeleiteten Biomolekülen oder der Messung der daraus entstehenden Modifikation zellulärer Parameter auf der einen und der Bindung an DNPI und jeweils davon abgeleiteten Biomolekülen oder der Messung der daraus entstehenden Modifikation zellulärer Parameter auf der anderen Seite zu verstehen, da gerade ein Vergleich angesichts der vollkommen getrennten aber eng aneinanderliegenden Verteilung der beiden Kanäle im Gewebe einen wichtigen Aufschluß über physiologische Funktionen geben kann. Damit erlaubt aber der differentielle Abgleich der Daten die Identifizierung optimiert pharmazeutisch bzw. medizinisch wirksamer Substanzen.

Weiter bevorzugt ist ein erfindungsgemäßes Verfahren worin die aufzufindenden Substanzen ausgewählt sind aus:
Substanzen mit Wirksamkeit in den Indikationen oder zur Behandlung von Sehstörungen, Retinitis pigmentosa, Opticus Degeneration, Hörstörungen, Tinitus, M. Menière, Hörsturz amyotrophe Lateralsklerose, Neuralgie, Gewichtsregulation, Obesitas, Morbus Parkinson, Katarakt, Virus-Infektionen oder bakterielle Infektionen, diabetische Neuropathie, autoimmuner Diabetes, alkoholische Neuropathie, HIV-Neuro-Aids; Retinadegeneration, Glaukom, Nystagmus, Netzhautablösung, Erkrankungen des Hör- und/oder Gleichgewichtsorgans, Erkrankungen der Hörbahn oder Vesibularbahn, Schlafstörungen, Drogenabhängigkeit, -sucht und -entzug insbesondere bei Alkohol, Nikotin, Opiaten, Ecstasy oder Kokain; Neuroinflammation, Insomnia, zur adjuvanten Therapie per Elektrostimulation des Nucleus subthalamicus bei Parkinson; bzw. Substanzen zur Behandlung von Erkrankungen des spinalen Motoneurons, Muskelatrophien oder Muskeldystrophien,
vorzugsweise
Substanzen mit Wirksamkeit in den Indikationen oder zur Behandlung von Sehstörungen, Retinitis pigmentosa, Opticus Degeneration, Hörstörungen, Tinitus, M. Menière, Hörsturz amyotrophe Lateralsklerose, Gewichtsregulation, Obesitas, Katarakt, Virus-Infektionen oder bakterielle Infektionen, Retinadegeneration, Glaukom, Nystagmus, Netzhautablösung, Erkrankungen des Hör- und/oder Gleichgewichtsorgans, Erkrankungen der Hörbahn oder Vesibularbahn, Drogenabhängigkeit, -sucht und -entzug insbesondere bei Alkohol, Nikotin, Opiaten, Ecstasy oder Kokain; Neuroinflammation; bzw. Substanzen zur Behandlung von Erkrankungen des spinalen Motoneurons, Muskelatrophien oder Muskeldystrophien,
insbesondere
Substanzen mit Wirksamkeit in den Indikationen oder zur Behandlung von Sehstörungen, Retinitis pigmentosa, Opticus Degeneration, Katarakt, Netzhautablösung, Retinadegeneration, Glaukom oder Nystagmus
   und/oder
   Hörstörungen, Tinitus, M. Menière, Hörsturz, Erkrankungen des Hör- und/oder Gleichgewichtsorgans oder Erkrankungen der Hörbahn oder Vesibularbahn;
   bzw. Substanzen zur Behandlung von Erkrankungen des spinalen Motoneurons, Muskelatrophien oder Muskeldystrophien.

Ein weiterer Gegenstand der Erfindung ist eine Verbindung identifizierbar als pharmazeutisch relevante Substanz mit Wirksamkeit in mindestens einer der vorgenannten Indikationen durch ein erfindungsgemäßes Verfahren. Hierbei bezieht sich Verbindung insbesondere auf niedermolekulare Wirkstoffe, aber auch auf Peptide, Proteine und Nukleinsäuren. Dabei bedeutet identifizierbar, daß die Verbindung das Merkmal aufweist, daß es beim erfindungsgemäßen Screeningverfahren bezüglich der Bindung deutlich stärker, vorzugsweise doppelt so stark bindet wie der Durchschnitt der zu testenden Substanzen oder bezüglich der Änderung der funktionellen Parameter deutlich vom Durchschnitt der zu testenden Substanzen abweicht. Besonders bevorzugt ist es, wenn die erfindungsgemäße Verbindung eine niedermolekulare Verbindung ist.

Ein weiterer Gegenstand der Erfindung ist die Verwendung
a. eines Polynukleotids, vorzugsweise einer DNA oder RNA, kodierend für BNPI oder DNPI oder eines Polynukleotids, vorzugsweise einer DNA oder RNA, welches einer der in einer der Abbildungen 1a), 1c), 1e), 1g), 2a), 2c) oder 2e) dargestellten Nukleotidsequenzen zu wenigstens 90%, vorzugsweise zu wenigstens 95%, insbesondere zu wenigstens 97% oder genau entspricht,
b. eines Polynukleotids, insbesondere eines Antisense Polynukleotids oder einer PNA, vorzugsweise eines DNA-Enzyms oder Ribozyms, eines Ribozyms oder sonstigen DNA-Enzyms oder einer katalytischen RNA oder DNA, das eine Nukleotid-Sequenz aufweist, die in der Lage ist, spezifisch an eines der unter Punkt a) aufgeführten Polynukleotide zu binden,
c. eines Vektors enthaltend ein Polynukleotid gemäß einem der Punkte a) oder b), insbesondere eines Expressionsvektors und/oder insbesondere abgeleitet von einem Virus, beispielsweise dem Adenovirus, Adenoassoziiertem Virus oder Herpesvirus und/oder insbesondere enthaltend mindestens eine LTR-, Poly A-, Promotor- und/oder ORI-Sequenz,
d. von BNPI und/oder DNPI und/oder eines Proteins gemäß einer der Abbildungen 1b), 1d), 1f), 1h), 2b), 2d) oder 2f) und/oder eines zu einem dieser vorgenannten Proteine zu mindestens 90 %, vorzugsweise mindestens 95%, insbesondere mindestens 97% ähnlichen Proteins und/oder eines Proteins, für das ein Polynukleotid gemäß einer der Abbildungen 1a), 1c), 1e), 1g), 2a), 2c) oder 2e) oder ein dazu zu mindestens 90 %, vorzugsweise mindestens 95%, insbesondere mindestens 97% ähnliches Polynukleotid kodiert, und/oder eines Proteins, das durch eine Nukleinsäure kodiert wird, die unter stringenten Bedingungen an ein Polynukleotid gemäß einer der Abbildungen 1a), 1c), 1e), 1g) 2a), 2c) oder 2e) oder dessen Antisense Polynukleotide bindet oder eines mindestens 10, vorzugsweise mindestens 15, insbesondere mindestens 20 Aminosäuren langen Teilproteins eines der vorgenannten Proteine, wobei das Protein oder Teilprotein gegebenenfalls posttranslational modifiziert, insbesondere glykosiliert, phosphoryliert, amidiert, methyliert, acetyliert, ADP-ribosyliert, hydroxyliert, mit einem Membrananker versehen, gespalten oder verkürzt wurde,
e. eines Antikörpers, vorzugsweise eines monoklonalen oder polyklonalen Antikörpers, gegen eines der Proteine oder Teilproteine gemäß Punkt d),
f. einer Zelle, vorzugsweise einer Amphibienzelle, Bakterienzelle, Hefezelle, Insektenzelle oder einer immortalisierten oder nativen Säugetierzelle, enthaltend ein Polynukleotid gemäß einem der Punkte a) oder b), einen Vektor gemäß Punkt c), ein Protein oder Teilprotein gemäß Punkt d) oder einen Antikörper gemäß Punkt e)
g. einer erfindungsgemäßen Verbindung identifizierbar als pharmazeutisch relevante Substanz mit Wirksamkeit in mindestens einer der vorgenannten Indikationen wie oben beschrieben und/oder
h. eines Wirkstoffs, vorzugsweise eines niedermolekularen Wirkstoffs, der an ein Protein oder Teilprotein gemäß Punkt d) bindet,

zur Herstellung eines Arzneimittels zur Behandlung von
Sehstörungen, Retinitis pigmentosa, Opticus Degeneration, Hörstörungen, Tinitus, M. Menière, Hörsturz, Schizophrenie, Manien, Depression, Schlaganfall, Hirntrauma, Querschnittslähmung, amyotrophe Lateralsklerose, Neuralgie, Gewichtsregulation, Obesitas, Anorexia nervosa, Epilepsie, Hemibalismus, Chorea Huntington, Stress, Morbus Parkinson, TIA (Transiente lschämische Attacken), Emesis, insbesondere Hyperemesis beispielsweise bei Chemotherapie, Schwindel, in jeglicher Form, Katarakt, Arthritis, Hyperaktivität, Entwicklungsstörungen, Tollwut, Virus-Infektionen oder bakterielle Infektionen, Grippe, Malaria, Creutzfeld-Jacob, Inflammatory Bowel disease, Morbus Crohn, cardiovaskuläre und cardiorespiratorische Funktionsstörungen, Hypertonie, Störungen der Baroafferenz oder Chemoafferenz, Toxoplasmose, Asthma, Autoimmunität im zentralen und peripheren Nervensystem, diabetische Neuropathie, autoimmuner Diabetes, alkoholische Neuropathie, HIV-Neuro-Aids; Störungen des autonomen Nervensystems, Störungen des Nervensystems des Verdauungstraktes, Übererregbarkeit, insbesondere glutamatvermittelte Übererregbarkeit, Neurodegeneration insbesondere bei Morbus Alzheimer, Morbus Alzheimer, Ischämie; Encephalitis insbesondere virale oder bakterielle; Prionerkrankung, Rasmussen-Encephalitis, HIV-Encephalitis, Demyelinisierung insbesondere bei multipler Sklerose, Retinadegeneration, Glaukom, Nystagmus, Netzhautablösung, Erkrankungen des Cerebellums, cerebelläre Ataxie, Erkrankungen der Basalganglien, Erkrankungen des Pallidums, Erkrankungen des Hör- und/oder Gleichgewichtsorgans, Erkrankungen der Hörbahn oder Vesibularbahn, Störungen des Gedächtnisses, Störungen des Lernens, Störungen der Kognition, Stiff-Man-Syndrom, Restless Leg-Syndrom, Angstzustände, Phobien, Schlafstörungen; Drogenabhängigkeit, -sucht und -entzug insbesondere bei Alkohol, Nikotin, Opiaten, Ecstasy oder Kokain; Hepatoencephalopathie mit Alkoholintoxikation, Hepatoencephalopathie ohne Alkoholintoxikation, neurotoxikologisch bedingte Erkrankungen, Erkrankungen des spinalen Motoneurons, Muskelatrophien, Muskeldystrophien, Erkrankungen der Hinterstrangbahnen, alkoholische Neuropathien, Neuroinflammation, Befindlichkeitsstörungen bei Infektionen oder Fieber, Stress, Geschmacksstörungen, Nahrungsmittelallergien, Chinese-Restaurant-Syndrom, Agression, Paranoia, Hirnerschütterung, neuroendokrine Störungen, Tourrette-Syndrom, cerebrovaskuläre Spasmen, neuronale Apoptose, Neurodegeneration, neuronale Nekrose, Astrocytose, Burn-out-Syndrom, Sudden-Infant-Death (plötzlicher Kindstod), Herzinfarkt, Insomnia, retrograde Amnesie, multiple Sklerose, Jet-lag, Störungen der Sexualfunktion, wie Impotenz oder Priapismus oder mit Wirksamkeit zur Förderung der Mikrogliaaktivierung, des Lernens, der Kognition oder des Gedächtnisses, zur Neuroprotektion, zur Liquordiagnostik neurostatischer Erkrankungen oder zur adjuvanten Therapie per Elektrostimulation des Nucleus subthalamicus bei Parkinson.

Ein weiterer Gegenstand der Erfindung ist ein Arzneimittel enthaltend mindestens
a. ein Polynukleotid, vorzugsweise eine DNA oder RNA, kodierend für BNPI oder DNPI oder ein Polynukleotids, vorzugsweise eine DNA oder RNA, welches einer der in einer der Abbildungen 1a), 1c), 1e), 1g), 2a), 2c) oder 2e) dargestellten Nukleotidsequenzen zu wenigstens 90%, vorzugsweise zu wenigstens 95%, insbesondere zu wenigstens 97% oder genau entspricht,
b. ein Polynukleotid, insbesondere ein Antisense Polynukleotid oder eine PNA, vorzugsweise ein DNA-Enzym oder Ribozym, eines Ribozym oder sonstiges DNA-Enzym oder eine katalytische RNA oder DNA, das eine Nukleotid-Sequenz aufweist, die in der Lage ist, spezifisch an eines der unter Punkt a) aufgeführten Polynukleotide zu binden,
c. einen Vektor enthaltend ein Polynukleotid gemäß einem der Punkte a) oder b), insbesondere ein Expressionsvektor und/oder insbesondere abgeleitet von einem Virus, beispielsweise dem Adenovirus, Adenoassoziiertem Virus oder Herpesvirus und/oder insbesondere enthaltend mindestens eine LTR-, Poly A-, Promotor- und/oder ORI-Sequenz,
d. BNPI und/oder DNPI und/oder ein Protein gemäß einer der Abbildungen 1b), 1d), 1f), 1h), 2b), 2d) oder 2f) und/oder ein zu einem dieser vorgenannten Proteine zu mindestens 90 %, vorzugsweise mindestens 95%, insbesondere mindestens 97% ähnliches Protein und/oder ein Protein, für das ein Polynukleotid gemäß einer der Abbildungen 1a), 1c), 1e), 1g), 2a), 2c) oder 2e) oder ein dazu zu mindestens 90 %, vorzugsweise mindestens 95%, insbesondere mindestens 97% ähnliches Polynukleotid kodiert, und/oder ein Protein, das durch eine Nukleinsäure kodiert wird, die unter stringenten Bedingungen an ein Polynukleotid gemäß einer der Abbildungen 1a), 1c), 1e), 1g) 2a), 2c) oder 2e) oder dessen Antisense Polynukleotide bindet oder ein mindestens 10, vorzugsweise mindestens 15, insbesondere mindestens 20 Aminosäuren langes Teilprotein eines der vorgenannten Proteine, wobei das Protein oder Teilprotein gegebenenfalls posttranslational modifiziert, insbesondere glykosiliert, phosphoryliert, amidiert, methyliert, acetyliert, ADP-ribosyliert, hydroxyliert, mit einem Membrananker versehen, gespalten oder verkürzt wurde,
e. einen Antikörper, vorzugsweise einen monoklonalen oder polyklonalen Antikörper, gegen eines der Proteine oder Teilproteine gemäß Punkt d),
f. eine Zelle, vorzugsweise eine Amphibienzelle, Bakterienzelle, Hefezelle, Insektenzelle oder eine immortalisierten oder native Säugetierzelle, enthaltend ein Polynukleotid gemäß einem der Punkte a) oder b), einen Vektor gemäß Punkt c), ein Protein oder Teilprotein gemäß Punkt d) oder einen Antikörper gemäß Punkt e)
g. eine erfindungsgemäße Verbindung identifizierbar als pharmazeutisch relevante Substanz mit Wirksamkeit in mindestens einer der vorgenannten Indikationen wie oben beschrieben und/oder
h. einen Wirkstoff, vorzugsweise einen niedermolekularen Wirkstoff, der an ein Protein oder Teilprotein gemäß Punkt d) bindet,
sowie gegebenenfalls enthaltend geeignete Zusatz- und/oder Hilfsstoffe und/oder gegebenenfalls weitere Wirkstoffe.

Die erfindungsgemäßen Arzneimittel enthalten neben mindestens einer erfindungsgemäß verwendeten Verbindung gegebenenfalls geeignete Zusatz- undloder Hilfsstoffe, so auch Trägermaterialien, Füllstoffe, Lösungsmittel, Verdünnungsmittel, Farbstoffe und/oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln, Pflaster oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, intranasal, buccal, rektal oder örtlich, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäß verwendete Verbindungen in einem Depot, in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäß verwendeten Verbindungen verzögert freisetzen. Prinzipiell können den erfindungsgemäßen Arzneimitteln andere dem Fachmann bekannte weitere Wirkstoffe zugesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,005 bis 1000 mg/kg appliziert.

Ein weiterer Gegenstand der Erfindung ist die Verwendung
a. eines Polynukleotids, vorzugsweise einer DNA oder RNA, kodierend für BNPI oder DNPI oder eines Polynukleotids, vorzugsweise einer DNA oder RNA, welches einer der in einer der Abbildungen 1a), 1c), 1e), 1g), 2a), 2c) oder 2e) dargestellten Nukleotidsequenzen zu wenigstens 90%, vorzugsweise zu wenigstens 95%, insbesondere zu wenigstens 97% entspricht,
b. eines Polynukleotids, insbesondere eines Antisense Polynukleotids oder einer PNA, vorzugsweise eines DNA-Enzyms oder Ribozyms, eines Ribozyms oder sonstigen DNA-Enzyms oder einer katalytischen RNA oder DNA, das eine Nukleotid-Sequenz aufweist, die in der Lage ist, spezifisch an eines der unter Punkt a) aufgeführten Polynukleotide zu binden,
c. eines Vektors enthaltend ein Polynukleotid gemäß einem der Punkte a) oder b), insbesondere eines Expressionsvektors und/oder insbesondere abgeleitet von einem Virus, beispielsweise dem Adenovirus, Adenoassoziiertem Virus öder Herpesvirus und/oder insbesondere enthaltend mindestens eine LTR-, Poly A-, Promotor- und/oder ORI-Sequenz,
f. einer Zelle, vorzugsweise einer Amphibienzelle, Bakterienzelle, Hefezelle, Insektenzelle oder einer immortalisierten oder nativen Säugetierzelle, enthaltend ein Polynukleotid gemäß einem der Punkte a) oder b) oder einen Vektor gemäß Punkt c)

zur Herstellung eines Arzneimittels zum Einsatz in der Gentherapie. Dabei ist es besonders bevorzugt, wenn es sich um In-vivo oder In-vitro Gentherapie handelt. Unter Gentherapie versteht man eine Therapieform, bei der durch die Einführung von Nukleinsäuren in Zellen ein Effektorgen, meist ein Protein, exprimiert wird. Man unterscheidet prinzipiell In-vivo- und In-vitro-Verfahren. In In-vitro-Verfahren werden Zellen aus dem Organismus entfernt und ex-vivo mit Vektoren transfiziert, um anschließend wieder in denselben oder in einen anderen Organismus eingebracht zu werden. Bei der In-vivo-Gentherapie werden Vektoren, beispielsweise zur Bekämpfung von Tumoren, systemisch (z.B. über die Blutbahn) oder direkt in das Zielgewebe (z.B. in einen Tumor) appliziert.

Bevorzugt ist es beim Einsatz in der Gentherapie weiter, wenn es sich um ein Arzneimittel mit Wirksamkeit in der Indikation oder zur Behandlung von
Sehstörungen, Retinitis pigmentosa, Opticus Degeneration, Hörstörungen, Tinitus, M. Menière, Hörsturz, Schizophrenie, Manien, Depression, Schlaganfall, Hirntrauma, Querschnittslähmung, amyotrophe Lateralsklerose, Neuralgie, Gewichtsregulation, Obesitas, Anorexia nervosa, Epilepsie, Hemibalismus, Chorea Huntington, Stress, Morbus Parkinson, TIA (Transiente Ischämische Attacken), Emesis, insbesondere Hyperemesis beispielsweise bei Chemotherapie, Schwindel, in jeglicher Form, Katarakt, Arthritis, Hyperaktivität, Entwicklungsstörungen, Tollwut, Virus-Infektionen oder bakterielle Infektionen, Grippe, Malaria, Creutzfeld-Jacob, Inflammatory Bowel disease, Morbus Crohn, cardiovaskuläre und cardiorespiratorische Funktionsstörungen, Hypertonie, Störungen der Baroafferenz oder Chemoafferenz, Toxoplasmose, Asthma, Autoimmunität im zentralen und peripheren Nervensystem, diabetische Neuropathie, autoimmuner Diabetes, alkoholische Neuropathie, HIV-Neuro-Aids; Störungen des autonomen Nervensystems, Störungen des Nervensystems des Verdauungstraktes, Übererregbarkeit, insbesondere glutamatvermittelte Übererregbarkeit, Neurodegeneration insbesondere bei Morbus Alzheimer, Morbus Alzheimer, Ischämie; Encephalitis insbesondere virale oder bakterielle; Prionerkrankung, Rasmussen-Encephalitis, HIV-Encephalitis, Demyelinisierung insbesondere bei multipler Sklerose, Retinadegeneration, Glaukom, Nystagmus, Netzhautablösung, Erkrankungen des Cerebellums, cerebelläre Ataxie, Erkrankungen der Basalganglien, Erkrankungen des Pallidums, Erkrankungen des Hör- und/oder Gleichgewichtsorgans, Erkrankungen der Hörbahn oder Vesibularbahn, Störungen des Gedächtnisses, Störungen des Lernens, Störungen der Kognition, Stiff-Man-Syndrom, Restless Leg-Syndrom, Angstzustände, Phobien, Schlafstörungen; Drogenabhängigkeit, -sucht und -entzug insbesondere bei Alkohol, Nikotin, Opiaten, Ecstasy oder Kokain; Hepatoencephalopathie mit Alkoholintoxikation, Hepatoencephalopathie ohne Alkoholintoxikation, neurotoxikologisch bedingte Erkrankungen, Erkrankungen des spinalen Motoneurons, Muskelatrophien, Muskeldystrophien, Erkrankungen der Hinterstrangbahnen, alkoholische Neuropathien, Neuroinflammation, Befindlichkeitsstörungen bei Infektionen oder Fieber, Stress, Geschmacksstörungen, Nahrungsmittelallergien, Chinese-Restaurant-Syndrom, Agression, Paranoia, Hirnerschütterung, neuroendokrine Störungen, Tourrette-Syndrom, cerebrovaskuläre Spasmen, neuronale Apoptose, Neurodegeneration, neuronale Nekrose, Astrocytose, Burn-out-Syndrom, Sudden-Infant-Death (plötzlicher Kindstod), Herzinfarkt, Insomnia, retrograde Amnesie, multiple Sklerose, Jet-lag, Störungen der Sexualfunktion, wie Impotenz oder Priapismus oder mit Wirksamkeit zur Förderung der Mikrogliaaktivierung, des Lernens, der Kognition oder des Gedächtnisses, zur Neuroprotektion, zur Liquordiagnostik neurostatischer Erkrankungen oder zur adjuvanten Therapie per Elektrostimulation des Nucleus subthalamicus bei Parkinson
handelt.

Bevorzugt beim Einsatz in der Gentherapie ist auch die Verwendung eines Polynukleotids, beim dem es sich um ein Antisense Polynukleotid oder PNA handelt, oder das Teil eines Ribozyms oder sonstigen DNA-Enzyms oder einer katalytischen RNA oder DNA ist.

Ein weiterer Gegenstand der Erfindung ist auch die Verwendung
a. eines Polynukleotids, vorzugsweise einer DNA oder RNA, kodierend für BNPI oder DNPI oder eines Polynukleotids, vorzugsweise einer DNA oder RNA, welches einer der in einer der Abbildungen 1a), 1c), 1e), 1g), 2a), 2c) oder 2e) dargestellten Nukleotidsequenzen zu wenigstens 90%, vorzugsweise 95%, insbesondere zu wenigstens 97% entspricht,
b. eines Polynukleotids, insbesondere eines Antisense Polynukleotids oder einer PNA, vorzugsweise eines DNA-Enzyms oder Ribozyms, eines Ribozyms oder sonstigen DNA-Enzyms oder einer katalytischen RNA oder DNA, das eine Nukleotid-Sequenz aufweist, die in der Lage ist, spezifisch an eines der unter Punkt a) aufgeführten Polynukleotide zu binden,
c. eines Vektors enthaltend ein Polynukleotid gemäß einem der Punkte a) oder b), insbesondere eines Expressionsvektors und/oder insbesondere abgeleitet von einem Virus, beispielsweise dem Adenovirus, Adenoassoziiertem Virus oder Herpesvirus und/oder insbesondere enthaltend mindestens eine LTR-, Poly A-, Promotor- und/oder ORI-Sequenz,
d. von BNPI und/oder DNPI und/oder eines Proteins gemäß einer der Abbildungen 1b), 1d), 1f), 1h), 2b), 2d) oder 2f) und/oder eines zu einem dieser vorgenannten Proteine zu mindestens 90 %, vorzugsweise mindestens 95%, insbesondere mindestens 97% ähnlichen Proteins und/oder eines Proteins, für das ein Polynukleotid gemäß einer der Abbildungen 1a), 1c), 1e), 1g), 2a), 2c) oder 2e) oder ein dazu zu mindestens 90 %, vorzugsweise mindestens 95%, insbesondere mindestens 97% ähnliches Polynukleotid kodiert, und/oder eines Proteins, das durch eine Nukleinsäure kodiert wird, die unter stringenten Bedingungen an ein Polynukleotid gemäß einer der Abbildungen 1a), 1c), 1e), 1g), 2a), 2c) oder 2e) oder dessen Antisense Polynukleotide bindet oder eines mindestens 10, vorzugsweise mindestens 15, insbesondere mindestens 20 Aminosäuren langen Teilproteins eines der vorgenannten Proteine, wobei das Protein oder Teilprotein gegebenenfalls posttranslational modifiziert, insbesondere glykosiliert, phosphoryliert, amidiert, methyliert, acetyliert, ADP-ribosyliert, hydroxyliert, mit einem Membrananker versehen, gespalten oder verkürzt wurde,
e. eines Antikörpers, vorzugsweise eines monoklonalen oder polyklonalen Antikörpers, gegen eines der Proteine oder Teilproteine gemäß Punkt d),
f. einer Zelle, vorzugsweise einer Amphibienzelle, Bakterienzelle, Hefezelle, Insektenzelle oder einer immortalisierten oder nativen Säugetierzelle, enthaltend ein Polynukleotid gemäß einem der Punkte a) oder b), einen Vektor gemäß Punkt c), ein Protein oder Teilprotein gemäß Punkt d) oder einen Antikörper gemäß Punkt e),
g. einer erfindungsgemäßen Verbindung identifizierbar als pharmazeutisch relevante Substanz mit Wirksamkeit in mindestens einer der vorgenannten Indikationen wie oben beschrieben und/oder
h. eines Wirkstoffs, vorzugsweise eines niedermolekularen Wirkstoffs, der an ein Protein oder Teilprotein gemäß Punkt d) bindet,
zur Herstellung eines Diagnostikums zur Diagnose eines Zustands ausgewählt aus

Sehstörungen, Retinitis pigmentosa, Opticus Degeneration, Hörstörungen, Tinitus, M. Menière, Hörsturz, Schizophrenie, Manien, Depression, Schlaganfall, Hirntrauma, Querschnittslähmung, amyotrophe Lateralsklerose, Neuralgie, Gewichtsregulation, Obesitas, Anorexia nervosa, Epilepsie, Hemibalismus, Chorea Huntington, Stress, Morbus Parkinson, TIA (Transiente Ischämische Attacken), Emesis, insbesondere Hyperemesis beispielsweise bei Chemotherapie, Schwindel, in jeglicher Form, Katarakt, Arthritis, Hyperaktivität, Entwicklungsstörungen, Tollwut, Virus-Infektionen oder bakterielle Infektionen, Grippe, Malaria, Creutzfeld-Jacob, Inflammatory Bowel disease; Morbus Crohn, cardiovaskuläre und cardiorespiratorische Funktionsstörungen, Hypertonie, Störungen der Baroafferenz oder Chemoafferenz, Toxoplasmose, Asthma, Autoimmunität im zentralen und peripheren Nervensystem, diabetische Neuropathie, autoimmuner Diabetes, alkoholische Neuropathie, HIV-Neuro-Aids; Störungen des autonomen Nervensystems, Störungen des Nervensystems des Verdauungstraktes, Übererregbarkeit, insbesondere glutamatvermittelte Übererregbarkeit, Neurodegeneration insbesondere bei Morbus Alzheimer, Morbus Alzheimer, Ischämie; Encephalitis insbesondere virale oder bakterielle; Prionerkrankung, Rasmussen-Encephalitis, HIV-Encephalitis, Demyelinisierung insbesondere bei multipler Sklerose, Retinadegeneration, Glaukom, Nystagmus, Netzhautablösung, Erkrankungen des Cerebellums, cerebelläre Ataxie, Erkrankungen der Basalganglien, Erkrankungen des Pallidums, Erkrankungen des Hör- und/oder Gleichgewichtsorgans, Erkrankungen der Hörbahn oder Vesibularbahn, Störungen des Gedächtnisses, Störungen des Lernens, Störungen der Kognition, Stiff-Man-Syndrom, Restless Leg-Syndrom, Angstzustände, Phobien, Schlafstörungen; Drogenabhängigkeit, -sucht und -entzug insbesondere bei Alkohol, Nikotin, Opiaten, Ecstasy oder Kokain; Hepatoencephalopathie mit Alkoholintoxikation, Hepatoencephalopathie ohne Alkoholintoxikation, neurotoxikologisch bedingte Erkrankungen, Erkrankungen des spinalen Motoneurons, Muskelatrophien, Muskeldystrophien, Erkrankungen der Hinterstrangbahnen, alkoholische Neuropathien, Neuroinflammation, Befindlichkeitsstörungen bei Infektionen oder Fieber, Stress, Geschmacksstörungen, Nahrungsmittelallergien, Chinese-Restaurant-Syndrom, Agression, Paranoia, Hirnerschütterung, neuroendokrine Störungen, Tourrette-Syndrom, cerebrovaskuläre Spasmen, neuronale Apoptose, Neurodegeneration, neuronale Nekrose, Astrocytose, Burn-out-Syndrom, Sudden-Infant-Death (plötzlicher Kindstod), Herzinfarkt, Insomnia, retrograde Amnesie, multiple Sklerose, Jet-lag, Störungen der Sexualfunktion, wie Impotenz oder Priapismus.

Dabei versteht man unter Diagnostik die Analyse von einem Krankheitsbild zugeordneten Symptomen und unter Wirksamkeitsuntersuchungen Untersuchungen über die Wirksamkeit zu testender Substanzen, insbesondere ihrer medizinischen Wirksamkeit.

Ein weiterer Gegenstand der Erfindung ist auch ein Verfahren zur Herstellung eines erfindungsgemäßen Peptids oder Proteins, bei dem eine erfindungsgemäße Zelle, die ein erfindungsgemäßes Polynukleotid und/oder einen erfindungsgemäßen Vektor enthält, kultiviert und gegebenfalls das Peptid oder Protein isoliert wird.

Ein weiterer Gegenstand der Erfindung ist die Verwendung
a. eines Polynukleotids , vorzugsweise einer DNA oder RNA, kodierend für BNPI oder DNPI oder eines Polynukleotids, vorzugsweise einer DNA oder RNA, welches einer der in einer der Abbildungen 1a), 1c), 1e), 1g), 2a), 2c) oder 2e) dargestellten Nukleotidsequenzen zu wenigstens 90%, vorzugsweise 95%, insbesondere zu wenigstens 97% entspricht,
b. eines Polynukleotids, insbesondere eines Antisense Polynukleotids oder einer PNA, vorzugsweise eines DNA-Enzyms oder Ribozyms, eines Ribozyms oder sonstigen DNA-Enzyms oder einer katalytischen RNA oder DNA, das eine Nukleotid-Sequenz aufweist, die in der Lage ist, spezifisch an eines der unter Punkt a) aufgeführten Polynukleotide zu binden,
c. eines Vektors enthaltend ein Polynukleotid gemäß einem der Punkte a) oder b), insbesondere eines Expressionsvektors und/oder insbesondere abgeleitet von einem Virus, beispielsweise dem Adenovirus, Adenoassoziiertem Virus oder Herpesvirus und/oder insbesondere enthaltend mindestens eine LTR-, Poly A-, Promotor- und/oder ORI-Sequenz,
d. von BNPI und/oder DNPI und/oder eines Proteins gemäß einer der Abbildungen 1b), 1d), 1f), 1h), 2b), 2d) oder 2f) und/oder eines zu einem dieser vorgenannten Proteine zu mindestens 90 %, vorzugsweise mindestens 95%, insbesondere mindestens 97% ähnlichen Proteins und/oder eines Proteins, für das ein Polynukleotid gemäß einer der Abbildungen 1a), 1c), 1e), 1g), 2a), 2c) oder 2e) oder ein dazu zu mindestens 90 %, vorzugsweise mindestens 95%, insbesondere mindestens 97% ähnliches Polynukleotid kodiert, und/oder eines Proteins, das durch eine Nukleinsäure kodiert wird, die unter stringenten Bedingungen an ein Polynukleotid gemäß einer der Abbildungen 1a), 1c), 1e), 1g), 2a), 2c) oder 2e) oder dessen Antisense Polynukleotide bindet oder eines mindestens 10, vorzugsweise mindestens 15, insbesondere mindestens 20 Aminosäuren langen Teilproteins eines der vorgenannten Proteine, wobei das Protein oder Teilprotein gegebenenfalls posttranslational modifiziert, insbesondere glykosiliert, phosphoryliert, amidiert, methyliert, acetyliert, ADP-ribosyliert, hydroxyliert, mit einem Membrananker versehen, gespalten oder verkürzt wurde,
e. eines Antikörpers, vorzugsweise eines monoklonalen oder polyklonalen Antikörpers, gegen eines der Proteine oder Teilproteine gemäß Punkt d),
f. einer Zelle, vorzugsweise einer Amphibienzelle, Bakterienzelle, Hefezelle, Insektenzelle oder einer immortalisierten oder nativen Säugetierzelle, enthaltend ein Polynukleotid gemäß einem der Punkte a) oder b), einen Vektor gemäß Punkt c), ein Protein oder Teilprotein gemäß Punkt d) oder einen Antikörper gemäß Punkt e)
in einem Verfahren zur Auffindung pharmazeutisch relevanter Substanzen mit Wirksamkeit in den Indikationen oder zur Behandlung von

Sehstörungen, Retinitis pigmentosa, Opticus Degeneration, Hörstörungen, Tinitus, M. Meniere, Hörsturz, Schizophrenie, Manien, Depression, Schlaganfall, Hirntrauma, Querschnittslähmung, amyotrophe Lateralsklerose, Neuralgie, Gewichtsregulation, Obesitas, Anorexia nervosa, Epilepsie, Hemibalismus, Chorea Huntington, Stress, Morbus Parkinson, TIA (Transiente Ischämische Attacken), Emesis, insbesondere Hyperemesis beispielsweise bei Chemotherapie, Schwindel, in jeglicher Form, Katarakt, Arthritis, Hyperaktivität, Entwicklungsstörungen, Tollwut, Virus-Infektionen oder bakterielle Infektionen, Grippe, Malaria, Creutzfeld-Jacob, Inflammatory Bowel disease, Morbus Crohn, cardiovaskuläre und cardiorespiratorische Funktionsstörungen, Hypertonie, Störungen der Baroafferenz oder Chemoafferenz, Toxoplasmose, Asthma, Autoimmunität im zentralen und peripheren Nervensystem, diabetische Neuropathie, autoimmuner Diabetes, alkoholische Neuropathie, HIV-Neuro-Aids; Störungen des autonomen Nervensystems, Störungen des Nervensystems des Verdauungstraktes, Übererregbarkeit, insbesondere glutamatvermittelte Übererregbarkeit, Neurodegeneration insbesondere bei Morbus Alzheimer, Morbus Alzheimer, Ischämie; Encephalitis insbesondere virale oder bakterielle; Prionerkrankung, Rasmussen-Encephalitis, HIV-Encephalitis, Demyelinisierung insbesondere bei multipler Sklerose, Retinadegeneration, Glaukom, Nystagmus, Netzhautablösung, Erkrankungen des Cerebellums, cerebelläre Ataxie, Erkrankungen der Basalganglien, Erkrankungen des Pallidums, Erkrankungen des Hör- und/oder Gleichgewichtsorgans, Erkrankungen der Hörbahn oder Vesibularbahn, Störungen des Gedächtnisses, Störungen des Lernens, Störungen der Kognition, Stiff-Man-Syndrom, Restless Leg-Syndrom, Angstzustände, Phobien, Schlafstörungen; Drogenabhängigkeit, -sucht und -entzug insbesondere bei Alkohol, Nikotin, Opiaten, Ecstasy oder Kokain; Hepatoencephalopathie mit Alkoholintoxikation, Hepatoencephalopathie ohne Alkoholintoxikation, neurotoxikologisch bedingte Erkrankungen, Erkrankungen des spinalen Motoneurons, Muskelatrophien, Muskeldystrophien, Erkrankungen der Hinterstrangbahnen, alkoholische Neuropathien, Neuroinflammation, Befindlichkeitsstörungen bei Infektionen oder Fieber, Stress, Geschmacksstörungen, Nahrungsmittelallergien, Chinese-Restaurant-Syndrom, Agression, Paranoia, Hirnerschütterung, neuroendokrine Störungen, Tourrette-Syndrom, cerebrovaskuläre Spasmen, neuronale Apoptose, Neurodegeneration, neuronale Nekrose, Astrocytose, Burn-out-Syndrom, Sudden-Infant-Death (plötzlicher Kindstod), Herzinfarkt, Insomnia, retrograde Amnesie, multiple Sklerose, Jet-lag, Störungen der Sexualfunktion, wie Impotenz oder Priapismus oder mit Wirksamkeit zur Förderung der Mikrogliaaktivierung, des Lernens, der Kognition oder des Gedächtnisses, zur Neuroprotektion, zur Liquordiagnostik neurostatischer Erkrankungen oder zur adjuvanten Therapie per Elektrostimulation des Nucleus subthalamicus bei Parkinson.

Generell ist es bei allen vorgenannten erfindungsgemäßen Verwendungen bevorzugt, wenn die Indikation oder die zu behandelnde oder zu diagnostizierende Krankheit ausgewählt ist aus
Sehstörungen, Retinitis pigmentosa, Opticus Degeneration, Hörstörungen, Tinitus, M. Menière, Hörsturz amyotrophe Lateralsklerose, Neuralgie, Gewichtsregulation, Obesitas, Morbus Parkinson, Katarakt, Virus-Infektionen oder bakterielle Infektionen, diabetische Neuropathie, autoimmuner Diabetes, alkoholische Neuropathie, HIV-Neuro-Aids; Retinadegeneration, Glaukom, Nystagmus, Netzhautablösung, Erkrankungen des Hör- und/oder Gleichgewichtsorgans, Erkrankungen der Hörbahn oder Vesibularbahn, Schlafstörungen, Drogenabhängigkeit, -sucht und -entzug insbesondere bei Alkohol, Nikotin, Opiaten, Ecstasy oder Kokain; Neuroinflammation, Insomnia, zur adjuvanten Therapie per Elektrostimulation des Nucleus subthalamicus bei Parkinson; bzw. Erkrankungen des spinalen Motoneurons, Muskelatrophien oder Muskeldystrophien,
vorzugsweise
Sehstörungen, Retinitis pigmentosa, Opticus Degeneration, Hörstörungen, Tinitus, M. Menière, Hörsturz amyotrophe Lateralsklerose, Gewichtsregulation, Obesitas, Katarakt, Virus-Infektionen oder bakterielle Infektionen, Retinadegeneration, Glaukom, Nystagmus, Netzhautablösung, Erkrankungen des Hör- und/oder Gleichgewichtsorgans, Erkrankungen der Hörbahn oder Vesibularbahn, Drogenabhängigkeit, -sucht und - entzug insbesondere bei Alkohol, Nikotin, Opiaten, Ecstasy oder Kokain; Neuroinflammation; bzw. Erkrankungen des spinalen Motoneurons, Muskelatrophien oder Muskeldystrophien,
insbesondere
Sehstörungen, Retinitis pigmentosa, Opticus Degeneration, Katarakt, Netzhautablösung, Retinadegeneration, Glaukom oder Nystagmus
und/oder
Hörstörungen, Tinitus, M. Meniere, Hörsturz, Erkrankungen des Hör- und/oder Gleichgewichtsorgans oder Erkrankungen der Hörbahn oder Vesibularbahn;
bzw. Erkrankungen des spinalen Motoneurons, Muskelatrophien oder Muskeldystrophien.

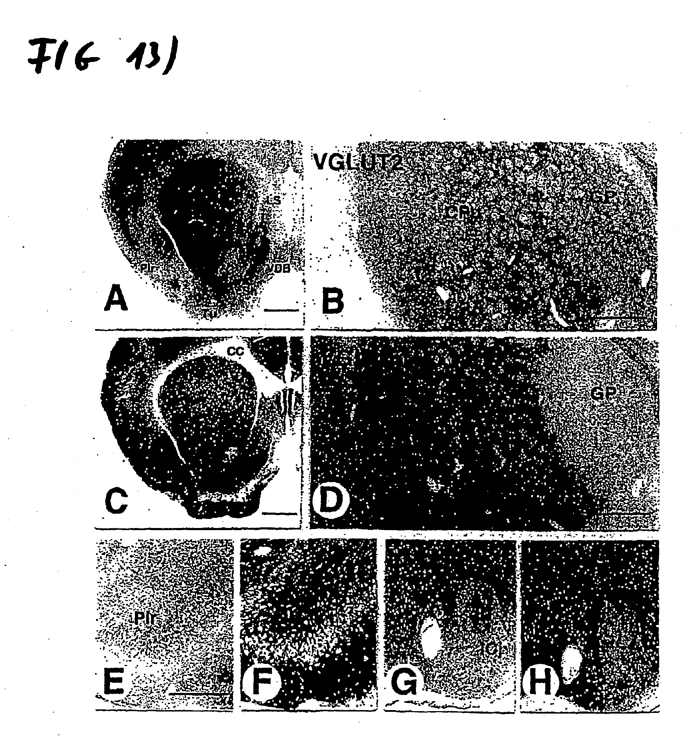

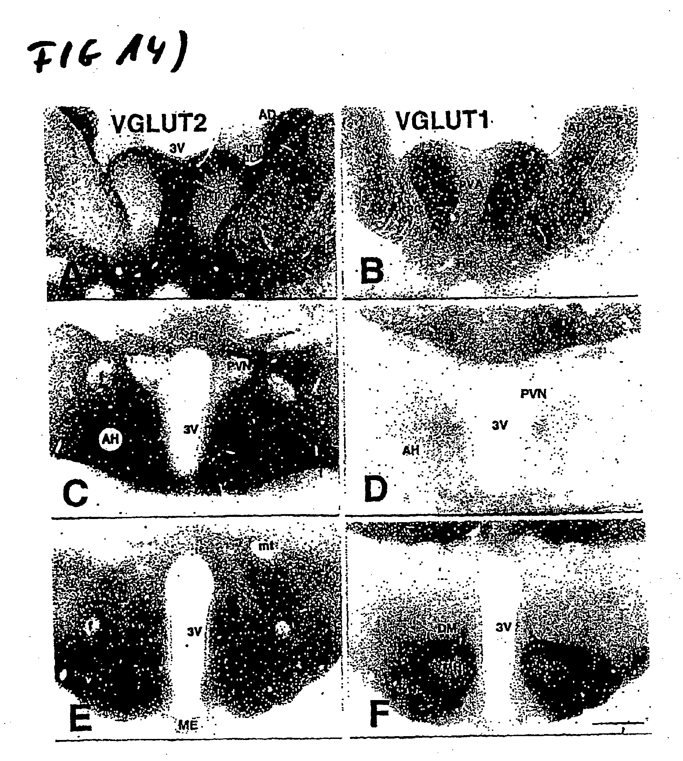

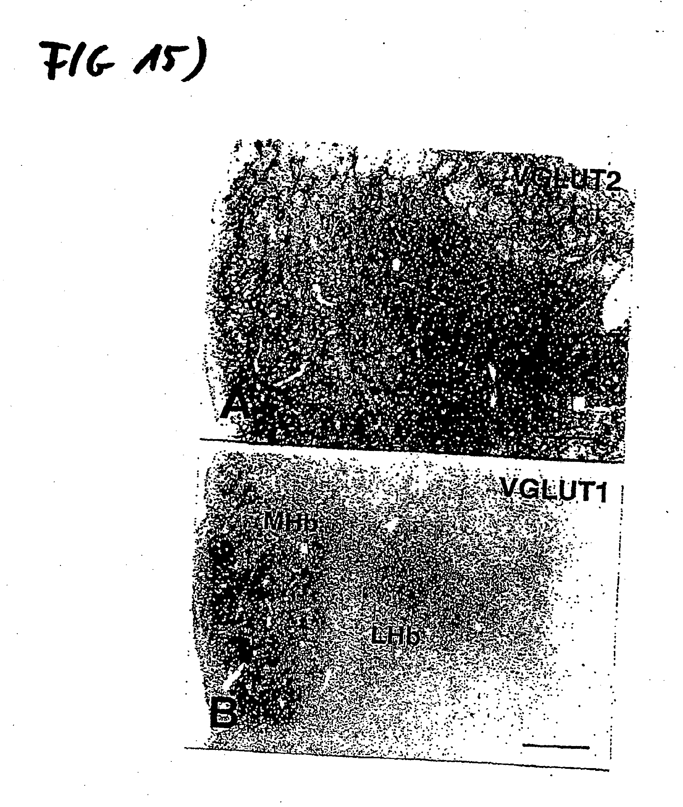

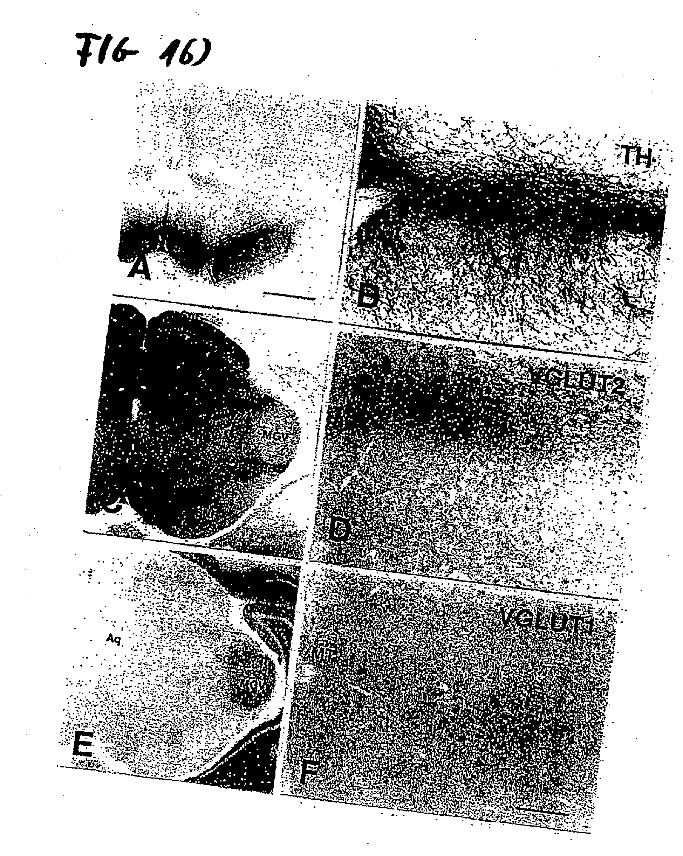

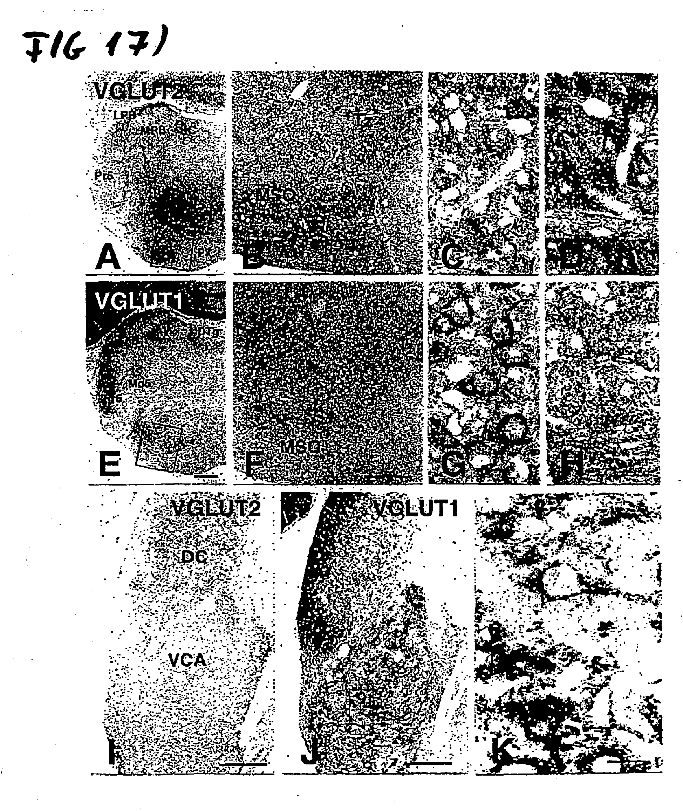

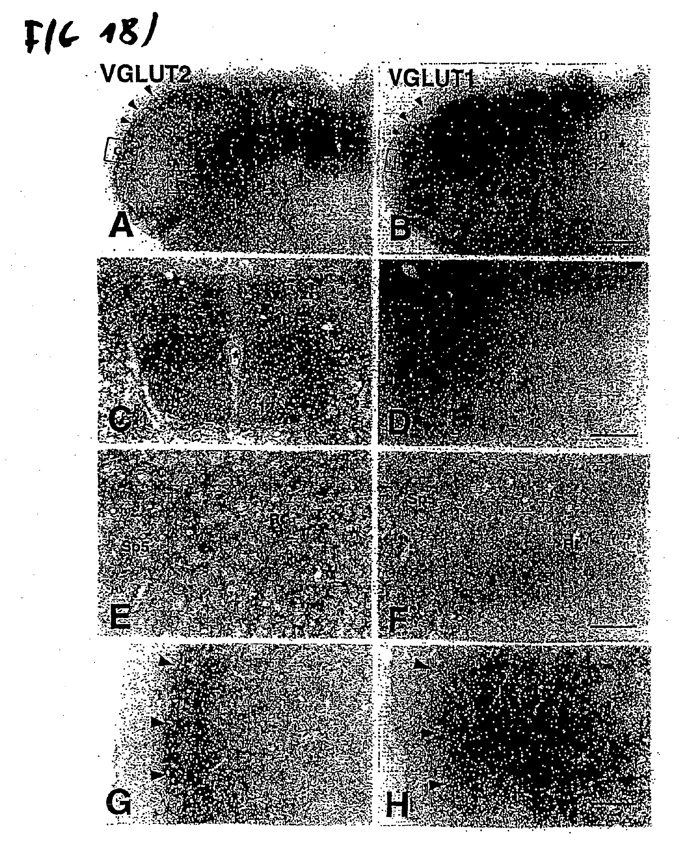

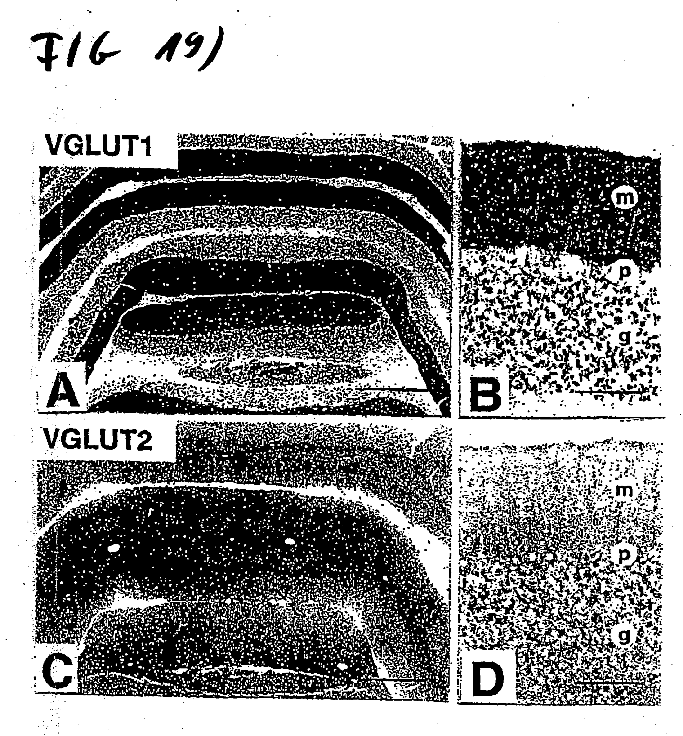

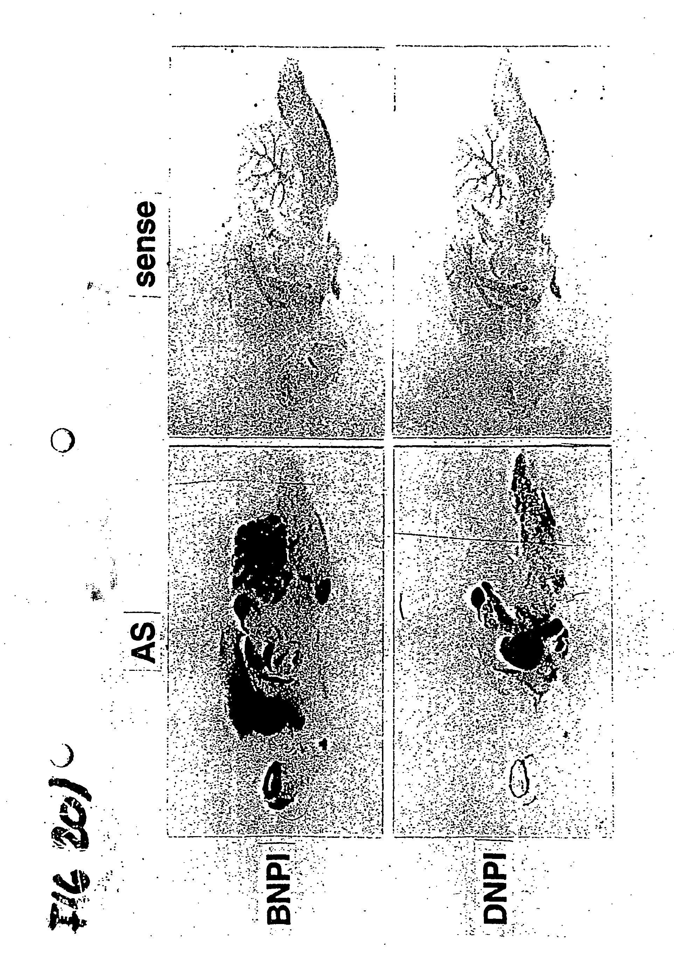

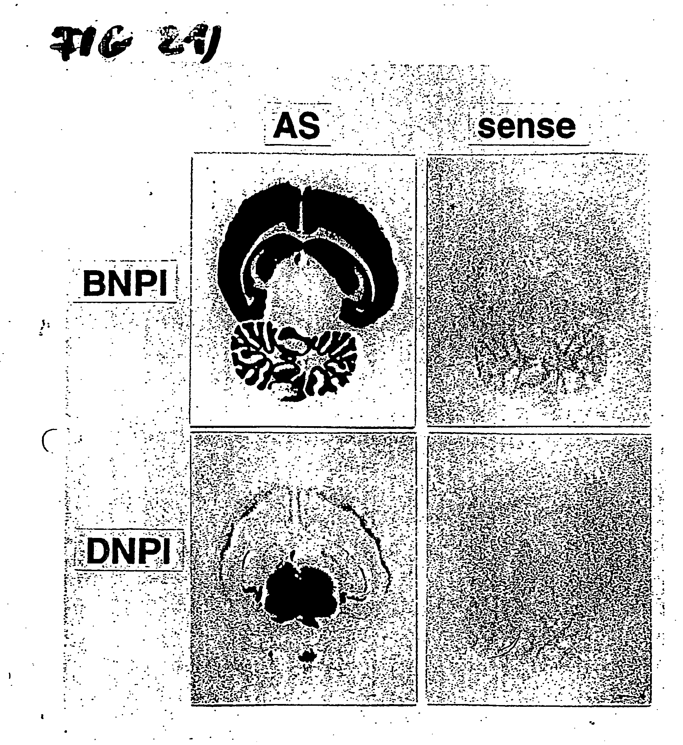

Mit den erfindungsgemäß verwendeten Polynukleotid sind auch die dargestellten Genfragmente selbst umfaßt, wie auch ein Polynukleotid, das entweder vollständig oder zumindest Teilen der kodierenden Sequenz des dem Fragment entsprechenden Gens entspricht. Damit sind auch Polynukleotide gemeint, die mindestens 90%-ige, vorzugsweise 95%-ige, insbesondere wenigstens 97%-ige Übereinstimmung in der Basenabfolge mit der kodierenden Sequenz der abgebildeten Polynukleotide oder der kodierenden Sequenz der Gens aufweisen. Es ist weiter bevorzugt, daß es sich bei dem Polynukleotid um RNA bzw. ein- oder doppelstängige DNA, insbesondere mRNA oder cDNA handelt. Ebenso ist es bevorzugt, daß es sich bei dem Polynukleotid um ein Antisense-Polynukleotid oder PNA handelt, das eine Sequenz aufweist, die in der Lage ist, spezifisch an ein erfindungsgemäßes Polynukleotid zu binden. Dabei versteht man unter PNA "peptidic nucleic acid" (peptidische Nukleinsäure), die zwar die Basenpaare trägt, aber dessen Rückrat peptidisch gebunden ist. Ein Antisense-Polynukleotid zeigt die komplementäre Basenabfolge zu mindestens einem Teil einer Basis-Nukleinsäure. Ebenfalls bevorzugt ist es, daß das Polynukleotid Teil eines Ribozym oder sonstigen DNA-Enzyms oder einer katalytischen RNA bzw. DNA ist. Unter Ribozym ist eine katalytisch aktive Ribonukleinsäure zu verstehen, unter DNA-Enzym ein entsprechende Desoxyribonukleinsäure, also katalytische RNA beziehungsweise DNA.
Ein ganz besonders bevorzugter Gegenstand der Erfindung ist ein, insbesondere erfindungsgemäß verwendetes Polynukleotid oder auch Oligonukleotid, bei dem mindestens eines der Nukleotide, insbesondere mehrere der Nukleotide, "Locked Nucleic Acids" ("LNA's") sind oder mindestens eines der Nukleotide, insbesondere alle Nukleotide, Phosphorothioate sind, vorzugsweise ein solches, bei dem mehrere der Nukleotide "Locked Nucleic Acids" ("LNA's") sind. "Locked nucleic acids" ("LNA's") sind Ribonukleotide, die eine Methylen-Brücke enthalten, die den 2'-Sauerstoff der Ribose mit dem 4'-Kohlenstoff verbindet (s. Abb. 27). Einen Überblick über die LNA's geben Braasch D.A. und Corey, D.R. (2001), Locked nucleic acids (LNA); fine-tuning the recognition of DNA und RNA. Chem. Biol. 8, 1-7. Dieser Artikel ist ausdrücklich Mitbestandteil der vorliegenden Beschreibung und Offenbarung. LNA's werden beispielsweise von der Firma Proligo, Boulder, CO, USA angeboten. Auch Phosphorothiate sind dem Fachmann bekannt und können beispielsweise bei MWG-Biotech AG, Ebersberg, Germany bestellt werden.

Unter dem erfindungsgemäß verwendeten Vektor versteht man ein Nukleinsäuremolekül, das bei gentechnischer Manipulation dazu dient, Fremdgene zu enthalten bzw. zu übertragen. Besonders bevorzugt ist dabei, daß es sich um einen Expressionsvektor handelt. Er dient damit der Expression des enthaltenen Fremdgens, des Polynukleotids. Weiter bevorzugt ist ein derartiger Vektor, der abgeleitet ist von einem Virus, beispielsweise dem Adenovirus, Adenoassoziiertem Virus oder Herpesvirus und/oder er mindestens eine LTR-, Poly A-, Promotor- und/oder ORI-Sequenz enthält. Ein LTR ist ein "Long-TerminalRepeat", ein am Ende befindlicher Abschnitt beispielsweise bei Viren. Poly-A-Sequenz ist ein mehr als 20 Adenosinreste langer Schwanz. Eine Promotorsequenz ist der Steuerungsbereich für die Transkription.

Bevorzugt ist es für ein verwendetes Protein bzw. ein daraus abgeleitetes Teilprotein, wenn dieses posttranslational modifiziert wurde, es insbesondere glykosiliert, phosphoryliert, amidiert, methyliert, acetyliert, ADP-ribosyliert, hydroxyliert, mit einem Membrananker versehen, gespalten oder verkürzt wurde. Posttranslationale Modifikationen sind beispielsweise dem Voet/Voet, Biochemistry, 1^{st} Edition, 1990, S. 935-938 zu entnehmen.

Dabei ist es für eine erfindungsgemäße Verwendung besonders bevorzugt, wenn das Polynukleotid (gegebenfalls gemäß Punkt a) und/oder Punkt b)) eine RNA oder eine ein- oder doppelstängige DNA, insbesondere mRNA oder cDNA ist.

Dabei ist es für eine erfindungsgemäße Verwendung besonders bevorzugt, wenn das Polynukleotid (gegebenfalls gemäß Punkt b)) Teil eines Ribozyms oder sonstigen DNA-Enzyms oder einer katalytischen RNA oder DNA ist.

Dabei ist es für eine erfindungsgemäße Verwendung besonders bevorzugt, wenn der Vektor (gegebenfalls gemäß Punkt c)) ein Expressionsvektor ist.

Dabei ist es weiter für eine erfindungsgemäße Verwendung besonders bevorzugt, wenn der Vektor (gegebenfalls gemäß Punkt c)) abgeleitet ist von einem Virus, beispielsweise dem Adenovirus, Adenoassoziiertem Virus oder Herpesvirus und/oder er mindestens eine LTR-, Poly A-, Promotor- und/oder ORI-Sequenz enthält.

Dabei ist es für eine erfindungsgemäße Verwendung (nicht Gentherapie) besonders bevorzugt, wenn das Protein oder Teilprotein (gegebenfalls gemäß Punkt d)) posttranslational modifiziert wurde, es insbesondere glykosiliert, phosphoryliert, amidiert, methyliert, acetyliert, ADP-ribosyliert, hydroxyliert, mit einem Membrananker versehen, gespalten oder verkürzt wurde.

Dabei ist es für eine erfindungsgemäße Verwendung (nicht Gentherapie) besonders bevorzugt, wenn der Antikörper (gegebenenfalls gemäß Punkt e)) ein monoklonaler oder polyklonaler Antikörper ist.

Dabei ist es für eine erfindungsgemäße Verwendung besonders bevorzugt, wenn es sich bei der Zelle (gegebenenfalls gemäß Punkt f)) um eine Amphibienzelle, Bakterienzelle, Hefezelle, Insektenzelle oder eine immortalisierte oder native Säugetierzelle handelt.

Dabei ist es für eine erfindungsgemäße Verwendung besonders bevorzugt, wenn es sich bei der Verbindung (gegebenenfalls gemäß Punkt g)) um eine niedermolekulare Verbindung handelt.

Dabei ist es für eine erfindungsgemäße Verwendung besonders bevorzugt, wenn der genannte Wirkstoff gemäß Punkt h) ein niedermolekularer Wirkstoff ist.

Ein weiterer Gegenstand der Erfindung ist ein Verfahren zur Behandlung, eines nichthumanen Säugetieres oder Menschen, das oder der eine Behandlung von Sehstörungen, Retinitis pigmentosa, Opticus Degeneration, Hörstörungen, Tinitus, M. Menière, Hörsturz, Schizophrenie, Manien, Depression, Schlaganfall, Hirntrauma, Querschnittslähmung, amyotrophe Lateralsklerose, Neuralgie, Gewichtsregulation, Obesitas, Anorexia nervosa, Epilepsie, Hemibalismus, Chorea Huntington, Stress, Morbus Parkinson, Alzheimer, TIA (Transiente Ischämische Attacken), Emesis, insbesondere Hyperemesis beispielsweise bei Chemotherapie, Schwindel, in jeglicher Form, Katarakt, Arthritis, Hyperaktivität, Entwicklungsstörungen, Tollwut, Virus-Infektionen oder bakterielle Infektionen, Grippe, Malaria, Creutzfeld-Jacob, Inflammatory Bowel disease, Morbus Crohn, cardiovaskuläre und cardiorespiratorische Funktionsstörungen, Hypertonie, Störungen der Baroafferenz oder Chemoafferenz, Toxoplasmose, Asthma, Autoimmunität im zentralen und peripheren Nervensystem, diabetische Neuropathie, autoimmuner Diabetes, alkoholische Neuropathie, HIV-Neuro-Aids; Störungen des autonomen Nervensystems, Störungen des Nervensystems des Verdauungstraktes, Übererregbarkeit, insbesondere glutamatvermittelte Übererregbarkeit, Neurodegeneration insbesondere bei Morbus Alzheimer, Morbus Alzheimer, lschämie; Encephalitis insbesondere virale oder bakterielle; Prionerkrankung, Rasmussen-Encephalitis, HIV-Encephalitis, amyotrophe Lateralsklerose, Demyelinisierung insbesondere bei multipler Sklerose, Retinadegeneration, Glaukom, Nystagmus, Netzhautablösung, Erkrankungen des Cerebellums, cerebelläre Ataxie, Erkrankungen der Basalganglien, Erkrankungen des Pallidums, Erkrankungen des Hör- und/oder Gleichgewichtsorgans, Erkrankungen der Hörbahn oder Vesibularbahn, Störungen des Gedächtnisses, Störungen des Lernens, Störungen der Kognition, Stiff-Man-Syndrom, Restless Leg-Syndrom, Angstzustände, Phobien, Schlafstörungen, Anorexia nervosa; Drogenabhängigkeit, -sucht und -entzug, insbesondere bei Alkohol, Nikotin, Opiaten, Ecstasy oder Kokain; Hepatoencephalopathie mit Alkoholintoxikation, Hepatoencephalopathie ohne Alkoholintoxikation, neurotoxikologisch bedingte Erkrankungen, Erkrankungen des spinalen Motoneurons, Muskelatrophien, Muskeldystrophien, Erkrankungen der Hinterstrangbahnen, alkoholische Neuropathien, Neuroinflammation, Befindlichkeitsstörungen bei Infektionen oder Fieber, Stress, Geschmacksstörungen, Nahrungsmittelallergien, Chinese-Restaurant-Syndrom, Agression, Paranoia, Hirnerschütterung, neuroendokrine Störungen, Tourrette-Syndrom, cerebrovaskuläre Spasmen, neuronale Apoptose, Neurodegeneration, neuronale Nekrose, Astrocytose, Burn-out-Syndrom, Sudden-Infant-Death (plötzlicher Kindstod), Herzinfarkt, Insomnia, retrograde Amnesie, multiple Sklerose, Jet-lag, Störungen der Sexualfunktion, wie Impotenz oder Priapismus oder eine Förderung der Mikrogliaaktivierung, des Lernens, der Kognition oder des Gedächtnisses, Neuroprotektion, Liquordiagnostik neurostatischer Erkrankungen oder adjuvante Therapie per Elektrostimulation des Nucleus subthalamicus bei Parkinson benötigt; durch Verabreichung eines erfindungsgemäßen Arzneimittels, insbesondere solche enthaltend eine erfindungsgemäße Substanz und/oder einen an BNPI und/oder DNPI bindenden Wirkstoff.

Die Verabreichung kann beispielsweise in Form eines Arzneimittels wie oben beschrieben erfolgen.

Die folgenden Beispiele und Abbildungen sollen die Erfindung erläutern, ohne sie darauf zu beschränken.

### Abbildungen und Beispiele

### Abbildungen:

Fig. 1a) cDNA-Sequenz von BNPI, human; AN: NM_020309
Fig. 1b) Aminosäure-Sequenz von BNPI, human; AN: NM_020309
Fig. 1c) cDNA-Sequenz von BNPI, human; Nr.: AAT42064 aus WO96/34288
Fig. 1d) Aminosäure-Sequenz von BNPI, human; Nr.: AAT42064 aus WO96/34288
Fig. 1e) cDNA-Sequenz von BNPI, Ratte; AN: U07609
Fig. 1f) Aminosäure-Sequenz von BNPI, Ratte; AN: U07609
Fig. 1g) cDNA-Sequenz von BNPI, Maus; AN: XM_133432
Fig. 1h) Aminosäure-Sequenz von BNPI, Maus; AN: XM_133432
Fig. 2a) cDNA-Sequenz von DNPI, human; AN: AB032435
Fig. 2b) Aminosäure-Sequenz von DNPI, human; AN: AB032435
Fig. 2c) cDNA-Sequenz von DNPI, Ratte; AN: AF271235
Fig. 2d) Aminosäure-Sequenz von DNPI, Ratte; AN: AF271235
Fig. 2e) cDNA-Sequenz von DNPI, Maus; AN: NM_080853
Fig. 2f) Aminosäure-Sequenz von DNPI, Maus;AN: NM_080853
Fig. 3) Differentielle Expression von DNPI und BNPI in Synapsen und motorischen Arealen des lumbalen Rückenmarks der Ratte (s. Beispiel 2a)
Fig. 4) Differentielle Expression von DNPI und BNPI in Synapsen der Dorsalhorn-Arealen des lumbaren Rückenmarks der Ratte (s. Beispiel 2b)
Fig. 5) Differentielle Expression von DNPI und BNPI in Synapsen des sakralen Rückenmarks der Ratte (s. Beispiel 2c)
Fig. 6) Differentielle Expression von DNPI und BNPI in Synapsen der medullo-cervicospinalen Leitung des Trigeminalnervs der Ratte (s. Beispiel 2d)
Fig. 7) Differentielle Expression von DNPI und BNPI in Hinregionen der Ratte (s. Beispiel 2e)
Fig. 8) Differentielle Expression von DNPI und BNPI in Hinregionen der Ratte (s. Beispiel 2f)
Fig. 9) Differentielle Expression von DNPI und BNPI in Hinregionen der Ratte (s. Beispiel 2g)
Fig. 10) Differentielle Expression von DNPI und BNPI in Hinregionen der Ratte (s. Beispiel 2h)
Fig. 11) Differentielle Expression von DNPI und BNPI in Hinregionen der Ratte (s. Beispiel 2i)
Fig. 12) Differentielle Expression von DNPI und BNPI in Hinregionen der Ratte (s. Beispiel 2j)
Fig. 13) Differentielle Expression von DNPI und BNPI in Hinregionen der Ratte (s. Beispiel 2k)
Fig. 14) Differentielle Expression von DNPI und BNPI in Hinregionen der Ratte (s. Beispiel 2l)
Fig. 15) Differentielle Expression von DNPI und BNPI in Hinregionen der Ratte (s. Beispiel 2m)
Fig. 16) Differentielle Expression von DNPI und BNPI in Hinregionen der Ratte (s. Beispiel 2n)
Fig. 17) Differentielle Expression von DNPI und BNPI in Hinregionen der Ratte (s. Beispiel 2o)
Fig. 18) Differentielle Expression von DNPI und BNPI in Hinregionen der Ratte (s. Beispiel 2p)
Fig. 19) Differentielle Expression von DNPI und BNPI in Hinregionen der Ratte (s. Beispiel 2q)
Fig. 20) Differentielle Expression von DNPI und BNPI in Hinregionen der Ratte, dabei heißt AS Anti-sense und bezieht sich auf die Färbung.

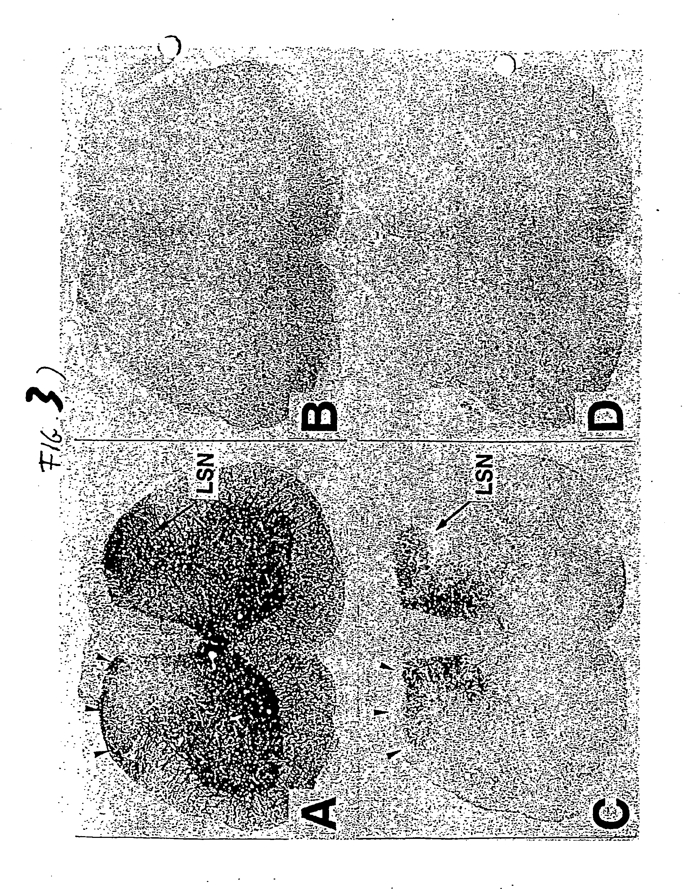

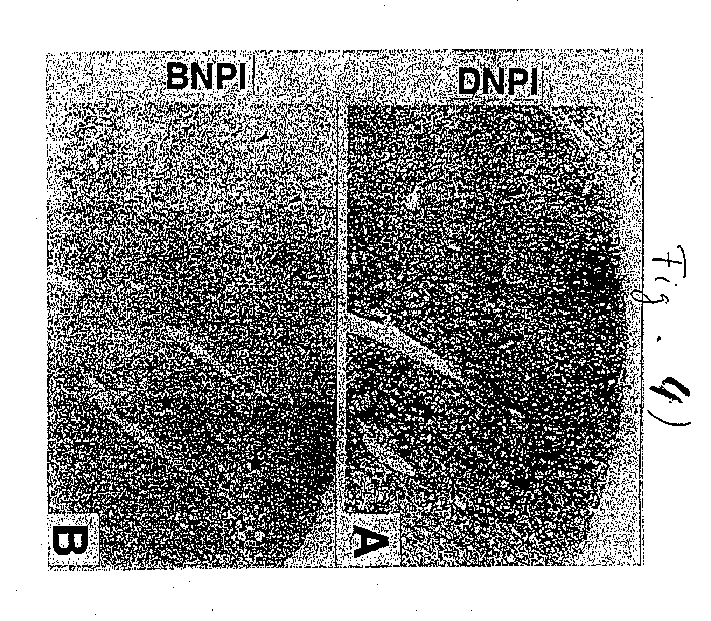

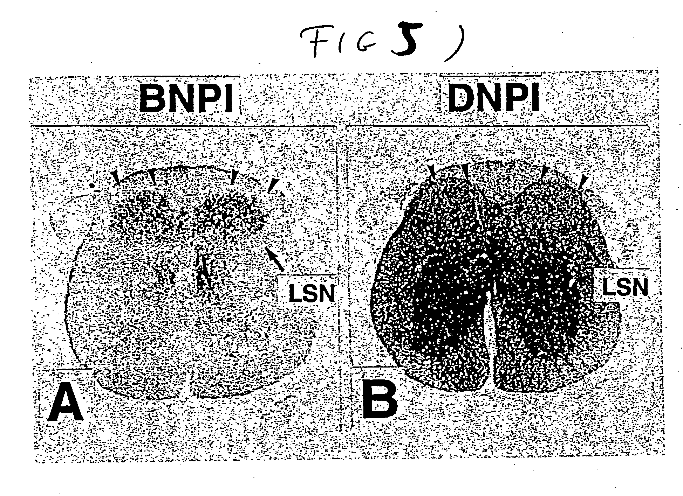

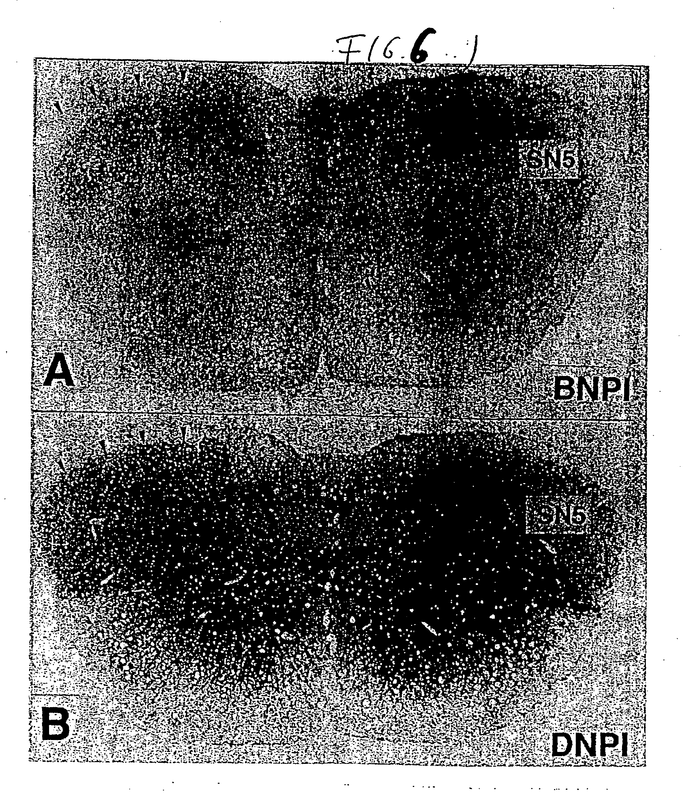

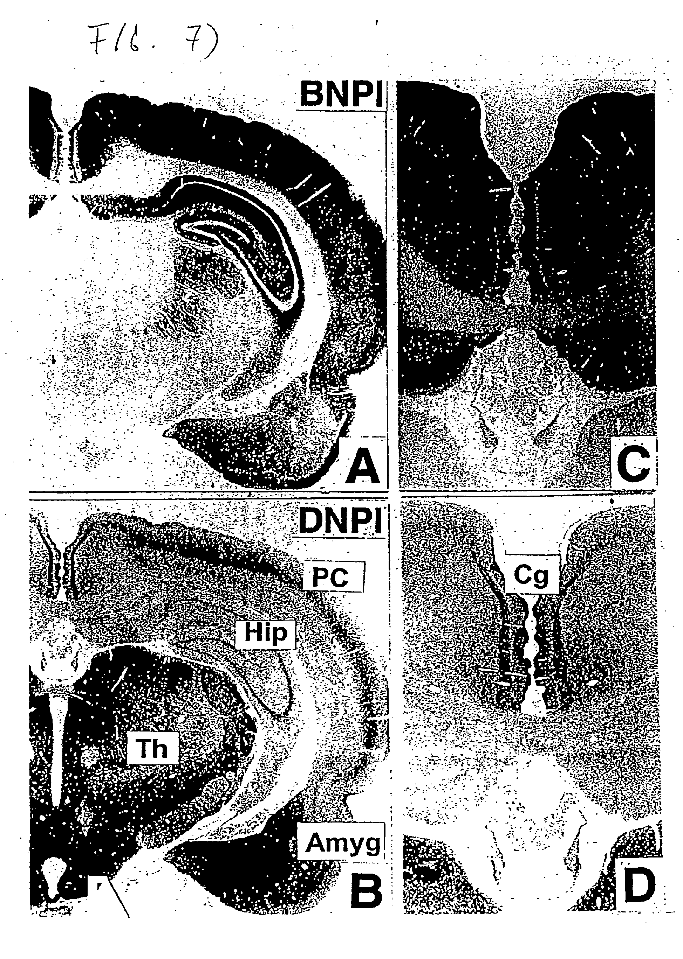

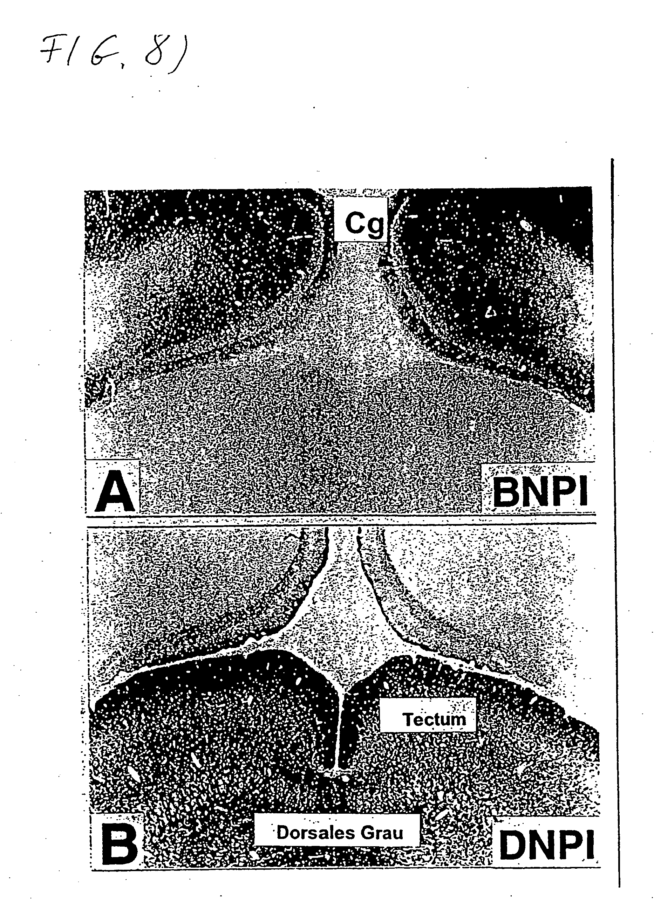

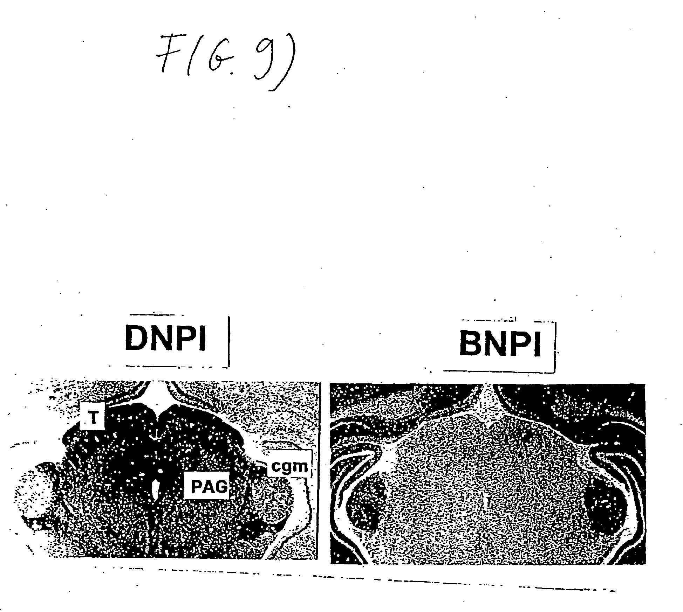

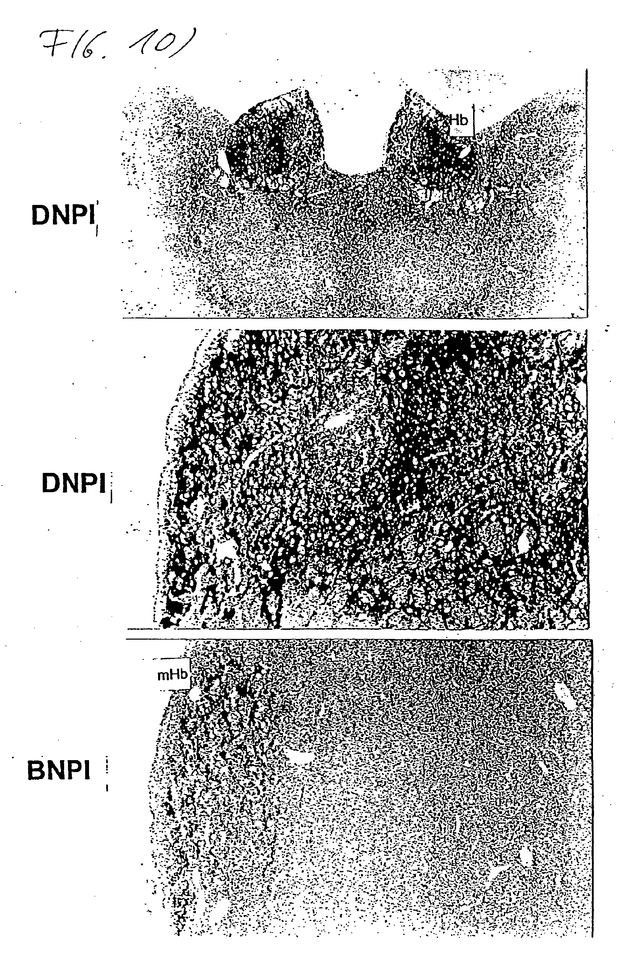

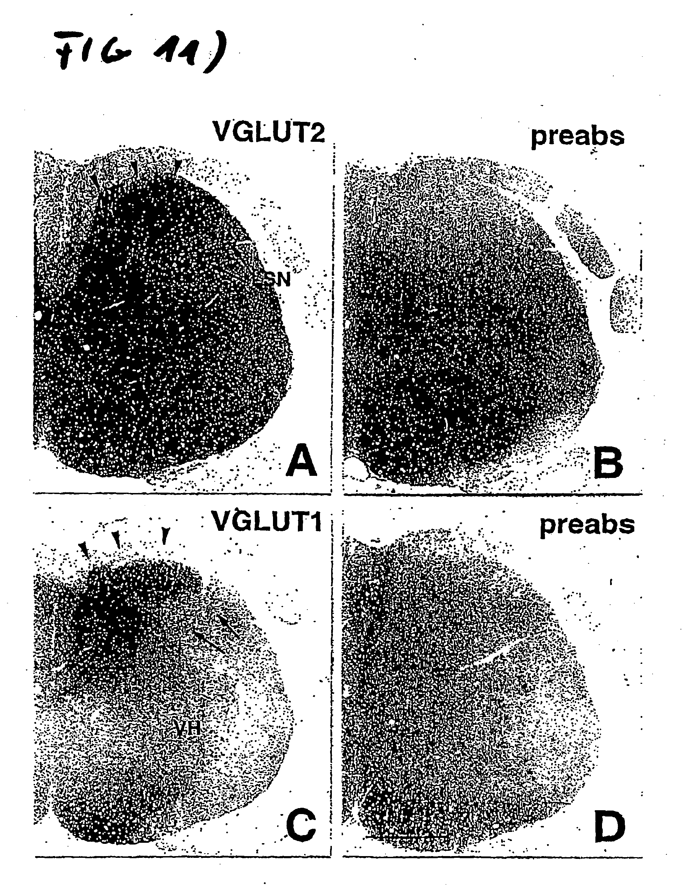

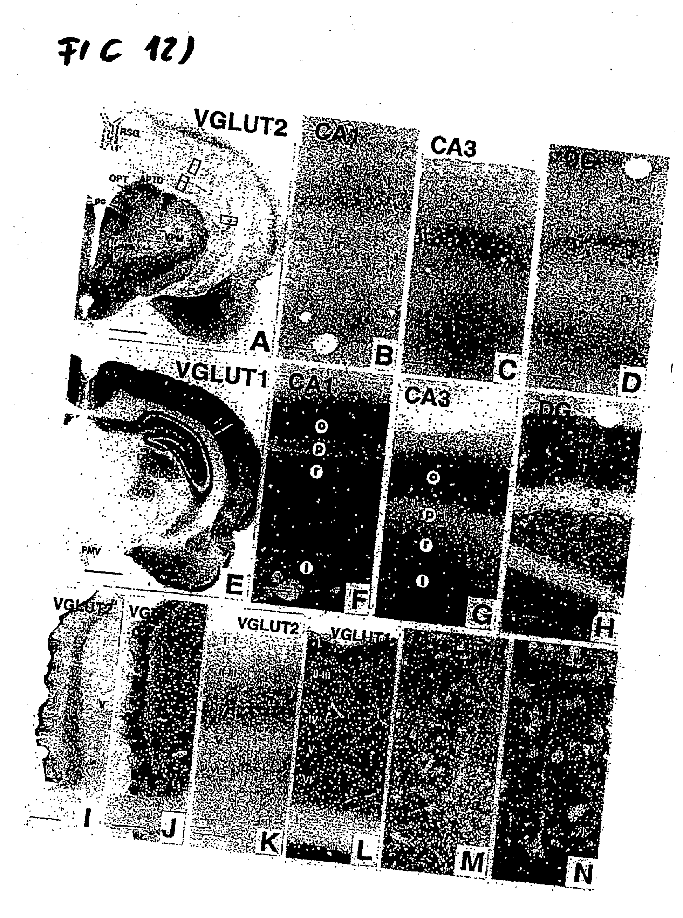

Fig. 21) Differentielle Expression von DNPI und BNPI in Hinregionen der Ratte, dabei heißt AS Anti-sense und bezieht sich auf die Färbung.

### Beispiele:

### Beispiel 1

### Differentielle Betrachtung der Expression zwischen DNPI und BNPI über immuncytochemische Färbung

Zur immunhistochemischen Anfärbung wurden polyklonale Kanichen-Antiseren gegen das rekombinante DNPI- bzw. BNPI-Fusionsprotein verwendet. Generell wurden Schnitte verschiedener Regionen des ZNS angelegt und die Expression von DNPI mit der von BNPI verglichen. Das Vorgehen entsprach bezüglich der Schnitte und der Färbung dem bei Persson S., Schäfer MK-H., Nohr D., Ekström G., Post C., Nyberg F. und Weihe E. (1994), Neuroscience 63; 313-326 bzw. Nohr D., Schäfer MK-H., Romeo H., Persson S., Nyberg F. Post C. und Weihe E. (1999), Neuroscience 93; 759-773 beschriebenen Verfahren, wobei die Offenbarung, Beschreibung bzw. technische Lehre dieser Artikel ausdrücklich zum Teil der hier vorgelegten Offenbarung, Beschreibung bzw. technischen Lehre dieser Erfindung gemacht wird.

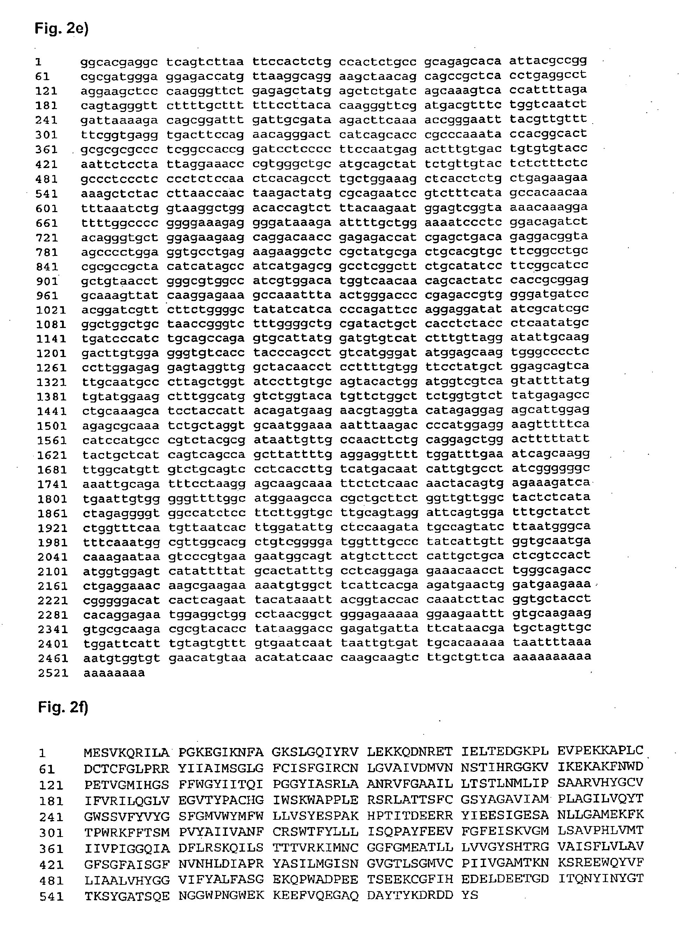

### Beispiel 2a zu Abbildung 3)

Es ist die differentielle Verteilung der Immunreaktivität von BNPI und DNPI im lumbaren Rückenmark der Ratte zu sehen. Die angrenzenden deparafinierten Abschnitte A- bis D sind wie folgt gefärbt:
A = anti-DNPI;
B = anti-DNPI präadsorbiert mit DNPI-Fusionsprotein;
C = anti-BNPI;
D = anti-BNPI präadsorbiert mit BNPI-Fusionsprotein;

Die DNPI- (A) und BNPI- (C) Immunfarbstoffe waren voll mit homologem rekombinanten BNPI- (D) und BNPI- (B) Fusionsprotein präadsorbierbar, was die Spezifität der Immunreaktion beweist.

Bemerkenswert ist das gegenseitig ausschließende Verteilungsmuster von DNPI und BNPI-Immunfärbung im äußeren und tiefen Dorsalhorn. (A;C).Punktierte Immunfärbung von DNPI ist in den synaptischen Emndungen des äußeren Dorsalhorns (Lamina 1 und Substantia gelatinosaa) (Pfeil in A), während BNPI Immunreaktivität vollständig fehlt (Pfeile in B). Akkumulation von starker positiver punktierter BNPI-Immunfärbung liegt im tieferen Dorsalhorn vor, während DNPI-Färbung relativ niedrig ist. DNPI ist präsent in der lateralen spinalen Nukleus (LSN in A), während BNPI völlig fehlt (LSN in C). DNPI ist in der Lamina X um den zentralen Kanal abundant, während BNPI selten ist. BNPI Immunfärbung ist im lateralen Ventralhorn schwach und gering oder fehlend im medialen Ventralhorn. Durch das ganze Ventralhorn ist punktförmige DNPI-Färbung abundant, etwas weniger im lateralen Horn im Vergleich zum medialen Ventralhorn. Es gibt eine schwache BNPI und DNPI Färbung in einigen Zellkörpern der im Ventralhorn gelegenen Motoneurone, was aber nicht durch die homologen transporter Fusionsproteine präadsorbiert wurde und daher als nichtspezifisch eingestuft wurde.

### Beispiel 2b zu Abbildung 4)

Es ist die differentielle Verteilung der Immunreaktivität von BNPI und DNPI im linken lateralen oberflächlichen dorsalen lumbaren Rückenmark (left lateral superficial dorsal lumbar spinal cord) der Ratte zu sehen. A und B jeweils für BNPI (A) und DNPI (B) gefärbt zeigen viele punktförmige Anfärbungen für DNPI, die in der Lamina I und substantia gelatinosa konzentriert sind wo BNPI fast vollständig fehlt. Weiter sind dichte Komplexe von DNPI positiven Punkten im lateralen spinalen Nukleus zu sehen, wo BNPI fast vollständig fehlt. Feine DNPI positive Punkte sind auch in den tieferen dorsalen Horn zu finden, wenn auch mit geringerer Dichte.

### Beispiel 2c zu Abbildung 5)

Es ist die differentielle Verteilung der Immunreaktivität von BNPI und DNPI im sakralen Rückenmark der Ratte zu sehen. Die angrenzenden Abschnitte A und B jeweils für BNPI (A) und DNPI (B) gefärbt, zeigen gegenseitige Exclusions-Zonen punktierter DNPI- und BNPI-Immunfärbung im Dorsalhorn. DNPI ist in der gesamten Grey Matter präsent und ist in den sehr äußeren Schichten des Dorsalhorns konzentriert, wo es es eine schmale Bande an der Grenze zu White Matter bildet. DNPI ist abundant im lateralen spinalen Nukleus und in der Lamina X wie auch in der Lamina V/VI und im ganzen ventralen Horn. BNPI ist abundant im tiefen Dorsalhorn und selten in Ventralhorn.

### Beispiel 2d zu Abbildung 6)

Es ist die differentielle Verteilung der Immunreaktivität von BNPI und DNPI in der unteren Medulla oblongate am Übergang zum cervicalen Rückenmark zu sehen. Die angrenzenden Abschnitte A und B jeweils für BNPI (A) und DNPI (B) gefärbt zeigen eine bevorzugte Akkumulation der BNPI-Färbung im medialen Teil des spinalen trigeminalen Nukleus und in dem mittleren und unteren Teil der dorsalen Medulla, es ist nur eine sehr schwache Färbung mit BNPI in den ventralen Medulla zu sehen. DNPI ist abundant in Grey Matter der Medulla. DNPI-Färbung überlappt mit der BNPI-Färbung im inneren spinalen Nucleus V. Es ist zu beachten, daß BNPI auch im oberen spinalen trigeminalen Nukleus, der gleich der spinalen substantia gelatinosa ist, zu finden ist. DNPI-Färbung ist in Gebieten schwächer, in denen BNPI präsent ist, schwächer als in Gebieten, wo BNPI niedrig ist oder fehlt. Einige wenige BNPI Punkte sind im ventralen Grey Motor Gebiet zu sehen.

### Beispiel 2e zu Abbildung 7)

Komplementär differentielle Verteilung von DNPI und BNPI Imunreaktivität in 2 Folgeschnitten des Rattenhirns in schmerzrelevanten Hirnregionen wie sensorischer parietaler Cortex; cingulärer Cortex, Thalamus, Corpus amygdaloideum sowie auch Hypothalamus. DNPI ist im Cortex in den granulären sensorischen Schichten insbesondere in Lamina IV konzentriert; BNPI ist im Cortex abundant aber schwächer in der Lamina IV als in anderen Laminae. Im cingulären Cortex (C vs D als Hochvergrößerung) ist die Verteilung von DNPI und BNPI komplementär wechselseitig excludierend bzw. reziprok in der Dichte der jeweiligen Synapsen. DNPI überwiegt im Thalamus eindeutig über BNPI, BNPI ist im Hypothalamus spärlich, DNPI abundant. Abundantes BNPI überwirgt im Hypocampus über spärliches DNPI bei wechselseitig komplementärer Verteilung.
- Thalamus: = Th,
- Amygdala: = Amyg.
- Hippocampus: = Hip,
- Cinguläerer Cortex: = Cg,
- Hypothalamus: = Hy,
- parietaler Cortex: = PC.

### Beispiel 2f zu Abbildung 8)

Komplementär differentielle Verteilung von DNPI und BNPI Immunreaktivität in schmerzrelevanten Hirnregionen wie cingulärer Cortex (Cg) und Tectum sowie dorsalem periaquäductalen Grau. DNPI Dominanz im Tectum und dorsalem Grau. Folgeschnitte eines Rattenhirns durch das obere Mesencephalon.

### Beispiel 2g zu Abbildung 9)

Komplementär differentielle Verteilung von DNPI und BNPI Immunreaktivität in schmerzrelevanten Hirnregionen wie Tectum (T) sowie periaquäductalen Grau (PAG). DNPI Dominanz im Tectum und dorsalem Grau. Man notiere differentielle Verteilung von DNPI und BNPI im corpus geniculatum mediale (cgm) der Hörbahn. Folgeschnitte eines Rattenhirns durch das obere Mesencephalon; Ebene colliculus superior.

### Beispiel 2h zu Abbildung 10)

Abundanz von DNPI über BNPI in den Habenulae (Hb). DNPI ist präsent im gesamten Habenularkomplex (niedrige Vergrößerung, obere Abbildung; hohe Vergrößerung, mittlere Abb.). BNPI ist nur im medialen Habenularkem (mHb untere Abb., Folgeschnitt zu mittlerer Abbildung).

In den folgenden Beispielen 2i bis 2q und den zugehörigen Abbildungen 11- 19 wird der Begriff VGLUT1 für BNPI und VGLUT2 für DNPI verwendet. Die Begriffe sind jeweils vollkommen synonym, inhaltlich völlig gleich und betreffen den gleichen Gegenstand, also VGLUT1 = BNPI und VGLUT2 = DNPI. "preabs" bedeutet die Präabsorption mit VGLUT1-oder VGLUT2-Fusionsprotein vor der Immunfärbung.

### Beispiel 2i zu Abbildung 11)

Dies ist ein Vergleich zwischen VGLUT1 und VGLUT2 bezüglich der Verteilung und der Spezifität im lumbaren Rückenmark über die Immunoreaktivität.

Die angrenzenden Abschnitte A-D sind abwechselnd mit anti-VGLUT2 (A), anti-VGLUT2, präabsorbiert mit VGLUT2-Fusionsprotein (B), anti-VGLUT1 (C) und anti-VGLUT1, präabsorbiert mit VGLUT1-Fusionsprotein (D) gefärbt. Die Immunoreaktionen in (A) und (C) werden vollständig mit homologem rekombinanten Fusionsprotein präabsorbiert (B) und (D), was die Spezifität der Immunreaktion zeigt. Zu beachten ist das differentielle Verteilungsmuster im oberflächlichen und tiefen Dorsalhörn (A;C). Punktförmige Immunfärbung für VGLUT2 ist im oberflächlichen Dorsalhorn zu erkennen (Pfeile kennzeichnen in A Lamina 1 und Substantia gelatinosa), wo die Immunreaktivität mit VGLUT1 minimal ist (Pfeile in C). Zu beachten ist weiter die Akkumulation stark positiver punktförmiger VGLUT1 im tiefen Dorsalhorn, wo VGLUT2 relativ schwach ist. VGLUT2 ist im lateralen spinalen Nukleus vorhanden (LSN; Pfeile in A), wo VGLUT1 nur gering vertreten ist (Pfeile in C).VGLUT2 ist stark in Lamina X um den zentralen Kanal herum vertreten, wo VGLUT1 selten ist. Die Immunfärbung von VGLUT1. ist schwach bis mittelmäßig i lateralen ventralen Horn und sehr dünn im medialen ventralen Horn. Eine feine punktförmige VGLUT2-Anfärbung ist dicht und reichlich im Ventralhorn (VH).

### Beispiel 2j zu Abbildung 12)

Dies ist ein Vergleich zwischen VGLUT1 und VGLUT2 bezüglich der Verteilung im Vorderhirn (1).

Paare von Low-Power- und High-Power-Micrographen von zwei angrenzenden Frontalsektionen sind alternativ für VGLUT2 (A-D, I, K, M) und VGLUT1 (E-H, J, L, N) gefärbt. Dies zeigt die deutliche Differenz und die teilweise gegenseitige Auschließlichkeit in der Verteilung, Dichte und Intensität von VGLUT1 und VGLUT2 Immunoreaktivität (ir) in ausgewählten corticalen, hippocampalen, diencephalischen und limbischen Bereichen.

### Hypothalamus und Thalamus (A,E):

Punktförmige VGLUT2-ir is relativ stark über den ganzen Hypothalamus und Thalamus, wo VGLUT-2 eine beschränkte Verteilung auf den hypothalamischen, ventralen premammillaren Nukleus (PMV) und auf Teile des thalamischen Nukleus inclusive des laterialen hinteren thalamischen Nukleus (LP), dem dorsalen lateralen geniculaten Nukleus (DLG) und dem ventralen posteromedialen thalamischen Nukleus (VPM) zeigt. Olivarer prtectaler Nukleus (OPT); dorsaler vorderer pretectaler Nukleus (APTD); precommisuraler Nukleus (PrC).

### Cortex (A; E; I; J; K; L; M; N):

Die VGLUT2-Färbung ist in einem Band des Neocortex enthaltend Lamina IV moderat. Sie ist schwach in einem neocorticalem Band enthalten Lamina VI und minimal in den anderen neocorticalen Schichten. Intensive punktförmige VGLUT1-ir ist sehr stark im gesamten Cortex, inclusive des pririform Cortex (Pir) und etwas schwächer ausgeprägt im neocortikalen Band der Lamina VI, wo moderate VCGLUT2-Färbung akkumuliert. Zu beachten ist der gegenseitige Ausschluß von VGLUT1- und VGLUT2-Färbung in den Schichten des retrospenialen granularen Cortex (RSG in A und E), die in starker Vergrößerung in (I) und (J) gezeigt werden. Die großen Vergrößerungen M und N aus der Lamina IV in K und L zeigen verschiedene Dichten der punktförmigen VGLUT1- und VGLUT2-ir im Vergleich zwischen immunonegativen neuronalen Zellkörpern und -Prozessen.

### Hippocampus (A, E; B-D, F-H):

Dünne VGLUT2-ir-Punkte sind meist beschränkt auf die granularen Schichten (g) des dentaten Gyrus (DG) und der pyramidalen Schicht (p) der Felder CA1, CA2 und CA3 des Hippocampus. Dichte V-GLUT1-ir-Punkte sind sehr stark über den gesamten Hippocampus vertreten mit Ausnahme der granularen (g) und pyramidalen (p) Zell-Schichten. Mit Rechtecken gekennzeichnete Abschnitte in A und entsprechende Abschnitte auf angrenzenden Sektionen in E werden mit großer vergrößerung jeweils in B-D und F-H gezeigt, zu beachten sind die differentielle Verteilung und Dichte der VGLUT1-ir und VGLUT2-ir in der Oriens-Schicht (o), pyramidalen Schicht (p), im Stratum radiatum (r) und dem Stratum lacunosum molekulare (I) von CA1 (B,F) und CA3 (C, G) und in der molekularen (m), granularen (g) und polymorphen (p) Schicht des dentaten Gyrus (DG) (D,H).

### Amygdala Complex:

Hier ist eine gewisse Überlappung festzustellen, aber die differentielle Dichte und Intensität der Immunfärbung von VGLUT1-ir und VGLUT2-ir Punkten im hinteren basomedialen amygdaloiden Nukleus (BMP), im lateralen amygdaloiden Nukleus (La) und im cortikalen amygdaloiden Nukleus (Co.) wie auch im angrenzenden dorsalen endopiriformen Nukleus (DEn) ist deutlich zu sehen.

Es ist weiter festzustellen, daß "White Matter" und Faser Trakte VGLUT1- und VGLUT2-negativ sind (hintere commisure (pc), fomix (f), fasciculus retroflexus (fr), mammillothalamischer Trakt (mt).

### Beispiel 2k zu Abbildung 13)

Dies ist ein Vergleich zwischen VGLUT1 und VGLUT2 bezüglich der Verteilung im Vorderhirn (2).
Paare von Low-Power- und High-Power-Micrographen von zwei angrenzenden Frontalsektionen sind alternativ für VGLUT2 (A-B, E, G) und VGLUT1 (C-D, F, H) gefärbt. Dies zeigt die deutliche Differenz und die teilweise gegenseitige Auschließlichkeit in der Verteilung, Dichte und Intensität von VGLUT1- und VGLUT2-Immunoreaktivität (ir) im Neocortex (Lamina IV, VI) Caudate Putamen (CPu), Globulus pallidum (GP), piriform cortex (Pir), Nukleus accumbens Kern (AcC), Nukleus accumbens Rand (AcSh), ventralem Pallidum (VP), olfaktorischem Tuberkel (Tu), Inseln von Calleja (ICj), dem ventralen diagonalen Band (VDB) und dem lateralen Septum (LS). Im CPu is VGLUT1 etwas schwächer vertreten als VGLUT2. VGLUT2 ist im Globus pallidum (GP) vorhanden (B), wo VGLUT1 fast vollständig fehlt (D). Im piriformen Cortex (Pir) und den Inseln von Calleja (ICj) ist die punktförmige VGLUT1-ir stärker und dichter als für VGLUT2-ir. Zu beachten ist die Akkumulation schwacher bis moderater VGLUT1-ir in den pyramidalen Zellschichten in (E), wo VGLUT2-ir fast völlig fehlt (F). Zu beachten ist auch eine gewisse Überlappung und Reziprozität in der Färbung von VGLUT1 und VGLUT2 in den ICj (G,H) wie auch die Abwesenheit von VGLUT1-ir und VGLUT2-ir im commissuralem FaserTrakten /Corpus callosum (cc(, vordere commissure (ac).
Balken in A, C = 1mm, in B, D = 500 µm; in E-H = 200 µm.

### Beispiel 21 zu Abbildung 14)

Dies ist ein Vergleich zwischen VGLUT1 und VGLUT2 bezüglich der Verteilung im thalamischen und hypothalamischen Nucleus.
Angrenzende frontale Sektionen (A,B) des Diencephalons zeigen die nukleus-spezifische differentielle starke Vorkommen von VGLUT2 (A) und VGLUT1 (B) im Thalamus und Hypothalamus. Zu beachten ist das sehr starke Vorkommen von VGLUT2-ir (A) im paraventrikulären thalamischen Nukleus (PVA), reunienten thalamischen Nukleus (Re), reticularen thalamischen Nukleus (Rt), paracentralem thalamischen Nukleus (PC) und anterodorsalem thalamischen Nukleus (AD). Hier fehlt VGLUT1-ir (B) fast vollständig oder kommt nur in niedriger Konzentration vor. VGLUT1 (B) kommt moderat im hinteren thalamischen Nukleus (PT) vor, wo VGLUT2 (A) selten ist. VGLUT1 ist fehlt fast völlig in der stria medullaris (sm), wo VGLUT2-ir selten ist.
Angrenzende frontale Abschnitte C, D des Diencephalons zeigen die Häufigkeit von VGLUT2 (C) im vorderen hypothalamischen Nukleus (AH) aber die Seltenheit im paraventrikulären Nukleus (PVN) und extreme Seltenheit von VGLUT1-ir im vorderen hypothalamischen Nukleus (AH) und völliges Fehlen im PVN. Zu beachten ist auch die Gegenwart von VGLUT1 (D) im Gegensatz zur Abwesenheit von CGLUT2 (C) im ventromedialen thalamischen Nukleus (VM) und dem Reuniens thalamischen Nukleus (Re).
Angrenzende frontale Sektionen des Hypothalamus (E,F) zeigen die Häufigkeit von VGLUT2 im LH, im ventromedialen thalamischen Nukleus (VHM) und dem dorsomedialen thalamischen Nukleus (DM) und die Seltenheit von VGLUT1 im Kern des VMH aber moderates Vorkommen in seinem Rand. Zu beachten ist die schwache Färbung von VGLUT2 in der medianen Emminenz (ME). VGLUT1 und VGLUT2 fehlen in den Fasertrakten des Formix (f) und des mammillothalamischen Traktes (mt). Dritter ventrikel (3V); Balken = 500 µm (für A-F).

### Beispiel 2m zu Abbildung 15)

Dies ist ein Vergleich zwischen VGLUT1 und VGLUT2 bezüglich der Verteilung im Epithalamus.
High-Power-Micrographen von angrenzenden Sektionen, die alternativ für VGLUT2 (A) und VGLUT1 (B) gefärbt sind, zeigen den Überfluß an VGLUT2 in sowohl dem medialen habenularen Nukleus (MHb) und dem lateralen habenularen Nukleus (LHb). Es ist zu beachten, daß VGLUT1-ir im MHb weniger dicht ist als VGLUT2-ir. CGLUT1 fehlt fast vollständig im LHb.
Balken = 100 µm (für A,B).

### Beispiel 2n zu Abbildung 16)

Dies ist ein Vergleich zwischen VGLUT1, Tyrosin-Hydroxylase und VGLUT2 bezüglich der Verteilung im Mesencephalon und Metathalamus.
Low-Power- (A,C,E) und High-Power-Micrographen (B,D,F) von drei angrenzenden Sektionen sind alternierend für Tyrosin-Hydroxylase (TH), VGLUT2 und VGLUT1 gefärbt und zeigen die differentielle Verteilung, Dichte und Intensität der Immunfärbung für VGLUT1 und VGLUT2 im Vergleich zu TH. VGLUT2-ir Punkte sind im Tectum konzentriert, wobei die höchsten Mengen in der superficialen "Grey"-Schicht des oberen Colliculus (SuG) und geringere Mengen in der intermediären "Grey"-Schicht des oberen Colliculus (InG) zu finden sind, während diese in der optischen Nukleus-Schicht des oberen Colliculus (Op) selten sind. VGLUT2-ir Punkte liegen im gesamten Tegmentum inclusive des Nukleus ruber (R) und der TH-positiven pars compacta der substantia nigra (SNC) vor und sind im dorsalen periaquäductalem Grey (PAG) besonders angereichert und insbesondere im medialen terminalen Nukleus des "accessory optic tract" (MT), des optischen Zugangstrakts, wie auch im mediocaudalen Teil des lateralen hinteren Nukleus (LPMC), im hinteren intralaminaren thalamischen Nukleus (PIL) im peripeduncularen Nukleus (PP) und im suprageniculaten thalamischen Nukleus (SG). VGKUT1-ir ist hier minimal. VGLUT 1-Färbung zeigt sich in moderaten Mengen im ventralen medialen geniculaten Nukleus (MGV), wo VGLUT2-Mengen minimal sind. VGLUT1 ist im gesamten Tectum, dem periaquaeductalem Grey und dem tegmetum nur minimal vorhanden und fehlt fast vollständig in der substantia nigra pars compacta (SNC) und der pars reticulais (SNR) Schwache VGLUT2-Färbung ist in den neuronalen Perikarya und Puncta in der SNR vorhanden (D, high-powered Micrograph aus der durch ein Rechteck gekennzeichneten in (C), wo VGLUT1 fast vollständig fehlt (korrespondierend in F)). Mesencephalischer Aquaedukt (Aq.)
Balken in A, C, E =1mm; in B, D, F = 200 µm.

### Beispiel 2o zu Abbildung 17)

Dies ist ein Vergleich zwischen VGLUT1 und VGLUT2 bezüglich der Verteilung im pontomedullären Hirnstamm.
Low-Power- und High-Power-Micrographen von zwei angrenzenden Frontalsektionen sind alternierend für VGLUT2 (A-D) und VGLUT1 (E-H) gefärbt. Dies zeigt eine starke Menge punktförmiger VGLUT2-Immunoreaktivität (ir) (B, D) in der medialen oberen Olive (MSO), wo VGLUT1-ir niedrig ist (F, H). VGLUT2-ir ist relativ schwach im Nukleus des trapezoiden Körpers (TZ) (B-C) ausgeprägt, wo VGLUT1-ir in großer Menge vorliegt (F-G). Es ist zu beachten, daß deutlich VGLUT1-positive confluente große Punkte immunonegative neuronale Zellkörper im TZ (G) umschließen. Starke VGLUT1-ir-Punkte sind in den zentralen sensorischen Nukleus des trigeminal Nervs (Pr5), wo VGLUT2-ir sehr niedrig ist. Moderat positive VGLUT1 Punkte sind in dem motorischen trigeminalen Nukleus (Mo5) vorhanden, wo VGLUT2-ir niedrig ist. VGLUT1-ir und VGLUT2-ir sind mit gerringer Menge im lateralen medialen parabrachialen Nukleus vertreten (LPB, MPB). Moderate VGLUT2-ir akkumuliert im locus coerulus (LC), wo VGLUT1-ir sehr gering ist. VGLUT1-ir und VGLUT2-ir fehlen im pyramidalen Trakt (pyr). Angrenzende Sektoren (I,J) alternierend für VGLUT2 (I) und VGLUT1 (J) gefärbt, zeigen eine starke Menge punktförmiger VGLUT1-Immunoreaktivität (ir) im vorderen ventralen cochlearen Nukleus (VCA), wo VGLUT2 im Prinzip völlig fehlt. Ein High-Power-Micrograph (K) von J zeigt stark positive VGLUT-ir Punkte, die immunonegative neuronale Zellkörper und Prozesse einschliessen. VGLUT1-ir Punkte sind in größerer Zahl im dorsalen cochlearen Nukleus (DC) als VGLUT2-ir-positive Punkte.
Balken in A, E = 500 µm; in B, F, I, J = 200 µm; in C, D, G, H, K = 25 µm.

### Beispiel 2p zu Abbildung 18)

Dies ist ein Vergleich zwischen VGLUT1 und VGLUT2 bezüglich der Verteilung im unteren Hirnstamm.

Low-Power- und High-Power-Micrographen von zwei angrenzenden Frontalsektionen sind alternierend für VGLUT2 (A, C, E, G) und VGLUT1 (B, D, F, H) gefärbt, zeigen eine moderate Menge kleiner punktförmiger VGLUT2-Immunoreaktivität (ir) im oberflächlichen spinalen trigeminalen Nukleus (Sp5), was durch Pfeile markiert ist (A, G), wo VGLUT1-ir im Prinzip völlig fehlt (Pfeile in B, H). VGLUT2 ist im doorsalen motorischen Nukleus des Vagus (10), hypoglossalen Nukleus (12), der retikularen Formation (Rt) und im ventralen Teil des solitären Traktes (SolV) (A,C,E,) moderat vorhanden, wo VGLUT1 im Prinzip völlig fehlt (B, D, F). VGLUT2-ir ist im dorsalen solitären Trakt (SolD) sehr niedrig. Zu beachten ist das Übergewicht VGLUT1 im tiefen Sp5 (B, F, H), im Cuneat (Cu) und dem grazilen Nukleus (GR), wo VGLUT2-ir niedrig ist. Steme markieren den zentralen Kanal.
Balken in A, B = 500 µm; C,D = 200 µm, E,F = 100 µm; G,H = 100 µm.

### Beispiel 2q zu Abbildung 19)

Dies ist ein Vergleich zwischen VGLUT1 und VGLUT2 bezüglich der Verteilung im Cerebellum.
Low-Power- und High-Power-Micrographen von zwei angrenzenden Frontatsektionen, alternierend für VGLUT2 (A, B) und VGLUT1 (C, D) gefärbt, zeigen eine extreme Dichte intensiv gefärbter VGLUT1-positiver Punkte in der molekularen Schicht (m), sehr wenige VGLUT1 Punkte um Somata der Purkinje Zelle in der Purkinje Zellschicht (p) und dichte glomeruli-ähnliche Akkumulation stark gefärbter konfluenter VGLUT1 Punkte in der granularen Schicht (g). VGLUT2-ir Punkte sind viel weniger dicht in der molekularen Schicht, wo Sie in einer bandförmigen Art angeordnet sind. VGLUT2-ir Punkte, die glomerula-ähnliche Strukturen in der glomerularen Schicht (g) bilden, sind weniger dicht als die, die füpr VGLUT1 färben.

Balken in A, C = 500 µm; in B,D = 100 µm.

### Diskussion und Analyse zu Beispiel 2 allgemein:

Die differentielle Verteilung von BNPI und DNPI in Synapsen des primärafferenten, spinalen trigeminalen und supraspinalen Systems ist eine starke Evidenz für eine selektive Beeinflußbarkeit sensorischer Funktionen durch selektive Modulation des DNPI bzw. BNPI-vermittelten Glutamat-Transports.

Die Präsenz von BNPI und DNP im DRG weist auf die Möglichkeit hin, periphere neurogene Entzündungen selektiv durch selektive Intervention am DNPI oder BNPI-Target zu beeinflussen. Die Präsenz im DRG indiziert auch eine immunmodulatorische Rolle und entsprechende Targetierung. Eine Präsenz von BNPI und DNPI im sensorischen Vagus oder Glossopharyngeus Ganglion indiziert das Target für Baroafferenz, Chemoafferenz, cardiovaskuläre oder cardiorespiratorische Funktion, inklusive Asthma, Hypertonie etc., wie auch für Emesis. Interessant ist die Verteilung auch für die Darm-Gehirn-Achse, also Regulation der Sättigung, der "Inflammatory bowel disease" oder Morbus Crohn ebenso wie für Autoimmunität im zentralen odere peripheren Nervensystem, autoimmuner Diabetes, alkoholischer Neuropathie, Alkohol induzierter chronischer Pankreatitis mit Neuroproliferation (Fink et al. mit Weihe; Neuroscience). Alleine die Verteilung im ZNS und PNS macht diese Targets zu interessanten Objekten in den restlichen bereits oben genannten Indikationen.

Ein entscheidender Punkt war aber auch, daß BNPI und DNPI in den afferenten Bereichen zu den sensorischen Bereichen des Auges und des Ohres nachgewiesen werden konnten, was in Kombination mit den anderen Erkenntnissen eine wichtige Rolle bei Sehstörungen, Retinitis pigmentosa, Opticus Degeneration, Katarakt, Netzhautablösung, Retinadegeneration, Glaukom oder Nystagmus oder Hörstörungen, Tinitus, M. Menière, Hörsturz, Erkrankungen des Hör- und/oder Gleichgewichtsorgans oder Erkrankungen der Hörbahn oder Vesibularbahn nahelegt.

Entscheidend ist weiter, daß die Verteilung von VGLUT2 (=DNPI) in den meisten Regionen des Gehirns und des Rückenmarks komplementär und gegenseitig ausschließend zur Expression von VGLUT1 (=BNPI) ist. Zusammen könnten die beiden Glutamat-Transporter für die Aufnahme von Glutamat durch synaptische Vesikel aller centralen glutamatergen Neuronen verantwortlich sein.

Es wurde hier gefunden, daß die thalamischen und Gehirnstamm-Relais-Zentren des visuellen und statoakkustischen Pfades durch differentielle VGLUT1- und VGLUT2- gesteuerte Signale getrieben werden. Thalamische und mesenephalische Relay-Zentren des visuellen Systems wie der colliculus superior und der dorsolaterale geniculate Nukleus und der mediale terminale Nukleus des "Accessory optic tracts", des zugehene optischen Sytems sind spezifisch VGLUT2-gesteuert, was nahelegt, daß die retinalen ganglionischen Zellen, die das dritte Neuron des optischen Sinnes repräsentieren, zumindest teilweise mit VGLUT2 beschichtet sind. Im Gegensatz dazu erhalten der Hirnstamm cochlear, olivary trapezoid und das metathalamische mediale geniculate Relay-Zenter des Gehör-Pfades starken Input von VGLUT1-beschichteten glutamatergen Synapsen.
Verschiedene Nuklei des Gehirnstamm visuellen Systems erhalten einen starken Input durch VGLUT2 synaptische Punkte. Daher scheint der Gehirnstamm des optischen Systems ausschließlich durch VGLUT2 glutamaterge Synapsen versorgt zu werden.
Es ergeben sich folgende Ergebnisse und Folgerungen aus den Untersuchungen: DNPI ist ein neuer Marker für glutamaterge synaptische Vesikel, wobei es 2 unterschiedliche Typen von Neuronen, bzw. Synapsen gibt. VGLUT1 und VGLUT2 zeigen ein differentielles Verteilungsmuster.
Insgesamt läßt sich aus der Verteilung der BNPI und DNPI im ZNS und PNS zeigen, daß diese eine Rolle bei den verschiedenen bereits oben genannten Indikationen spielen, für die mit dem erfindungsgemäßen Verfahren an diesen Targets ansetzende arzneilich wirksame Verbindungen gesucht werden.

### Beispiel 3:

### Durchführung des Screeningverfahrens mit Messung der Bindung über die Verdrängung eines radioaktiv markierten Liganden

Ein Nukleinsäureabschnitt, der für BNPI kodiert, wird in einem Expressionsvektor kloniert, der eine konstitutive Expression (z.B. CMV-Promoter) oder eine induzierbare Expression in eukaryonten Zellen erlaubt. Die DNA wird mit geeignetem Transfektionsverfahren, z.B. mit Lipofectamin (Fa. Roche Diagnostics) in eukaryontischen Zellen (z.B. CHO-Zellen, HEK293-Zellen oder NIH-3T3-Zellen) hineingebracht. Die Zellen werden in Anwesenheit eines Selektionsreagenzen (z.B. Zeocin; Hygromycin oder Neomycin) kultiviert so daß nur die Zellen überleben, die das DNA-Konstrukt aufgenommen haben, und bei länger andauernder Selektion, auch in das Genom inkorporiert haben.
Ausgehend von diesen Zellen werden Membranfraktionen gewonnen, die BNPI in großer Menge enthalten und für einen Bindungsassay verwendet werden können. Dieser besteht aus 1.) dem BNPI enthaltenden Membranen 2.) einem radioaktiv markierten Liganden 3.) einem Bindungspuffer (z.B. 50 mM HEPES pH 7.4, 1 mM EDTA) und dem auf Bindung zu untersuchenden Liganden. Nach Inkubation der oben genannten Reaktionsgemische (für z.B. 30-60 min) bei einer geeigneten Temperatur (meistens Raumtemperatur) werden die nichtgebundenen radioaktiven Ligandenmoleküle abfiltriert. Die Restmenge an gebundenem Liganden wird nach Zugabe eines Szintillationscocktails in einem β-Counter (z.B. Trilux, Fa. Wallac) vermessen. Zeigt die Testsubstanz eine Bindung an BMPI, so wird dies als verringerter radioaktiver Einbau detektiert. Dieses Verfahren wird geeignetermaßen so miniaturisiert, daß es in (96-, 384- oder 1536-well) Mikrotiterplatten durchgeführt werden kann, um dieses Verfahren mittels eines Roboters im sogenannten Hightroughput-Screening (HTS)-Verfahren durchzuführen.

### Beispiel 4:

### Durchführung des erfindungsgemäßen Screeningverfahrens mit BNPI und Messung der durch die Bindung der Substanz veränderten funktionellen Parameter

Ein Nukleinsäureabschnitt, der für BNPI kodiert, wird in einem Expressionsvektor kloniert, der eine induzierbare Expression in Prokaryonten, wie z.B. E.coli erlaubt. Hierbei wird der Nukleinsäureabschnitt so modifiziert, daß er als Fusionsprotein mit einer zusätzlichen N- oder C-terminalen Aminosäuresequenz exprimiert wird. Diese Sequenz sollte bei unveränderter Funktion des BNPI eine Aufreinigung über ein spezifisches Verfahren erlauben, z.B. Glutathion S-Transferasefragment, das über Bindung an Glutathion eine Isolierung aus dem Proteingemisch erlaubt. Nach Transfektion der Bakterien, Induktion des Genes (z.B. mit IPTG beim lac-Promoter) und Aufschließen der Bakterien werden die Fusionsproteine aufgereinigt und in einem in vitro-Kinase Experiment eingesetzt. Hierbei werden 5 µg Protein bei 30°C für 30 Minuten in 50 µl Kinasepuffer (20 mM PIPES, pH 7.0, 5 mM MnCl₂, 7 mM β-Mercaptoethanol, 0.4 mM Spermin, 10 mM rATP) ergänzt mit 10 µCi [γ³²P] ATP. Als Substrate werden gereinigtes Histon H1-Protein (Fa. Sigma) oder bakteriell exprimiertes GST-NFATc1-Fusionsprotein hinzugegeben. Nach der Inkubationszeit wird das nicht-inkorpierte [γ-³²P] ATP abfiltriert und die Menge an eingebautem ³²Phosphat durch β-Szintillation (Trilux, Fa. Wallac) bestimmt. In einem Experiment zum Aufspüren neuer BNPI-Inhibitoren werden in diesem Ansatz die Testsubstanzen mitinkubiert und eine Abnahme der ³²P-Inkorporation als Indikator für einen Inhibitor benutzt. Dieses Verfahren wird geeignetermaßen so miniaturisiert, daß es in (96-, 384- oder 1536-well) Mikrotiterplatten durchgeführt werden kann, um dieses Verfahren mittels eines Roboters im sogenannten Hightroughput-Screening (HTS)-Verfahren durchzuführen.

### Beispiel 5:

### Durchführung des erfindungsgemäßen Screeningverfahrens mit DNPI und Messung der durch die Bindung der Substanz veränderten funktionellen Parameter

Das Verfahren wird wie in Beispiel 4 beschrieben durchgeführt mit der Ausnahme, daß statt eines Nukleinsäureabschnitt, der für BNPI kodiert, ein Nukleinsäureabschnitt eingesetzt wurde, der für DNPI kodiert.

### Beispiel 6:

### Beispiel für ein Arzneimittel zur Tinitus-Behandlung oder zur Behandlung motoneuronischer Störungen enthaltend eine erfindungsgemäße Verbindung - Tablettenformulierung

Tabletten können durch direktes Verpressen von Mischungen der erfindungsgemäßen Verbindung mit entprechenden Hilfsstoffen oder durch Verpressen von verbindungshaltigen Granulaten (mit gegebenenfalls weiteren Hilfsstoffen) hergestellt werden. Die Granulate können dabei entweder durch Feuchtgranulation mit z.B. wäßrigen Granulierflüssigkeiten und anschließender Trocknung dieser Granulate oder durch Trockengranulation z.B. über Kompaktierung hergestellt werden.
■ Direktverpressung

| | | |
|---|---|---|
| z.B. pro Tablette: | 25 mg | erfindungsgemäße Verbindung |
| | 271 mg | LudipressTM (Granulat zur Direkttablettierung aus Lactose monohydrat, Povidon K30 und Crospovidon) |
| | 4 mg | Magnesiumstearat |
| | 300 mg | Gesamt |

Homogene Mischung des Wirkstoffes mit den Hilfsstoffen herstellen und diese auf einer Tablettenpresse zu Tabletten mit einem ∅ von 10 mm verpressen.
■ Trockengranulation

| | | |
|---|---|---|
| z.B. pro Tablette: | 25 mg | erfindungsgemäße Verbindung |
| | 166 mg | Microcristalline Cellulose |
| | 80 mg | Niedrig substituierte Hydroxypropylcellulose (I-HPC LH 11™) |
| | 5 mg | Hochdisperses Siliziumdioxid |
| | 4 mg | Magnesiumstearat |
| | 280 mg | Gesamt |

Homogene Mischung der Verbindung mit der Mikrokristallinen Cellulose und der I-HPC herstellen und diese Kopaktieren. Nach dem Sieben der Komprimate wird das entstandene Granulat mit Magnesiumstearat und Siliziumdioxid gemischt und auf einer Tablettenpresse zu Tabletten mit einem ∅ von 9 mm verpreßt.
■ Feuchtgranulation

| | | |
|---|---|---|
| z.B. pro Tablette: | 25 mg | erfindungsgemäße Verbindung |
| | 205 mg | Mikrokristalline Cellulose |
| | 6 mg | Povidon K30 |
| | 10 mg | Crospovidon |
| | 4 mg | Magnesiumstearat |
| | 250 mg | Gesamt |

Homogene Mischung der Verbindung mit der Mikrokristallinen Cellulose und dem Crospovidon herstellen und diese in einem Granulator mit einer wäßrigen Lösung des Povidons granulieren. Das feuchte Granulat wird anschließend nachgranuliert und nach der Trocknung im Trockenschrank (50°C) 10 h getrocknet. Das trockene Granulat wird mit dem Magnesiumstearat zusammen gesiebt, endgemischt und auf einer Tablettenpresse zu Tabletten mit einem ∅ von 8 mm verpreßt.

### Beispiel 7:

### Beispiel für ein Arzneimittel zur Tinitus-Behandlung oder zur Behandlung motoneuronischer Störungen enthaltend eine erfindungsgemäße Verbindung - parenterale Lösung

1 g einer erfindungsgemäßen Verbindung wird in 1 I Wasser für Injektionszwecke bei Raumtemperatur gelöst und anschließend durch Zugabe von NaCl (Natriumchlorid) auf isotone Bedingungen eingestellt.

### Literatur:

Aihara Y, Mashima H. Onda H. Hisano Setsuji, Kasuya H., Hori T. Yamada S., Tomura H. Yamada Y., Inoue I., Kojima I. und Takeda J. (2000), J. Neurochem. 74: 2622 - 2625
Akopian AN, Sivilotti L & Wood JN (1995) Nature 379: 257-262
Ausubel FM, Brent R, Kingdton RE, Moore DD, Seidman JG, Smith JA & Struhl K eds.(1190) Current protocols in molecular biology. John Wiley &Sons, Inc. New York, NY.
Baba H, Doubell TP, Woolf CJ 1999: Peripheral inflammation facilitates Aβ fiber-mediated synaptic input to the substantia gelatinosa of the adult rat spinal cord. J Neurosci 19: 859-867
Bauer D, Müller H, Reich J, Riedel H, Ahrenkiel V, Warthoe P & Strauss M (1993): Identification of differentially expressed mRNA species by an improved display technique (DDRT-PCR) Nucl Acids Res 21: 4272-4280.
Bonini A, Anderson SM, Steiner DF (1997) Molecular cloning and tissue expression of a novel orphan G Protein-coupled receptor from rat lung. Biochem Biophys Res Comm 234: 190-193.
Chih-Cheng et al., (1995): A P2X prinoceptor expressed by a subset of sensory neurons. Nature 377:428-432
Corderre TJ, Katz J, Vaccarino AL, Melzack R (1993): Contribution of central plasticity to pathological pain: review of clinical and experimental evidence. Pain 52: 259-285.
Dickenson (1995) Novel pharmacological targets in the treatment of pain. Pain Rev., **2**, 1-12.
Dubuisson et al., 1997 Pain, 4:161-174.
Feng Y & Gregor P (1997) Cloning of a novel member of the G Protein-coupled receptor family related to peptide receptors. Biochem Biophys Res Comm 231: 651-654.
Furukawa T, Yang Y, Nakamoto B, Stamatoyannopoulos G, Papayannopoulou T (1996): Identification of new genes expressed in a human erythroleukemia cell line. Bloods Cell Mol & Dis 22:11-22.
Gunasekar PG, Kanthasamy, AG, Borowitz JL, Isom GE 1995: NMDA receptor activation produces concurrent generation of nitric oxide and reactive oxygen species: implication for cell death. J Neurochem 65: 2016-2021.
Hawes BE, Fried S, Yao X, Weig B, Graziano MP 1998: Nociceptin (ORL1) and µ-Opioid receptors mediate mitogen-activated protein kinase activation in CHO cells through a Gi-coupled signaling pathway: evidence for distinct mechanisms of agonist-mediated desensitization. J Neurochem 71: 1024-1033.
Hubank M & Schatz DG (1994): Identifying differences in mRNA expression by representational difference analysis of cDNA. Nucl Acids Res 22: 5640-5648.
Klußmann S et al., 1996: Nature Biotechnology 14: 1112-1115.
Li L-Y & Chang K-J 1996: The stimulatory effect of opioids on mitogen-activated protein kinase in chinese hamster ovary cells transfected to express µ-opioid receptors. Mol Pharm 50:599-602.
Liang P & Pardee AB 1992: Differential Display of eukaryotic messenger RNA by means of the polymerase chain reaction. Science 257:967-971.
Methner A, Hermey G, Schinke B, Hermanns-Borgmeyer I (1997): A novel G Protein-coupled receptor with homology to neuropeptide and chemoattractant receptors expressed during bone development. Biochem Biophys Res Comm 233: 336-342.
Mohit AA, Martin JH & Miller CA 1995: p493F12 Kinase: a novel MAP kinase expressed in a subset of neurons in the human nervous system. Neuron 14: 67-78.
Poirier GM-C, Pyati J, Wan JS, Erlander MG 1997: Screening differentially expressed cDNA clones obtained by differential display using amplified RNA. Nucleic Acids Research 25: 913-914.
Sambrook J, Fritsch EF & Maniatis T 1989: Molecular Cloning: A Laboratory Manual. Second Edition. Cold Spring Harbor Laboratory Press, Cold Spring Harbor.
Sompayrac L, Jane S, Burn TC, Tenen DG & Danna KJ 1995: Overcoming limitations of the mRNA differential display technique. Nucleic Acids Research 23: 4738-4739.
Tal M 1996: A novel antioxidant alleviates heat hyperalgesia in rats with an experimental painful neuropathy. Neurreport 7: 1382-1384.
Tölle TR (1997): Chronischer Schmerz. In: Klinische Neurobiologie, Herdergen T, Tölle TR, Bähr M (Hrsg.): S. 307-336; Spektrum Verlag, Heidelberg.
U.S.Patent 5.262.311
Velculescu VE, Zhang L, Vogelstein B, Kinzler KW (1995): Serial analysis of gene expression. Science 270: 484-487.
Wan JS, Sharp JS et al. (1996): Cloning differentially expressed mRNAs Nature Biotech 14:1685-1691.
Watson JB & Margulies JE (1993) Differential cDNA screening strategies to identify novel stage-specific proteins in the developing mammalian brain. Dev Neurosci 15: 77-86.
Wilks AF (1989) Two putative protein-tyrosine kinases identified by application of the polymerase chain reaction. Poc Natl Acad Sci USA 86: 1603-1607.
WO96/34288
Woolf CJ, Shortland P, Coggeshall RE 1992: Peripheral nerve injury triggers central sprouting of myelinated afferents. Nature 355:75-78.
Zimmermann, M & Herdegen, T (1996): Plasticity of the nervous system at the systemic, cellular and molecular levels: a mechanism of chronic pain and hyperalgesia. Progr Brain Res 110: 233-259

## Patentansprüche

1. Verfahren zur Auffindung pharmazeutisch relevanter Substanzen mit Wirksamkeit in den Indikationen oder zur Behandlung von
Sehstörungen, Retinitis pigmentosa, Opticus Degeneration, Hörstörungen, Tinitus, M. Menière, Hörsturz, Schizophrenie, Manien, Depression, Schlaganfall, Hirntrauma, Querschnittslähmung, Neuralgie, Gewichtsregulation, Obesitas, Anorexia nervosa, Epilepsie, Hemibalismus, Chorea Huntington, Stress, Morbus Parkinson, TIA (Transiente Ischämische Attacken), Emesis, insbesondere Hyperemesis beispielsweise bei Chemotherapie, Schwindel, in jeglicher Form, Katarakt, Arthritis, Hyperaktivität, Entwicklungsstörungen, Tollwut, Virus-Infektionen oder bakterielle Infektionen, Grippe, Malaria, Creutzfeld-Jacob, Inflammatory Bowel disease, Morbus Crohn, cardiovaskuläre und cardiorespiratorische Funktionsstörungen, Hypertonie, Störungen der Baroafferenz oder Chemoafferenz, Toxoplasmose, Asthma, Autoimmunität im zentralen und peripheren Nervensystem, diabetische Neuropathie, autoimmuner Diabetes, alkoholische Neuropathie, HIV-Neuro-Aids; Störungen des autonomen Nervensystems, Störungen des Nervensystems des Verdauungstraktes, Übererregbarkeit, insbesondere glutamatvermittelte Übererregbarkeit, Neurodegeneration insbesondere bei Morbus Alzheimer, Morbus Alzheimer, Ischämie; Encephalitis insbesondere virale oder bakterielle; Prionerkrankung, Rasmussen-Encephalitis, HIV-Encephalitis, Demyelinisierung insbesondere bei multipler Sklerose, Retinadegeneration, Glaukom, Nystagmus, Netzhautablösung, Erkrankungen des Cerebellums, cerebelläre Ataxie, Erkrankungen der Basalganglien, Erkrankungen des Pallidums, Erkrankungen des Hör- und/oder Gleichgewichtsorgans, Erkrankungen der Hörbahn oder Vesibularbahn, Störungen des Gedächtnisses, Störungen des Lernens, Störungen der Kognition, Stiff-Man-Syndrom, Restless Leg-Syndrom, Angstzustände, Phobien, Schlafstörungen; Drogenabhängigkeit, -sucht und -entzug insbesondere bei Alkohol, Nikotin, Opiaten, Ecstasy oder Kokain; Hepatoencephalopathie mit Alkoholintoxikation, Hepatoencephalopathie ohne Alkoholintoxikation, neurotoxikologisch bedingte Erkrankungen, Erkrankungen der Hinterstrangbahnen, alkoholische Neuropathien, Neuroinflammation, Befindlichkeitsstörungen bei Infektionen oder Fieber, Stress, Geschmacksstörungen, Nahrungsmittelallergien, Chinese-Restaurant-Syndrom, Agression, Paranoia, Hirnerschütterung, neuroendokrine Störungen, Tourrette-Syndrom, cerebrovaskuläre Spasmen, neuronale Apoptose, Neurodegeneration, neuronale Nekrose, Astrocytose, Burn-out-Syndrom, Sudden-Infant-Death (plötzlicher Kindstod), Herzinfarkt, Insomnia, retrograde Amnesie, Jet-lag, Störungen der Sexualfunktion, wie Impotenz oder Priapismus oder mit Wirksamkeit zur Förderung der Mikrogliaaktivierung, des Lernens, der Kognition oder des Gedächtnisses, zur Neuroprotektion, zur Liquordiagnostik neurostatischer Erkrankungen oder zur adjuvanten Therapie per Elektrostimulation des Nucleus subthalamicus bei Parkinson
mit folgenden Verfahrensschritten:
(a) Inkubation einer zu testenden Substanz unter geeigneten Bedingungen mit mindestens einem Biomolekül aus Gruppe I ausgewählt aus:
dem Protein BNPI und/oder einem Protein gemäß einer der Abbildungen 1b), 1d), 1f), 1h), und/oder einem zu einem dieser vorgenannten Proteine zu mindestens 90 % ähnlichen Protein und/oder einem Protein, für das ein Polynukleotid gemäß einer der Abbildungen 1a), 1c), 1e), 1g), oder ein dazu zu mindestens 90 % ähnliches Polynukleotid kodiert, und/oder einem Protein, das durch eine Nukleinsäure kodiert wird, die unter stringenten Bedingungen an ein Polynukleotid gemäß einer der Abbildungen 1a), 1c), 1e), 1g), oder deren Antisense Polynukleotide bindet, oder einem mindestens 10, vorzugsweise mindestens 15, insbesondere mindestens 20 Aminosäuren langen Teilprotein eines der vorgenannten Proteine
und/oder einer Zelle und/oder einer Präparation aus einer solchen Zelle, die mindestens eines der vorgenannten Proteine und Teilproteine, bzw. Biomoleküle aus Gruppe I, synthetisiert hat,
(b) Messung der Bindung der Testsubstanz an dem oder den gegebenenfalls von einer Zelle synthetisierten Biomolekül/en aus Gruppe I oder an einer Zelle und/oder einer Präparation aus einer solchen Zelle, die mindestens eines der vorgenannten Biomoleküle aus Gruppe I synthetisiert hat, oder Messung mindestens eines der durch die Bindung der Testsubstanz an dem oder den gegebenenfalls von einer Zelle synthetisierten Biomolekül/en aus Gruppe I oder an einer Zelle und/oder einer Präparation aus einer solchen Zelle, die mindestens eines der vorgenannten Biomoleküle aus Gruppe I synthetisiert hat, veränderten funktionellen Parameter.

2. Verfahren gemäß Anspruch 1, **dadurch gekennzeichnet, daß** die Zelle vor dem Schritt (a) gentechnisch manipuliert wird.

3. Verfahren gemäß Anspruch 2, **dadurch gekennzeichnet, daß** die gentechnische Manipulation die Messung mindestens eines der durch die Testsubstanz veränderten funktionellen Parameter erlaubt.

4. Verfahren gemäß Anspruch 3, **dadurch gekennzeichnet, daß** durch die gentechnische Manipulation eine in der Zelle nicht endogen exprimierte Form eines G-Proteins exprimiert oder ein Reportergen eingeführt wird.

5. Verfahren gemäß einem der Ansprüche 2-4, **dadurch gekennzeichnet, daß** die Zelle gentechnisch so manipuliert wird, daß die Zelle mindestens ein Polynukleotid gemäß einer der Abbildungen 1a), 1c), 1e), 1g), oder ein dazu zu mindestens 90 %, vorzugsweise zu mindestens 95 %, insbesondere zu mindestens 97 % ähnliches Polynukleotid enthält.

6. Verfahren gemäß Anspruch 5, **dadurch gekennzeichnet, daß** das Polynukleotid in einem rekombinanten DNA-Konstrukt enthalten ist.

7. Verfahren gemäß einem der Ansprüche 2 bis 6, **dadurch gekennzeichnet, daß** die Zelle nach der gentechnischen Manipulation gemäß Anspruch 2 und vor dem Schritt (a) gemäß Anspruch 1 unter Bedingungen, die eine Expression erlauben, kultiviert wird, gegebenenfalls unter Selektionsdruck.

8. Verfahren gemäß einem der Ansprüche 1 bis 7, **dadurch gekennzeichnet, daß** die Zelle eine Amphibienzelle, Bakterienzelle, Hefezelle, Insektenzelle oder eine immortalisierte oder native Säugetierzelle ist.

9. Verfahren gemäß einem der Ansprüche 1 bis 8, **dadurch gekennzeichnet, daß** die Messung der Bindung über die Verdrängung eines bekannten markierten Liganden des Biomoleküls, bzw. des Teilproteins und/oder Proteins und/oder über die daran gebundene Aktivität einer markierten Testsubstanz erfolgt.

10. Verfahren gemäß einem der Ansprüche 1 bis 8, **dadurch gekennzeichnet, daß** die Messung mindestens eines der durch die Testsubstanz veränderten funktionellen Parameter über Messung der Regulation, Hemmung und/oder Aktivierung von Rezeptoren, lonenkanälen und/oder Enzymen erfolgt, insbesondere über Messung der Veränderung der Genexpression, des Ionenmilieus, des pH oder des Membranpotentials, über Veränderung der Enzymaktivität oder der Konzentration der 2^{nd} messenger.

11. Verfahren gemäß einem der Ansprüche 1 bis 10, bei dem ein erstes Verfahren gemäß einem der Ansprüche 1 bis 10 mit einem zweiten Verfahren gemäß einem der Ansprüche 1 bis 10 derart gekoppelt wird, daß die Meßwerte und Ergebnisse des ersten Verfahrens hinsichtlich der zu messenden Substanz mit den Meßwerten und Ergebnissen des zweiten Verfahrens hinsichtlich der zu messenden Substanz verglichen werden, **dadurch gekennzeichnet, daß** in einem der zwei Verfahren, im folgenden Hauptverfahren genannt, im Schritt (a) die zu testende Substanz
entweder
mit einem Biomolekül ausgewählt aus Gruppe II:
dem Protein BNPI und/oder einem Protein gemäß einer der Abbildungen 1b), 1d), 1f) oder 1h) und/oder einem zu einem dieser vorgenannten Proteine zu mindestens 90 % ähnlichen Protein und/oder einem Protein, für das ein Polynukleotid gemäß einer der Abbildungen 1a), 1c), 1e) oder 1g) oder ein dazu zu mindestens 90 % ähnlichen Polynukleotid kodiert, und/oder einem Protein, das durch eine Nukleinsäure kodiert wird, die unter stringenten Bedingungen an ein Polynukleotid gemäß einer der Abbildungen 1a), 1c), 1e) oder 1g) oder deren Antisense Polynukleotide bindet, oder einem mindestens 10, vorzugsweise mindestens 15, insbesondere mindestens 20 Aminosäuren langen Teilprotein eines der vorgenannten Proteine
und/oder einer Zelle und/oder einer Präparation aus einer solchen Zelle, die mindestens eines der vorgenannten Proteine und Teilproteine, bzw. Biomoleküle aus Gruppe II, synthetisiert hat,
und
daß im anderen der zwei Verfahren, im folgenden Nebenverfahren genannt, im Schritt (a) die zu testende Substanz mit einem Biomolekül aus der Gruppe I oder mit einem Biomolekül aus derjenigen Gruppe ausgewählt aus Gruppe II und Gruppe III inkubiert wird, aus der das Biomolekül, mit der die Substanz im Hauptverfahren inkubiert wird, nicht ausgewählt ist.

12. Verfahren gemäß einem der Ansprüche 1 bis 11, **dadurch gekennzeichnet, daß** die aufzufindenden Substanzen ausgewählt sind aus:
Substanzen mit Wirksamkeit in den Indikationen oder zur Behandlung von Sehstörungen, Retinitis pigmentosa, Opticus Degeneration, Hörstörungen, Tinitus, M. Menière, Hörsturz Neuralgie, Gewichtsregulation, Obesitas, Morbus Parkinson, Katarakt, Virus-Infektionen oder bakterielle Infektionen, diabetische Neuropathie, autoimmuner Diabetes, alkoholische Neuropathie, HIV-Neuro-Aids; Retinadegeneration, Glaukom, Nystagmus, Netzhautablösung, Erkrankungen des Hör- und/oder Gleichgewichtsorgans, Erkrankungen der Hörbahn oder Vesibularbahn, Schlafstörungen, Drogenabhängigkeit, -sucht und -entzug insbesondere bei Alkohol, Nikotin, Opiaten, Ecstasy oder Kokain; Neuroinflammation, Insomnia, zur adjuvanten Therapie per Elektrostimulation des Nucleus subthalamicus bei Parkinson;
vorzugsweise
Substanzen mit Wirksamkeit in den Indikationen oder zur Behandlung von Sehstörungen, Retinitis pigmentosa, Opticus Degeneration, Hörstörungen, Tinitus, M. Menière, Hörsturz Gewichtsregulation, Obesitas, Katarakt, Virus-Infektionen oder bakterielle Infektionen, Retinadegeneration, Glaukom, Nystagmus, Netzhautablösung, Erkrankungen des Hör- und/oder Gleichgewichtsorgans, Erkrankungen der Hörbahn oder Vesibularbahn, Drogenabhängigkeit, -sucht und -entzug insbesondere bei Alkohol, Nikotin, Opiaten, Ecstasy oder Kokain; Neuroinflammation;
insbesondere
Substanzen mit Wirksamkeit in den Indikationen oder zur Behandlung von Sehstörungen, Retinitis pigmentosa, Opticus Degeneration, Katarakt, Netzhautablösung, Retinadegeneration, Glaukom oder Nystagmus
und/oder
Hörstörungen, Tinitus, M. Menière, Hörsturz, Erkrankungen des Hör- und/oder Gleichgewichtsorgans oder Erkrankungen der Hörbahn oder Vesibularbahn;

13. Verbindung identifizierbar durch ein Verfahren gemäß einem der Ansprüche 1 bis 12 als pharmazeutisch relevante Substanz mit Wirksamkeit in mindestens einer der Indikationen gemäß Anspruch 1 oder 12.

14. Verbindung gemäß Anspruch 13, **dadurch gekennzeichnet, daß** sie eine niedermolekulare Verbindung ist.

15. Verwendung
a. eines Polynukleotids, vorzugsweise einer DNA oder RNA, kodierend für BNPI oder eines Polynukleotids, vorzugsweise einer DNA oder RNA, welches einer der in einer der Abbildungen 1a), 1c), 1e), 1g), dargestellten Nukleotidsequenzen zu wenigstens 90%, vorzugsweise zu wenigstens 95%, insbesondere zu wenigstens 97% oder genau entspricht,
b. eines Polynukleotids, insbesondere eines Antisense Polynukleotids oder einer PNA, vorzugsweise eines DNA-Enzyms oder Ribozyms, eines Ribozyms oder sonstigen DNA-Enzyms oder einer katalytischen RNA oder DNA, das eine Nukleotid-Sequenz aufweist, die in der Lage ist, spezifisch an eines der unter Punkt a) aufgeführten Polynukleotide zu binden,
c. eines Vektors enthaltend ein Polynukleotid gemäß einem der Punkte a) oder b), insbesondere eines Expressionsvektors und/oder insbesondere abgeleitet von einem Virus, beispielsweise dem Adenovirus, Adenoassoziiertem Virus oder Herpesvirus und/oder insbesondere enthaltend mindestens eine LTR-, Poly A-, Promotor- und/oder ORI-Sequenz,
d. von BNPI und/oder eines Proteins gemäß einer der Abbildungen 1b), 1d), 1f), 1h), und/oder eines zu einem dieser vorgenannten Proteine zu mindestens 90 %, vorzugsweise mindestens 95%, insbesondere mindestens 97% ähnlichen Proteins und/oder eines Proteins, für das ein Polynukleotid gemäß einer der Abbildungen 1a), 1c), 1e), 1g), oder ein dazu zu mindestens 90 %, vorzugsweise mindestens 95%, insbesondere mindestens 97% ähnliches Polynukleotid kodiert, und/oder eines Proteins, das durch eine Nukleinsäure kodiert wird, die unter stringenten Bedingungen an ein Polynukleotid gemäß einer der Abbildungen 1a), 1c), 1e), 1g), oder dessen Antisense Polynukleotide bindet oder eines mindestens 10, vorzugsweise mindestens 15, insbesondere mindestens 20 Aminosäuren langen Teilproteins eines der vorgenannten Proteine, wobei das Protein oder Teilprotein gegebenenfalls posttranslational modifiziert, insbesondere glykosiliert, phosphoryliert, amidiert, methyliert, acetyliert, ADP-ribosyliert, hydroxyliert, mit einem Membrananker versehen, gespalten oder verkürzt wurde,
e. eines Antikörpers, vorzugsweise eines monoklonalen oder polyklonalen Antikörpers, gegen eines der Proteine oder Teilproteine gemäß Punkt d),
f. einer Zelle, vorzugsweise einer Amphibienzelle, Bakterienzelle, Hefezelle, Insektenzelle oder einer immortalisierten oder nativen Säugetierzelle, enthaltend ein Polynukleotid gemäß einem der Punkte a) oder b), einen Vektor gemäß Punkt c), ein Protein oder Teilprotein gemäß Punkt d) oder einen Antikörper gemäß Punkt e)
g. einer Verbindung gemäß einem der Ansprüche 13 oder 14 und/oder
h. eines Wirkstoffs, vorzugsweise eines niedermolekularen Wirkstoffs, der an ein Protein oder Teilprotein gemäß Punkt d) bindet,
zur Herstellung eines Arzneimittels zur Behandlung von
Sehstörungen, Retinitis pigmentosa, Opticus Degeneration, Hörstörungen, Tinitus, M. Menière, Hörsturz, Schizophrenie, Manien, Depression, Schlaganfall, Hirntrauma, Querschnittslähmung, Neuralgie, Gewichtsregulation, Obesitas, Anorexia nervosa, Epilepsie, Hemibalismus, Chorea Huntington, Stress, Morbus Parkinson, TIA (Transiente Ischämische Attacken), Emesis, insbesondere Hyperemesis beispielsweise bei Chemotherapie, Schwindel, in jeglicher Form, Katarakt, Arthritis, Hyperaktivität, Entwicklungsstörungen, Tollwut, Virus-Infektionen oder bakterielle Infektionen, Grippe, Malaria, Creutzfeld-Jacob, Inflammatory Bowel disease, Morbus Crohn, cardiovaskuläre und cardiorespiratorische Funktionsstörungen, Hypertonie, Störungen der Baroafferenz oder Chemoafferenz, Toxoplasmose, Asthma, Autoimmunität im zentralen und peripheren Nervensystem, diabetische Neuropathie, autoimmuner Diabetes, alkoholische Neuropathie, HIV-Neuro-Aids; Störungen des autonomen Nervensystems, Störungen des Nervensystems des Verdauungstraktes, Übererregbarkeit, insbesondere glutamatvermittelte Übererregbarkeit, Neurodegeneration insbesondere bei Morbus Alzheimer, Morbus Alzheimer, Ischämie; Encephalitis insbesondere virale oder bakterielle; Prionerkrankung, Rasmussen-Encephalitis, HIV-Encephalitis, Demyelinisierung insbesondere bei multipler Sklerose, Retinadegeneration, Glaukom, Nystagmus, Netzhautablösung, Erkrankungen des Cerebellums, cerebelläre Ataxie, Erkrankungen der Basalganglien, Erkrankungen des Pallidums, Erkrankungen des Hör- und/oder Gleichgewichtsorgans, Erkrankungen der Hörbahn oder Vesibularbahn, Störungen des Gedächtnisses, Störungen des Lernens, Störungen der Kognition, Stiff-Man-Syndrom, Restless Leg-Syndrom, Angstzustände, Phobien, Schlafstörungen; Drogenabhängigkeit, -sucht und -entzug insbesondere bei Alkohol, Nikotin, Opiaten, Ecstasy oder Kokain; Hepatoencephalopathie mit Alkoholintoxikation, Hepatoencephalopathie ohne Alkoholintoxikation, neurotoxikologisch bedingte Erkrankungen, Erkrankungen der Hinterstrangbahnen, alkoholische Neuropathien, Neuroinflammation, Befindlichkeitsstörungen bei Infektionen oder Fieber, Stress, Geschmacksstörungen, Nahrungsmittelallergien, Chinese-Restaurant-Syndrom, Agression, Paranoia, Hirnerschütterung, neuroendokrine Störungen, Tourrette-Syndrom, cerebrovaskuläre Spasmen, neuronale Apoptose, Neurodegeneration, neuronale Nekrose, Astrocytose, Burn-out-Syndrom, Sudden-Infant-Death (plötzlicher Kindstod), Herzinfarkt, Insomnia, retrograde Amnesie, Jet-lag, Störungen der Sexualfunktion, wie Impotenz oder Priapismus oder mit Wirksamkeit zur Förderung der Mikrogliaaktivierung, des Lernens, der Kognition oder des Gedächtnisses, zur Neuroprotektion, zur Liquordiagnostik neurostatischer Erkrankungen oder zur adjuvanten Therapie per Elektrostimulation des Nucleus subthalamicus bei Parkinson.

16. Verwendung
a. eines Polynukleotids, vorzugsweise einer DNA oder RNA, kodierend für BNPI oder DNPI oder eines Polynukleotids, vorzugsweise einer DNA oder RNA, welches einer der in einer der Abbildungen 1a), 1c), 1e), 1g), dargestellten Nukleotidsequenzen zu wenigstens 90%, vorzugsweise zu wenigstens 95%, insbesondere zu wenigstens 97% entspricht,
b. eines Polynukleotids, insbesondere eines Antisense Polynukleotids oder einer PNA, vorzugsweise eines DNA-Enzyms oder Ribozyms, eines Ribozyms oder sonstigen DNA-Enzyms oder einer katalytischen RNA oder DNA, das eine Nukleotid-Sequenz aufweist, die in der Lage ist, spezifisch an eines der unter Punkt a) aufgeführten Polynukleotide zu binden,
c. eines Vektors enthaltend ein Polynukleotid gemäß einem der Punkte a) oder b), insbesondere eines Expressionsvektors und/oder insbesondere abgeleitet von einem Virus, beispielsweise dem Adenovirus, Adenoassoziiertem Virus oder Herpesvirus und/oder insbesondere enthaltend mindestens eine LTR-, Poly A-, Promotor- und/oder ORI-Sequenz,
f. einer Zelle, vorzugsweise einer Amphibienzelle, Bakterienzelle, Hefezelle, Insektenzelle oder einer immortalisierten oder nativen Säugetierzelle, enthaltend ein Polynukleotid gemäß einem der Punkte a) oder b) oder einen Vektor gemäß Punkt c)
zur Herstellung eines Arzneimittels zum Einsatz in der Gentherapie.

17. Verwendung gemäß Anspruch 16, **dadurch gekennzeichnet, daß** es sich um In-vivo oder In-vitro Gentherapie handelt.

18. Verwendung gemäß einem der Ansprüche 16 oder 17, **dadurch gekennzeichnet, daß** es sich um ein Arzneimittel mit Wirksamkeit in der Indikation oder zur Behandlung von
Sehstörungen, Retinitis pigmentosa, Opticus Degeneration, Hörstörungen, Tinitus, M. Menière, Hörsturz, Schizophrenie, Manien, Depression, Schlaganfall, Hirntrauma, Querschnittslähmung, Neuralgie, Gewichtsregulation, Obesitas, Anorexia nervosa, Epilepsie, Hemibalismus, Chorea Huntington, Stress, Morbus Parkinson, TIA (Transiente Ischämische Attacken), Emesis, insbesondere Hyperemesis beispielsweise bei Chemotherapie, Schwindel, in jeglicher Form, Katarakt, Arthritis, Hyperaktivität, Entwicklungsstörungen, Tollwut, Virus-Infektionen oder bakterielle Infektionen, Grippe, Malaria, Creutzfeld-Jacob, Inflammatory Bowel disease, Morbus Crohn, cardiovaskuläre und cardiorespiratorische Funktionsstörungen, Hypertonie, Störungen der Baroafferenz oder Chemoafferenz, Toxoplasmose, Asthma, Autoimmunität im zentralen und peripheren Nervensystem, diabetische Neuropathie, autoimmuner Diabetes, alkoholische Neuropathie, HIV-Neuro-Aids; Störungen des autonomen Nervensystems, Störungen des Nervensystems des Verdauungstraktes, Übererregbarkeit, insbesondere glutamatvermittelte Übererregbarkeit, Neurodegeneration insbesondere bei Morbus Alzheimer, Morbus Alzheimer, Ischämie; Encephalitis insbesondere virale oder bakterielle; Prionerkrankung, Rasmussen-Encephalitis, HIV-Encephalitis, Demyelinisierung insbesondere bei multipler Sklerose, Retinadegeneration, Glaukom, Nystagmus, Netzhautablösung, Erkrankungen des Cerebellums, cerebelläre Ataxie, Erkrankungen der Basalganglien, Erkrankungen des Pallidums, Erkrankungen des Hör- und/oder Gleichgewichtsorgans, Erkrankungen der Hörbahn oder Vesibularbahn, Störungen des Gedächtnisses, Störungen des Lernens, Störungen der Kognition, Stiff-Man-Syndrom, Restless Leg-Syndrom, Angstzustände, Phobien, Schlafstörungen; Drogenabhängigkeit, -sucht und -entzug insbesondere bei Alkohol, Nikotin, Opiaten, Ecstasy oder Kokain; Hepatoencephalopathie mit Alkoholintoxikation, Hepatoencephalopathie ohne Alkoholintoxikation, neurotoxikologisch bedingte Erkrankungen, Erkrankungen der Hinterstrangbahnen, alkoholische Neuropathien, Neuroinflammation, Befindlichkeitsstörungen bei Infektionen oder Fieber, Stress, Geschmacksstörungen, Nahrungsmittelallergien, Chinese-Restaurant-Syndrom, Agression, Paranoia, Hirnerschütterung, neuroendokrine Störungen, Tourrette-Syndrom, cerebrovaskuläre Spasmen, neuronale Apoptose, Neurodegeneration, neuronale Nekrose, Astrocytose, Burn-out-Syndrom, Sudden-Infant-Death (plötzlicher Kindstod), Herzinfarkt, Insomnia, retrograde Amnesie, Jet-lag, Störungen der Sexualfunktion, wie Impotenz oder Priapismus oder mit Wirksamkeit zur Förderung der Mikrogliaaktivierung, des Lernens, der Kognition oder des Gedächtnisses, zur Neuroprotektion, zur Liquordiagnostik neurostatischer Erkrankungen oder zur adjuvanten Therapie per Elektrostimulation des Nucleus subthalamicus bei Parkinson
handelt.

19. Verwendung
a. eines Polynukleotids, vorzugsweise einer DNA oder RNA, kodierend für BNPI oder eines Polynukleotids, vorzugsweise einer DNA oder RNA, welches einer der in einer der Abbildungen 1a), 1c), 1e), 1g), dargestellten Nukleotidsequenzen zu wenigstens 90%, vorzugsweise 95%, insbesondere zu wenigstens 97% entspricht,
b. eines Polynukleotids, insbesondere eines Antisense Polynukleotids oder einer PNA, vorzugsweise eines DNA-Enzyms oder Ribozyms, eines Ribozyms oder sonstigen DNA-Enzyms oder einer katalytischen RNA oder DNA, das eine Nukleotid-Sequenz aufweist, die in der Lage ist, spezifisch an eines der unter Punkt a) aufgeführten Polynukleotide zu binden,
c. eines Vektors enthaltend ein Polynukleotid gemäß einem der Punkte a) oder b), insbesondere eines Expressionsvektors und/oder insbesondere abgeleitet von einem Virus, beispielsweise dem Adenovirus, Adenoassoziiertem Virus oder Herpesvirus und/oder insbesondere enthaltend mindestens eine LTR-, Poly A-, Promotor- und/oder ORI-Sequenz,
d. von BNPI und/oder eines Proteins gemäß einer der Abbildungen 1b), 1d), 1f), 1h), und/oder eines zu einem dieser vorgenannten Proteine zu mindestens 90 %, vorzugsweise mindestens 95%, insbesondere mindestens 97% ähnlichen Proteins und/oder eines Proteins, für das ein Polynukleotid gemäß einer der Abbildungen 1a), 1c), 1e), 1g), oder ein dazu zu mindestens 90 %, vorzugsweise mindestens 95%, insbesondere mindestens 97% ähnliches Polynukleotid kodiert, und/oder eines Proteins, das durch eine Nukleinsäure kodiert wird, die unter stringenten Bedingungen an ein Polynukleotid gemäß einer der Abbildungen 1a), 1c), 1e), 1g), 2a), 2c) oder 2e) oder dessen Antisense Polynukleotide bindet oder eines mindestens 10, vorzugsweise mindestens 15, insbesondere mindestens 20 Aminosäuren langen Teilproteins eines der vorgenannten Proteine, wobei das Protein oder Teilprotein gegebenenfalls posttranslational modifiziert, insbesondere glykosiliert, phosphoryliert, amidiert, methyliert, acetyliert, ADP-ribosyliert, hydroxyliert, mit einem Membrananker versehen, gespalten oder verkürzt wurde,
e. eines Antikörpers, vorzugsweise eines monoklonalen oder polyklonalen Antikörpers, gegen eines der Proteine oder Teilproteine gemäß Punkt d),
f. einer Zelle, vorzugsweise einer Amphibienzelle, Bakterienzelle, Hefezelle, Insektenzelle oder einer immortalisierten oder nativen Säugetierzelle, enthaltend ein Polynukleotid gemäß einem der Punkte a) oder b), einen Vektor gemäß Punkt c), ein Protein oder Teilprotein gemäß Punkt d) oder einen Antikörper gemäß Punkt e),
g. einer Verbindung gemäß einem der Ansprüche 13 oder 14 und/oder
h. eines Wirkstoffs, vorzugsweise eines niedermolekularen Wirkstoffs, der an ein Protein oder Teilprotein gemäß Punkt d) bindet,
zur Herstellung eines Diagnostikums zur Diagnose eines Zustands ausgewählt aus
Sehstörungen, Retinitis pigmentosa, Opticus Degeneration, Hörstörungen, Tinitus, M. Menière, Hörsturz, Schizophrenie, Manien, Depression, Schlaganfall, Hirntrauma, Querschnittslähmung, Neuralgie, Gewichtsregulation, Obesitas, Anorexia nervosa, Epilepsie, Hemibalismus, Chorea Huntington, Stress, Morbus Parkinson, TIA (Transiente lschämische Attacken), Emesis, insbesondere Hyperemesis beispielsweise bei Chemotherapie, Schwindel, in jeglicher Form, Katarakt, Arthritis, Hyperaktivität, Entwicklungsstörungen, Tollwut, Virus-Infektionen oder bakterielle Infektionen, Grippe, Malaria, Creutzfeld-Jacob, Inflammatory Bowel disease, Morbus Crohn, cardiovaskuläre und cardiorespiratorische Funktionsstörungen, Hypertonie, Störungen der Baroafferenz oder Chemoafferenz, Toxoplasmose, Asthma, Autoimmunität im zentralen und peripheren Nervensystem, diabetische Neuropathie, autoimmuner Diabetes, alkoholische Neuropathie, HIV-Neuro-Aids; Störungen des autonomen Nervensystems, Störungen des Nervensystems des Verdauungstraktes, Übererregbarkeit, insbesondere glutamatvermittelte Übererregbarkeit, Neurodegeneration insbesondere bei Morbus Alzheimer, Morbus Alzheimer, Ischämie; Encephalitis insbesondere virale oder bakterielle; Prionerkrankung, Rasmussen-Encephalitis, HIV-Encephalitis, Demyelinisierung insbesondere bei multipler Sklerose, Retinadegeneration, Glaukom, Nystagmus, Netzhautablösung, Erkrankungen des Cerebellums, cerebelläre Ataxie, Erkrankungen der Basalganglien, Erkrankungen des Pallidums, Erkrankungen des Hör- und/oder Gleichgewichtsorgans, Erkrankungen der Hörbahn oder Vesibularbahn, Störungen des Gedächtnisses, Störungen des Lernens, Störungen der Kognition, Stiff-Man-Syndrom, Restless Leg-Syndrom, Angstzustände, Phobien, Schlafstörungen; Drogenabhängigkeit, -sucht und -entzug insbesondere bei Alkohol, Nikotin, Opiaten, Ecstasy oder Kokain; Hepatoencephalopathie mit Alkoholintoxikation, Hepatoencephalopathie ohne Alkoholintoxikation, neurotoxikologisch bedingte Erkrankungen, Erkrankungen der Hinterstrangbahnen, alkoholische Neuropathien, Neuroinflammation, Befindlichkeitsstörungen bei Infektionen oder Fieber, Stress, Geschmacksstörungen, Nahrungsmittelallergien, Chinese-Restaurant-Syndrom, Agression, Paranoia, Hirnerschütterung, neuroendokrine Störungen, Tourrette-Syndrom, cerebrovaskuläre Spasmen, neuronale Apoptose, Neurodegeneration, neuronale Nekrose, Astrocytose, Burn-out-Syndrom, Sudden-Infant-Death (plötzlicher Kindstod), Herzinfarkt, Insomnia, retrograde Amnesie, Jet-lag, Störungen der Sexualfunktion, wie Impotenz oder Priapismus.

20. Verwendung
a. eines Polynukleotids , vorzugsweise einer DNA oder RNA, kodierend für BNPI oder eines Polynukleotids, vorzugsweise einer DNA oder RNA, welches einer der in einer der Abbildungen 1a), 1c), 1e), 1g), dargestellten Nukleotidsequenzen zu wenigstens 90%, vorzugsweise 95%, insbesondere zu wenigstens 97% entspricht,
b. eines Polynukleotids, insbesondere eines Antisense Polynukleotids oder einer PNA, vorzugsweise eines DNA-Enzyms oder Ribozyms, eines Ribozyms oder sonstigen DNA-Enzyms oder einer katalytischen RNA oder DNA, das eine Nukleotid-Sequenz aufweist, die in der Lage ist, spezifisch an eines der unter Punkt a) aufgeführten Polynukleotide zu binden,
c. eines Vektors enthaltend ein Polynukleotid gemäß einem der Punkte a) oder b), insbesondere eines Expressionsvektors und/oder insbesondere abgeleitet von einem Virus, beispielsweise dem Adenovirus, Adenoassoziiertem Virus oder Herpesvirus und/oder insbesondere enthaltend mindestens eine LTR-, Poly A-, Promotor- und/oder ORI-Sequenz,
d. von BNPI und/oder eines Proteins gemäß einer der Abbildungen 1b), 1d), 1f), 1h), und/oder eines zu einem dieser vorgenannten Proteine zu mindestens 90 %, vorzugsweise mindestens 95%, insbesondere mindestens 97% ähnlichen Proteins und/oder eines Proteins, für das ein Polynukleotid gemäß einer der Abbildungen 1a), 1c), 1e), 1g), oder ein dazu zu mindestens 90 %, vorzugsweise mindestens 95%, insbesondere mindestens 97% ähnliches Polynukleotid kodiert, und/oder eines Proteins, das durch eine Nukleinsäure kodiert wird, die unter stringenten Bedingungen an ein Polynukleotid gemäß einer der Abbildungen 1a), 1c), 1e), 1g), oder dessen Antisense Polynukleotide bindet oder eines mindestens 10, vorzugsweise mindestens 15, insbesondere mindestens 20 Aminosäuren langen Teilproteins eines der vorgenannten Proteine, wobei das Protein oder Teilprotein gegebenenfalls posttranslational modifiziert, insbesondere glykosiliert, phosphoryliert, amidiert, methyliert, acetyliert, ADP-ribosyliert, hydroxyliert, mit einem Membrananker versehen, gespalten oder verkürzt wurde,
e. eines Antikörpers, vorzugsweise eines monoklonalen oder polyklonalen Antikörpers, gegen eines der Proteine oder Teilproteine gemäß Punkt d),
f. einer Zelle, vorzugsweise einer Amphibienzelle, Bakterienzelle, Hefezelle, Insektenzelle oder einer immortalisierten oder nativen Säugetierzelle, enthaltend ein Polynukleotid gemäß einem der Punkte a) oder b), einen Vektor gemäß Punkt c), ein Protein oder Teilprotein gemäß Punkt d) oder einen Antikörper gemäß Punkt e) in einem Verfahren zur Auffindung pharmazeutisch relevanter Substanzen mit Wirksamkeit in den Indikationen oder zur Behandlung von Sehstörungen, Retinitis pigmentosa, Opticus Degeneration, Hörstörungen, Tinitus, M. Menière, Hörsturz, Schizophrenie, Manien, Depression, Schlaganfall, Hirntrauma, Querschnittslähmung, Neuralgie, Gewichtsregulation, Obesitas, Anorexia nervosa, Epilepsie, Hemibalismus, Chorea Huntington, Stress, Morbus Parkinson, TIA (Transiente Ischämische Attacken), Emesis, insbesondere Hyperemesis beispielsweise bei Chemotherapie, Schwindel, in jeglicher Form, Katarakt, Arthritis, Hyperaktivität, Entwicklungsstörungen, Tollwut, Virus-Infektionen oder bakterielle Infektionen, Grippe, Malaria, Creutzfeld-Jacob, Inflammatory Bowel disease, Morbus Crohn, cardiovaskuläre und cardiorespiratorische Funktionsstörungen, Hypertonie, Störungen der Baroafferenz oder Chemoafferenz, Toxoplasmose, Asthma, Autoimmunität im zentralen und peripheren Nervensystem, diabetische Neuropathie, autoimmuner Diabetes, alkoholische Neuropathie, HIV-Neuro-Aids; Störungen des autonomen Nervensystems, Störungen des Nervensystems des Verdauungstraktes, Übererregbarkeit, insbesondere glutamatvermittelte Übererregbarkeit, Neurodegeneration insbesondere bei Morbus Alzheimer, Morbus Alzheimer, Ischämie; Encephalitis insbesondere virale oder bakterielle; Prionerkrankung, Rasmussen-Encephalitis, HIV-Encephalitis, Demyelinisierung insbesondere bei multipler Sklerose, Retinadegeneration, Glaukom, Nystagmus, Netzhautablösung, Erkrankungen des Cerebellums, cerebelläre Ataxie, Erkrankungen der Basalganglien, Erkrankungen des Pallidums, Erkrankungen des Hör- und/oder Gleichgewichtsorgans, Erkrankungen der Hörbahn oder Vesibularbahn, Störungen des Gedächtnisses, Störungen des Lernens, Störungen der Kognition, Stiff-Man-Syndrom, Restless Leg-Syndrom, Angstzustände, Phobien, Schlafstörungen; Drogenabhängigkeit, -sucht und -entzug insbesondere bei Alkohol, Nikotin, Opiaten, Ecstasy oder Kokain; Hepatoencephalopathie mit Alkoholintoxikation, Hepatoencephalopathie ohne Alkoholintoxikation, neurotoxikologisch bedingte Erkrankungen, Erkrankungen der Hinterstrangbahnen, alkoholische Neuropathien, Neuroinflammation, Befindlichkeitsstörungen bei Infektionen oder Fieber, Stress, Geschmacksstörungen, Nahrungsmittelallergien, Chinese-Restaurant-Syndrom, Agression, Paranoia, Hirnerschütterung, neuroendokrine Störungen, Tourrette-Syndrom, cerebrovaskuläre Spasmen, neuronale Apoptose, Neurodegeneration, neuronale Nekrose, Astrocytose, Burn-out-Syndrom, Sudden-Infant-Death (plötzlicher Kindstod), Herzinfarkt, Insomnia, retrograde Amnesie, Jet-lag, Störungen der Sexualfunktion, wie Impotenz oder Priapismus oder mit Wirksamkeit zur Förderung der Mikrogliaaktivierung, des Lernens, der Kognition oder des Gedächtnisses, zur Neuroprotektion, zur Liquordiagnostik neurostatischer Erkrankungen oder zur adjuvanten Therapie per Elektrostimulation des Nucleus subthalamicus bei Parkinson.

21. Verwendung gemäß einem der Ansprüche 15, 18, 19 oder 20, **dadurch gekennzeichnet, daß** die Indikation oder die zu behandelnde oder zu diagnostizierende Krankheit ausgewählt ist aus Sehstörungen, Retinitis pigmentosa, Opticus Degeneration, Hörstörungen, Tinitus, M. Menière, Hörsturz, Neuralgie, Gewichtsregulation, Obesitas, Morbus Parkinson, Katarakt, Virus-Infektionen oder bakterielle Infektionen, diabetische Neuropathie, autoimmuner Diabetes, alkoholische Neuropathie, HIV-Neuro-Aids; Retinadegeneration, Glaukom, Nystagmus, Netzhautablösung, Erkrankungen des Hör- und/oder Gleichgewichtsorgans, Erkrankungen der Hörbahn oder Vesibularbahn, Schlafstörungen, Drogenabhängigkeit, -sucht und -entzug insbesondere bei Alkohol, Nikotin, Opiaten, Ecstasy oder Kokain; Neuroinflammation, Insomnia, zur adjuvanten Therapie per Elektrostimulation des Nucleus subthalamicus bei Parkinson;
vorzugsweise
Sehstörungen, Retinitis pigmentosa, Opticus Degeneration, Hörstörungen, Tinitus, M. Menière, Hörsturz, Gewichtsregulation, Obesitas, Katarakt, Virus-Infektionen oder bakterielle Infektionen, Retinadegeneration, Glaukom, Nystagmus, Netzhautablösung, Erkrankungen des Hör- und/oder Gleichgewichtsorgans, Erkrankungen der Hörbahn oder Vesibularbahn, Drogenabhängigkeit, -sucht und - entzug insbesondere bei Alkohol, Nikotin, Opiaten, Ecstasy oder Kokain; Neuroinflammation;
insbesondere
Sehstörungen, Retinitis pigmentosa, Opticus Degeneration, Katarakt, Netzhautablösung, Retinadegeneration, Glaukom oder Nystagmus
und/oder
Hörstörungen, Tinitus, M. Meniere, Hörsturz, Erkrankungen des Hör- und/oder Gleichgewichtsorgans oder Erkrankungen der Hörbahn oder Vesibularbahn;
